# EUROPEAN PATENT APPLICATION

(11) **EP 4 083 037 A1**
(43) Date of publication of application: **02.11.2022**
(21) Application number: 20906363.5
(22) Date of filing: 22.12.2020
(51) Int. Cl.: C07D 417/06, A61K 31/427, A61K 31/437, A61K 31/4178, C07D 277/36, C07D 401/06, C07D 405/06, C07D 409/06, C07D 471/04, A61P 25/28

(54) **PYRROLIDINE DERIVATIVE, AND PHARMACEUTICAL COMPOSITION FOR PREVENTING OR TREATING BETA-AMYLOID OR TAU PROTEIN-ASSOCIATED DISEASES CONTAINING SAME**

(30) Priority: 26.12.2019 KR 20190175321
(71) Applicant: Yonsei University, University-Industry Foundation(UIF)., Seoul 03722 (KR); Daegu-Gyeongbuk Medical Innovation Foundation, Daegu 41061 (KR)
(72) Inventor: KIM, Youngsoo, Incheon 21982 (KR); SHIN, Jisu, Incheon 21022 (KR); HONG, Ki Bum, Daegu 42286 (KR); YU, Ji Hoon, Daegu 41061 (KR); LEE, Ji Hoon, Seoul 04403 (KR); KIM, Da Rong, Daegu 42251 (KR); JEON, Hui-Jeon, Daegu 41061 (KR); SONG, Jaeyoung, Daegu 41061 (KR); PARK, Jin Wan, Cheongju-si, Chungcheongbuk-do 28165 (KR); LEE, Ju Suk, Daegu 41076 (KR); LEE, Won Seok, Seoul 08209 (KR); KIM, Young-Kyu, Daegu 41072 (KR); KIM, Sung Hwan, Daegu 41782 (KR); YOON, Heeseok, Daegu 41072 (KR)
(74) Representative: Schön, Christoph
(86) International application number: PCT/KR2020/018932
(87) International publication number: WO 2021/133037

(57) **Abstract**

Provided are: a novel pyrrolidine derivative or a pharmaceutically acceptable salt thereof; and a pharmaceutical composition containing same and having the effects of inhibiting beta-amyloid and/or tau protein aggregation, and/or breaking down beta-amyloid and/or tau protein aggregates, and/or preventing and/or treating beta-amyloid and/or tau protein-associated diseases.

## Description

### [Technical Field]

The present invention relates to a novel pyrrolidine derivative or a pharmaceutically acceptable salt thereof, a pharmaceutical composition containing the same and having effects of inhibiting aggregation of beta-amyloids and/or tau proteins, degrading aggregates of beta-amyloids and/or tau proteins, and/or preventing and/or treating beta-amyloid- and/or tau protein-related diseases, and a proteolysis-targeting chimera (PROTAC) compound containing the pyrrolidine derivative or a pharmaceutically acceptable salt thereof.

### [Background Art]

An important pathological feature of Alzheimer's disease, which is a representative neurodegenerative disease, is the formation of peptide aggregates called "senile plaques", which causes synaptic dysfunction and neuronal death. The main component of these senile plaques is beta-amyloid (Aβ), which is 40 to 42 amino acids in length. Beta-amyloid monomers are easily self-assembled into oligomers, protofibrils and beta-sheet-rich fibers, and are related to the pathogenesis of neurotoxicity.

In addition, recently, because the tau proteins have been known to have abnormal structures and form inclusion bodies, which cause neurodegenerative diseases, they are emerging as a novel target for these diseases. Tau proteins are required in order to stabilize microtubules, and excessive accumulation of tau proteins is known to cause microtubular collapse and disruption of normal neuronal networks. A disease characterized by accumulation of tau proteins and aggregation of neuronal cells is called "tauopathy", which is considered to be cause various neurodegenerative diseases.

Therefore, there is a need for the development of a drug that is capable of inhibiting aggregation (accumulation) of beta-amyloids and/or tau proteins or degrading aggregates thereof and is thus applicable to the treatment of neurodegenerative diseases.

### [Disclosure]

### [Technical Problem]

Therefore, the present invention has been made in view of the above problems of the prior art, and it is one object of the present invention to provide a novel compound or a pharmaceutically acceptable salt thereof that is applicable to the treatment of neurodegenerative diseases due to the activity of inhibiting the aggregation (accumulation) of beta-amyloids and/or tau proteins or decomposing the aggregates thereof, a pharmaceutical or food composition containing the same and the use of the compound.

It is another object of the present invention to provide a proteolysis-targeting chimera (PROTAC) compound containing the novel compound or a pharmaceutically acceptable salt thereof, a pharmaceutical or food composition containing the same, and the use of the compound.

### [Technical Solution]

In accordance with one aspect of the present invention, the above and other objects can be accomplished by the provision of a pyrrolidine derivative represented by the following Formula I, II, III, IV, or V, or a pharmaceutically acceptable salt thereof: 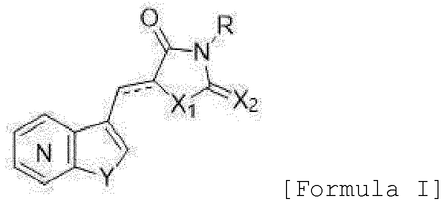 in Formula I, X₁ and X₂ are the same as or different from each other, and are each independently NH, O, or S, Y is NH or O, and R is H or an alkyl group having 1 to 6 carbon atoms ("N" in the 6-membered ring means that any one randomly selected from the 6 carbons constituting the ring is substituted); 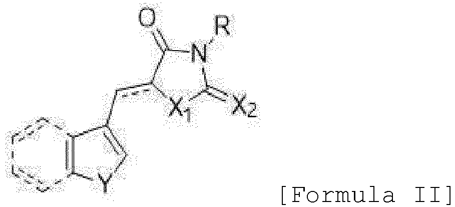 in Formula II, X₁ and X₂ are the same as or different from each other, and are each independently NH, O, or S, Y is NH, O, or S, and R is H or an alkyl group having 1 to 6 carbon atoms; 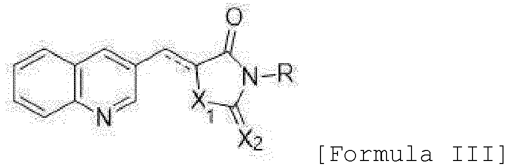 in Formula III, X₁ and X₂ are the same as or different from each other, and are each independently NH, O or S (e.g., X₁ is NH or S, and X₂ is NH, O, or S), and R is H or an alkyl group having 1 to 6 carbon atoms; 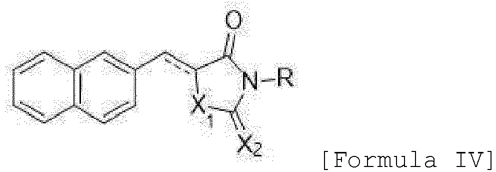 in Formula IV, X₁ and X₂ are the same as or different from each other, and are each independently NH, O or S (e.g., X₁ is NH or S, and X₂ is O or S), and R is H or an alkyl group having 1 to 6 carbon atoms; and 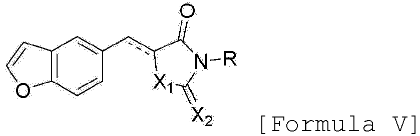 in Formula V, X₁ and X₂ are the same as or different from each other, and are each independently NH, O, or S (e.g., X₁ is NH or S, and X₂ is O or S), and R is H or an alkyl group having 1 to 6 carbon atoms.

In another embodiment, the present invention provides a pharmaceutical composition for inhibiting aggregation of beta-amyloids and/or tau proteins containing at least one selected from the group consisting of pyrrolidine derivatives represented by Formulas I, II, III, IV, and V, and pharmaceutically acceptable salts thereof as an active ingredient.

In another embodiment, the present invention provides a method for inhibiting aggregation of beta-amyloids and/or tau proteins including administering a pharmaceutically effective amount of at least one selected from the group consisting of pyrrolidine derivatives represented by Formulas I, II, III, IV, and V, and pharmaceutically acceptable salts thereof to a patient in need of inhibition of aggregation of beta-amyloids and/or tau proteins.

In another embodiment, the present invention provides the use of at least one selected from the group consisting of pyrrolidine derivatives represented by Formulas I, II, III, IV, and V and pharmaceutically acceptable salts thereof for inhibition of aggregation of beta-amyloids and/or tau proteins, or for preparation of a composition for inhibiting aggregation of beta-amyloids and/or tau proteins.

In another embodiment, the present invention provides a pharmaceutical composition for degrading aggregates of beta-amyloids and/or tau proteins containing at least one selected from the group consisting of pyrrolidine derivatives represented by Formulas I, II, III, IV, and V and pharmaceutically acceptable salts thereof as an active ingredient.

In another embodiment, the present invention provides a method for degrading aggregates of beta-amyloids and/or tau proteins including administering a pharmaceutically effective amount of at least one selected from the group consisting of pyrrolidine derivatives represented by Formulas I, II, III, IV, and V and pharmaceutically acceptable salts thereof to a patient in need of degradation of aggregates of beta-amyloids and/or tau proteins.

In another embodiment, the present invention provides the use of at least one selected from the group consisting of pyrrolidine derivatives represented by Formulas I, II, III, IV, and V and pharmaceutically acceptable salts thereof for degradation of aggregates of beta-amyloids and/or tau proteins, or for preparation of a composition for degrading aggregates of beta-amyloids and/or tau proteins.

In another embodiment, the present invention provides a pharmaceutical composition for protecting neurons containing at least one selected from the group consisting of pyrrolidine derivatives represented by Formulas I, II, III, IV, and V and pharmaceutically acceptable salts thereof as an active ingredient. The protection of neurons includes protection of the neurons from damage attributable to aggregation (accumulation) of beta-amyloids and/or tau proteins.

In another embodiment, the present invention provides a method for protecting neurons including administering a pharmaceutically effective amount of at least one selected from the group consisting of pyrrolidine derivatives represented by Formulas I, II, III, IV, and V and pharmaceutically acceptable salts thereof to a patient in need of protection of neurons.

In another embodiment, the present invention provides the use of at least one selected from the group consisting of pyrrolidine derivatives represented by Formulas I, II, III, IV, and V and pharmaceutically acceptable salts thereof for protection of neurons, or for preparation of a composition for protecting neurons.

In another embodiment, the present invention provides a composition for preventing and/or treating beta-amyloid- and/or tau protein-related diseases containing at least one selected from the group consisting of pyrrolidine derivatives represented by Formulas I, II, III, IV, and V and pharmaceutically acceptable salts thereof as an active ingredient.

In another embodiment, the present invention provides a health functional food for preventing and/or ameliorating beta-amyloid- and/or tau protein-related diseases containing at least one selected from the group consisting of pyrrolidine derivatives represented by Formulas I, II, III, IV, and V and pharmaceutically acceptable salts thereof as an active ingredient.

In another embodiment, the present invention provides a method for preventing and/or treating beta-amyloid- and/or tau protein-related diseases including administering a pharmaceutically effective amount of at least one selected from the group consisting of pyrrolidine derivatives represented by Formulas I, II, III, IV, and V and pharmaceutically acceptable salts thereof to a patient in need of prevention and/or treatment of beta-amyloid- and/or tau protein-related diseases.

In another embodiment, the present invention provides the use of at least one selected from the group consisting of pyrrolidine derivatives represented by Formulas I, II, III, IV, and V and pharmaceutically acceptable salts thereof for prevention and/or treatment of beta-amyloid- and/or tau protein-related diseases, or for preparation of a composition for preventing and/or treating beta-amyloid- and/or tau protein-related diseases.

The beta-amyloid- and/or tau protein-related disease may be selected from amyloid diseases, tauopathies, and beta-amyloid- and/or tau protein-related neurodegenerative diseases, for example, the beta-amyloid- and/or tau protein-related disease may be selected from dementia (e.g., Alzheimer's disease, vascular dementia, etc.), mild cognitive impairment, cerebral amyloid angiopathy, Down's syndrome, amyloid stroke, systemic amyloid disease, Dutch amyloidosis, tauopathy, dementia with Lewy bodies (DLB), multi-infarct dementia (MID), frontotemporal lobar degeneration (FTLD), Pick's disease, corticobasal degeneration (CBD), progressive supranuclear palsy (PSP), Parkinson's disease, Huntington's disease, and the like.

### [Advantageous effects]

The pyrrolidine derivative represented by Formula I, II, III, IV, or V, or a pharmaceutically acceptable salt thereof disclosed herein is highly effective in inhibiting aggregation of beta-amyloids and/or tau proteins and/or degrading the aggregates of beta-amyloids and/or tau proteins, and is thus highly useful for preventing and/or treating neurological diseases caused by aggregation and/or accumulation of beta-amyloids and/or tau proteins.

### [Description of Drawings]

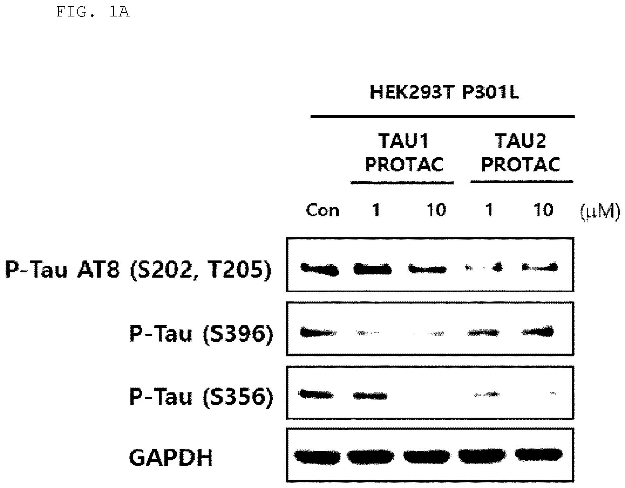

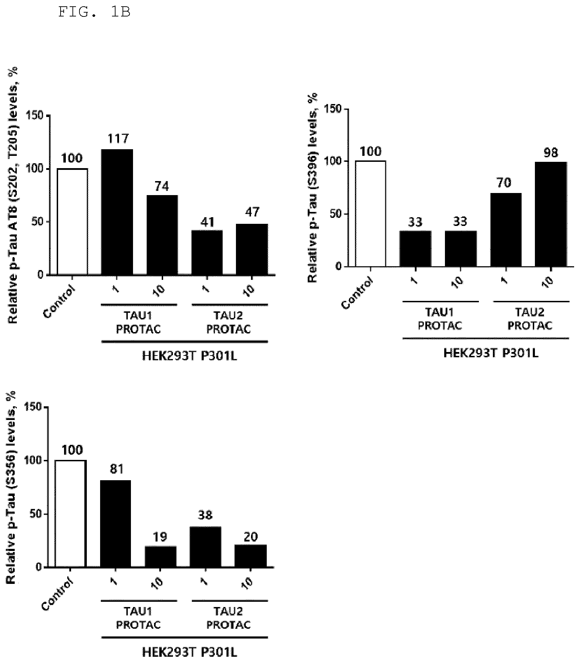

FIGS. 1A and 1B are diagrams identifying that TAU-1 and TAU-2 PROTAC compounds containing the compound according to the present invention exhibit degradation activity in three types of phosphorylated tau proteins. Specifically, FIG. 1A is an image obtained using ImageQuant LAS 4000 (GE healthcare, IL, USA) by western blotting with respect to the tau degradation activity of TAU-1 and TAU-2, and FIG. 1B shows the result of quantification of the image using Image J program.

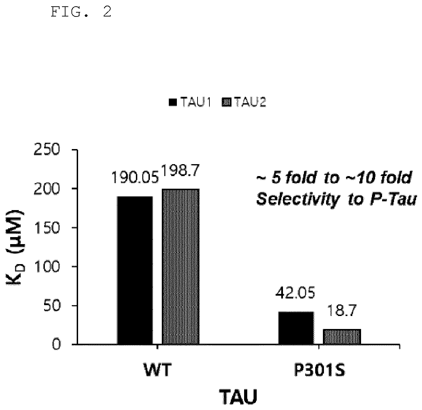

FIG. 2 is a graph showing the binding affinity (K_{d}) of TAU-1 and TAU-2 PROTAC compounds containing the compound according to the present invention to a nonpathological WT tau protein and a pathological P301S tau protein.

### [Best Mode]

Hereinafter, the present invention disclosed herein will be described in more detail.

First, one embodiment of the present invention provides a pyrrolidine derivative represented by the following Formula I, II, III, IV, or V, or a pharmaceutically acceptable salt thereof: 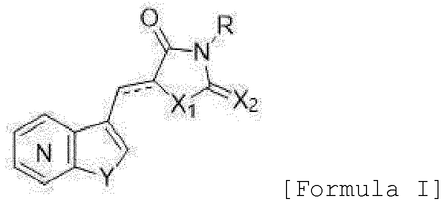 in Formula I, X₁ and X₂ are the same as or different from each other, and are each independently NH, O, or S, Y is NH or O, and R is H or an alkyl group having 1 to 6 carbon atoms ("N" in the 6-membered ring means that any one randomly selected from the 6 carbons constituting the ring is substituted); 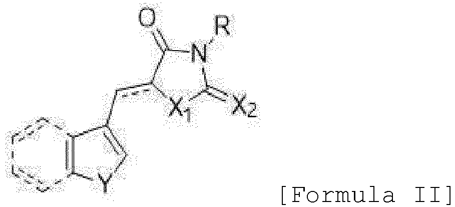 in Formula II, X₁ and X₂ are the same as or different from each other, and are each independently NH, O, or S, Y is NH, O, or S, and R is H or an alkyl group having 1 to 6 carbon atoms; 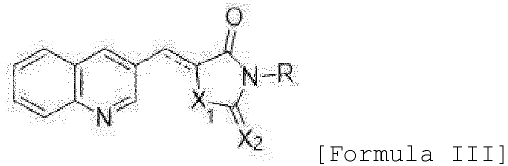 in Formula III, X₁ and X₂ are the same as or different from each other, and are each independently NH or S (e.g., X₁ is NH or S, and X₂ is NH, O, or S), and R is H or an alkyl group having 1 to 6 carbon atoms; 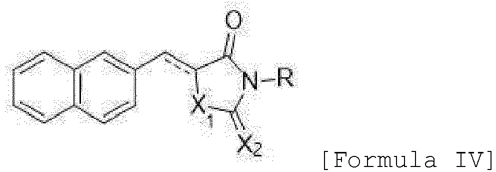 in Formula IV, X₁ and X₂ are the same as or different from each other, and are each independently NH, O or S (e.g., X₁ is NH or S, and X₂ is O or S), and R is H or an alkyl group having 1 to 6 carbon atoms; and 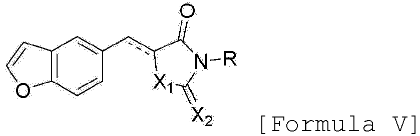 in Formula V, X₁ and X₂ are the same as or different from each other, and are each independently NH, O, or S (e.g., X₁ is NH or S, and X₂ is O or S), and R is H or an alkyl group having 1 to 6 carbon atoms.

Specifically, in Formulas I to V, R may be H or an alkyl having 1 to 4 carbon atoms, and more specifically, R may be H, methyl, ethyl or isopropyl.

Specifically, 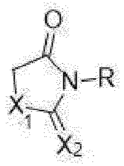 in Formulas I to V may be 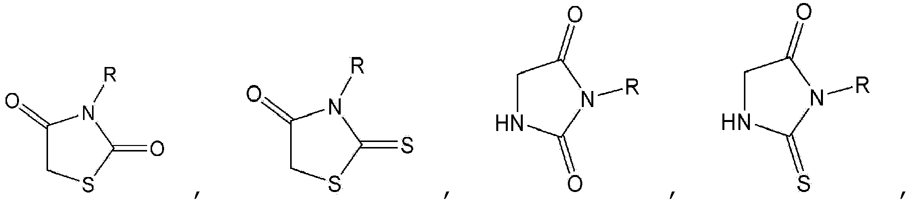 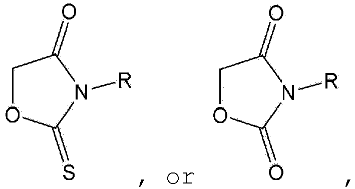 but is not limited thereto.

Specifically, 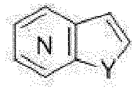 of Formula I may be 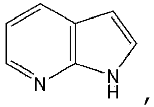 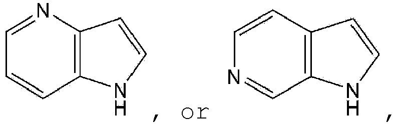 but is not limited thereto.

Specifically, 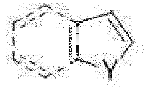 in Formula II may be 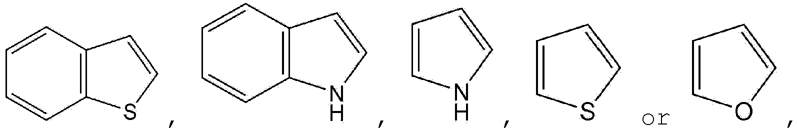 but is not limited thereto.

Specifically, the pyrrolidine derivative may be selected from the group consisting of the compounds set forth in Table 1 below.

**[Table 1]**

| No. | Compound name | Structural Formula | common name of compound | Formula |
|---|---|---|---|---|
| 1 | DN204284 | 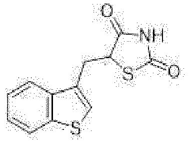 | (Z)-5-(benzo[b]thiophen-3-ylmethylene)thiazolidine-2,4-dione | II |
| 2 | DN204286 | 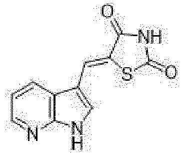 | (Z)-5-((1H-pyrrolo[2,3-b]pyridin-3-yl)methylene)thiazolidine-2,4-dione | I |
| 3 | DN204289 | 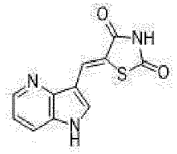 | (Z)-5-((1H-pyrrolo[3,2-b]pyridin-3-yl)methylene)thiazolidine-2,4-dione | I |
| 4 | DN204290 | 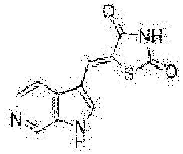 | (Z)-5-((1H-pyrrolo[2,3-c]pyridin-3-yl)methylene)thiazolidine-2,4-dione | I |
| 5 | DN204291 | 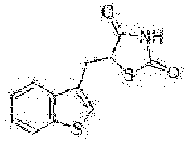 | 5-(benzo[b]thiophen-3-ylmethyl)thiazolidine-2,4-dione | II |
| 6 | DN204300 | 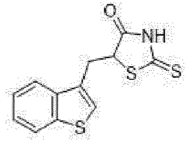 | 5-(benzo[b]thiophen-3-ylmethyl)-2-thioxothiazolidin-4-one | II |
| 7 | DN204304 | 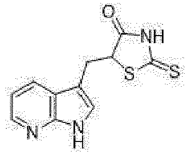 | 5-((1H-pyrrolo[2,3-b]pyridin-3-yl)methyl)-2-thioxothiazolidin-4-one | I |
| 8 | DN204807 | 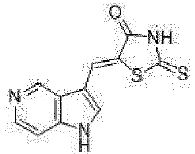 | (Z)-5-((1H-pyrrolo[3,2-c]pyridin-3-yl)methylene)-2-thioxothiazolidin-4-one | I |
| 9 | DN204808 | 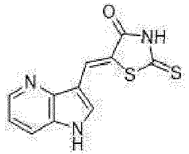 | (Z)-5-((1H-pyrrolo[3,2-b]pyridin-3-yl)methylene)-2-thioxothiazolidin-4-one | I |
| 10 | DN204810 | 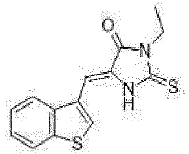 | (Z)-5-(benzo[b]thiophen-3-ylmethylene)-3-ethyl-2-thioxoimidazolidin-4-one | II |
| 11 | DN204816 | 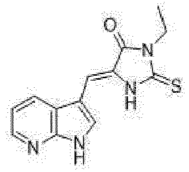 | (Z)-5-((1H-pyrrolo[2,3-b]pyridin-3-yl)methylene)-3-ethyl-2-thioxoimidazolidin-4-one | I |
| 12 | DN204817 | 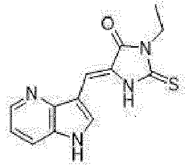 | (Z)-5-((1H-pyrrolo[3,2-b]pyridin-3-yl)methylene)-3-ethyl-2-thioxoimidazolidin-4-one | I |
| 13 | DN204671 | 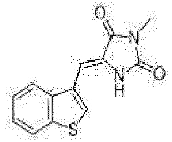 | (Z)-5-(benzo[b]thiophen-3-ylmethylene)-3-methylimidazolidine-2,4-dione | II |
| 14 | DN204673 | 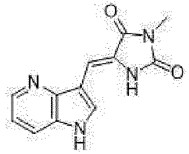 | (Z)-5-((1H-pyrrolo[3,2-b]pyridin-3-yl)methylene)-3-methylimidazolidine-2,4-dione | I |
| 15 | DN205111 | 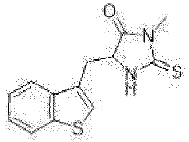 | 5-(benzo[b]thiophen-3-ylmethyl)-3-methyl-2-thioxoimidazolidin-4-one | II |
| 16 | DN205355 | 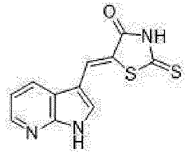 | (Z)-5-((1H-pyrrolo[2,3-b]pyridin-3-yl)methylene)-2-thioxothiazolidin-4-one | I |
| 17 | DN204288 | 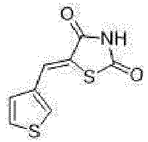 | (Z)-5-(thiophen-3-ylmethylene)thiazolidine-2,4-dione | II |
| 18 | DN204292 | 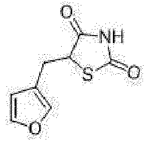 | 5-(furan-3-ylmethyl)thiazolidine-2,4-dione | II |
| 19 | DN204293 | 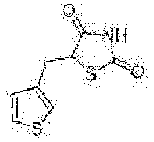 | 5-(thiophen-3-ylmethyl)thiazolidine-2,4-dione | II |
| 20 | DN204294 | 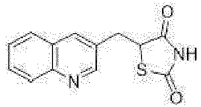 | 5-(quinolin-3-ylmethyl)thiazolidine-2,4-dione | III |
| 21 | DN204307 | 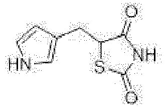 | 5-((1H-pyrrol-3-yl)methyl)thiazolidine-2,4-dione | II |
| 22 | DN204297 | 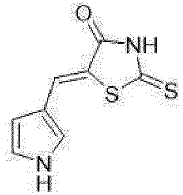 | (Z)-5-((1H-pyrrol-3-yl)methylene)-2-thioxothiazolidin-4-one | II |
| 23 | DN204302 | 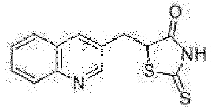 | 5-(quinolin-3-ylmethyl)-2-thioxothiazolidin-4-one | III |
| 24 | DN204303 | 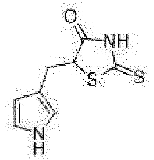 | 5-((1H-pyrrol-3-yl)methyl)-2-thioxothiazolidin-4-one | II |
| 25 | DN204305 | 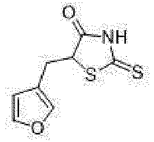 | 5-(furan-3-ylmethyl)-2-thioxothiazolidin-4-one | II |
| 26 | DN204812 | 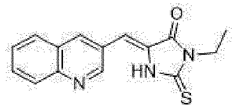 | (Z)-3-ethyl-5-(quinolin-4-ylmethylene)-2-thioxoimidazolidin-4-one | III |
| 27 | DN204813 | 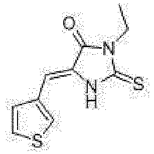 | (Z)-3-ethyl-5-(thiophen-3-ylmethylene)-2-thioxoimidazolidin-4-one | II |
| 28 | DN204814 | 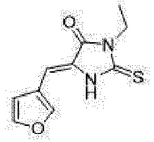 | (Z)-3-ethyl-5-(furan-3-ylmethylene)-2-thioxoimidazolidin-4-one | II |
| 29 | DN204819 | 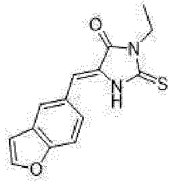 | (Z)-5-(benzofuran-5-ylmethylene)-3-ethyl-2-thioxoimidazolidin-4-one | V |
| 30 | DN204669 | 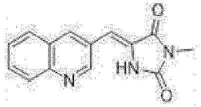 | (Z)-5-(isoquinolin-4-ylmethylene)-3-methylimidazolidine-2,4-dione | III |
| 31 | DN204670 | 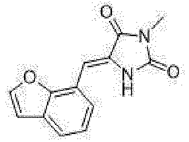 | (Z)-5-(benzofuran-7-ylmethylene)-3-methylimidazolidine-2,4-dione | V |
| 32 | DN205106 | 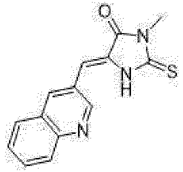 | (Z)-3-methyl-5-(quinolin-3-ylmethylene)imidazolidine-2,4-dione | III |
| 33 | DN205108 | 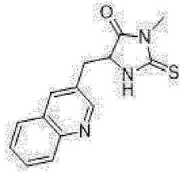 | 3-methyl-5-(quinolin-3-ylmethyl)imidazolidine-2,4-dione | III |
| 34 | DN205109 | 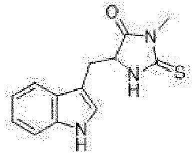 | 5-((1H-indol-3-yl)methyl)-3-methylimidazolidine-2,4-dione | II |
| 35 | DN205110 | 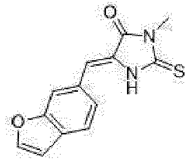 | (Z)-5-(benzofuran-6-ylmethylene)-3-methylimidazolidine-2,4-dione | V |
| 36 | DN205353 | 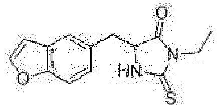 | 5-(benzofuran-5-ylmethyl)-3-ethyl-2-thioxoimidazolidin-4-one | V |
| 37 | DN205354 | 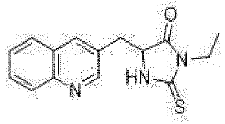 | 3-ethyl-5-(quinolin-4-ylmethyl)-2-thioxoimidazolidin-4-one | III |
| 38 | DN204818 | 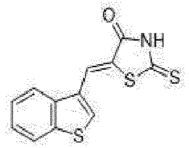 | (Z)-5-(benzo[b]thiophen-3-ylmethylene)-2-thioxothiazolidin-4-one | II |
| 39 | DN204285 | 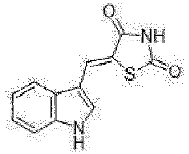 | (Z)-5-((1H-indol-3-yl)methylene)thiazolidine-2,4-dione | II |
| 40 | DN204295 | 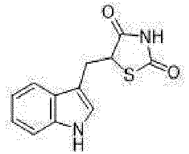 | 5-((1H-indol-3-yl)methyl)thiazolidine-2,4-dione | II |
| 41 | DN204301 | 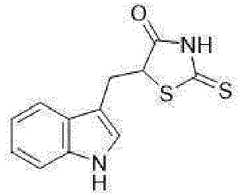 | 5-((1H-indol-3-yl)methyl)-2-thioxothiazolidin-4-one | II |
| 42 | DN204811 | 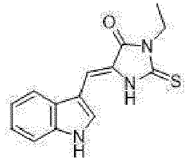 | (Z)-5-((1H-indol-3-yl)methylene)-3-ethyl-2-thioxoimidazolidin-4-one | II |
| 43 | DN204815 | 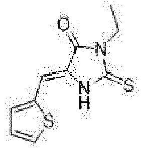 | (Z)-3-ethyl-5-(thiophen-2-ylmethylene)-2-thioxoimidazolidin-4-one | II |
| 44 | DN204672 | 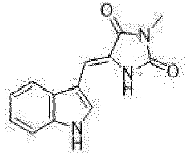 | (Z)-5-((1H-indol-3-yl)methylene)-3-methylimidazolidine-2,4-dione | II |
| 45 | DN205779 | 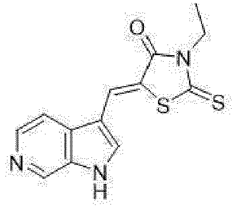 | (Z)-5-((1H-pyrrolo[2,3-c]pyridin-3-yl)methylene)-3-ethyl-2-thioxothiazolidin-4-one | I |
| 46 | DN205780 | 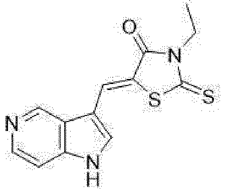 | (Z)-5-((1H-pyrrolo[3,2-c]pyridin-3-yl)methylene)-3-ethyl-2-thioxothiazolidin-4-one | I |
| 47 | DN205781 | 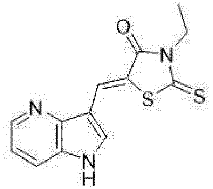 | (Z)-5-((1H-pyrrolo[3,2-b]pyridin-3-yl)methylene)-3-ethyl-2-thioxothiazolidin-4-one | I |
| 48 | DN205782 | 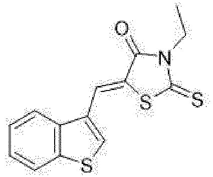 | (Z)-5-(benzo[b]thiophen-3-ylmethylene)-3-ethyl-2-thioxothiazolidin-4-one | II |
| 49 | DN205783 | 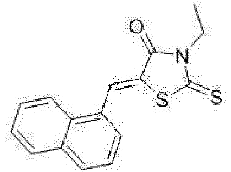 | (Z)-3-ethyl-5-(naphthalen-2-ylmethylene)-2-thioxothiazolidin-4-one | IV |
| 50 | DN205821 | 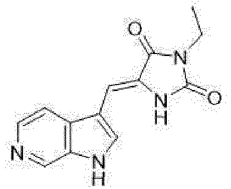 | (Z)-5-((1H-pyrrolo[2,3-c]pyridin-3-yl)methylene)-3-ethylimidazolidine-2,4-dione | I |
| 51 | DN205822 | 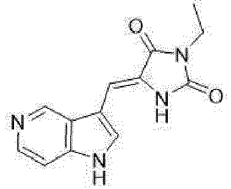 | (Z)-5-((1H-pyrrolo[3,2-c]pyridin-3-yl)methylene)-3-ethylimidazolidine-2,4-dione | I |
| 52 | DN205826 | 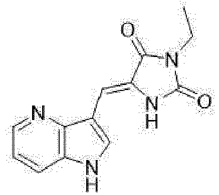 | (Z)-5-((1H-pyrrolo[3,2-b]pyridin-3-yl)methylene)-3-ethylimidazolidine-2,4-dione | I |
| 53 | DN205827 | 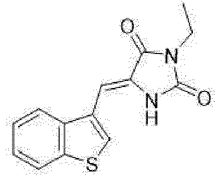 | (Z)-5-(benzo[b]thiophen-3-ylmethylene)-3-ethylimidazolidine-2,4-dione | II |
| 54 | DN205828 | 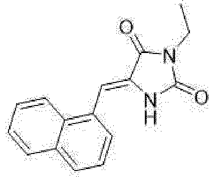 | (Z)-3-ethyl-5-(naphthalen-2-ylmethylene)imidazolidine-2,4-dione | IV |
| 55 | DN204287 | 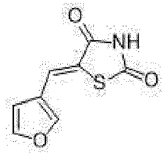 | (Z)-5-(furan-3-ylmethylene)thiazolidine-2,4-dione | II |
| 56 | DN204296 | 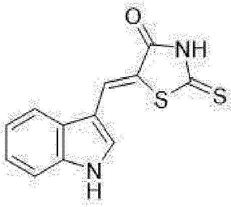 | (Z)-5-((1H-indol-3-yl)methylene)-2-thioxothiazolidin-4-one | II |
| 57 | DN204298 | 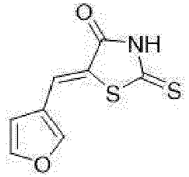 | (Z)-5-(furan-3-ylmethylene)-2-thioxothiazolidin-4-one | II |
| 58 | DN204299 | 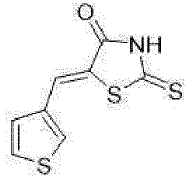 | (Z)-5-(thiophen-3-ylmethylene)-2-thioxothiazolidin-4-one | II |
| 59 | DN204306 | 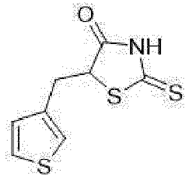 | 5-(thiophen-3-ylmethyl)-2-thioxothiazolidin-4-one | II |
| 60 | DN204809 | 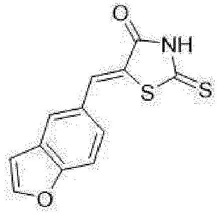 | (Z)-5-(benzofuran-5-ylmethylene)-2-thioxothiazolidin-4-one | V |
| 61 | DN205107 | 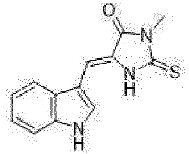 | (Z)-5-((1H-indol-3-yl)methylene)-3-methylimidazolidine-2,4-dione | II |
| 62 | DN207294 | 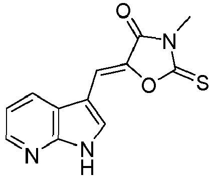 | (Z)-5-((1H-pyrrolo[2,3-b]pyridin-3-yl)methylene)-3-methyl-2-thioxooxazolidin-4-one | I |
| 63 | DN207295 | 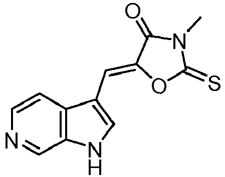 | (Z)-5-((1H-pyrrolo[2,3-c]pyridin-3-yl)methylene)-3-methyl-2-thioxooxazolidin-4-one | I |
| 64 | DN207296 | 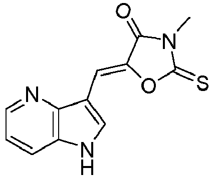 | (Z)-5-((1H-pyrrolo[3,2-b]pyridin-3-yl)methylene)-3-methyl-2-thioxooxazolidin-4-one | I |
| 65 | DN207297 | 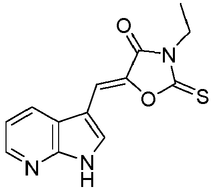 | (Z)-5-((1H-pyrrolo[2,3-b]pyridin-3-yl)methylene)-3-ethyl-2-thioxooxazolidin-4-one | I |
| 66 | DN207298 | 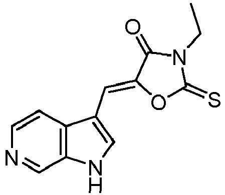 | (Z)-5-((1H-pyrrolo[2,3-c]pyridin-3-yl)methylene)-3-ethyl-2-thioxooxazolidin-4-one | I |
| 67 | DN207299 | 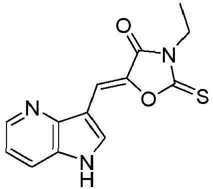 | (Z)-5-((1H-pyrrolo[3,2-b]pyridin-3-yl)methylene)-3-ethyl-2-thioxooxazolidin-4-one | I |
| 68 | DN207300 | 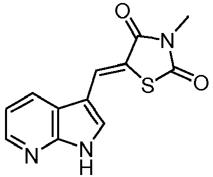 | (Z)-5-((1H-pyrrolo[2,3-b]pyridin-3-yl)methylene)-3-methylthiazolidine-2,4-dione | I |
| 69 | DN207301 | 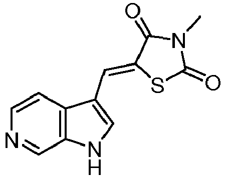 | (Z)-5-((1H-pyrrolo[2,3-c]pyridin-3-yl)methylene)-3-methylthiazolidine-2,4-dione | I |
| 70 | DN207302 | 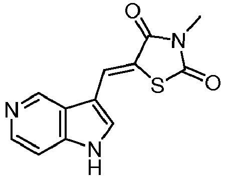 | (Z)-5-((1H-pyrrolo[3,2-c]pyridin-3-yl)methylene)-3-methylthiazolidine-2,4-dione | I |
| 71 | DN207303 | 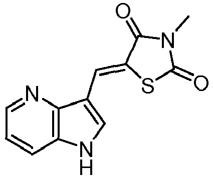 | (Z)-5-((1H-pyrrolo[3,2-b]pyridin-3-yl)methylene)-3-methylthiazolidine-2,4-dione | I |
| 72 | DN207304 | 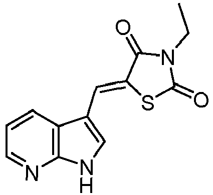 | (Z)-5-((1H-pyrrolo[2,3-b]pyridin-3-yl)methylene)-3-ethylthiazolidine-2,4-dione | I |
| 73 | DN207305 | 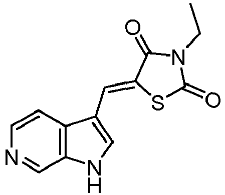 | (Z)-5-((1H-pyrrolo[2,3-c]pyridin-3-yl)methylene)-3-ethylthiazolidine-2,4-dione | I |
| 74 | DN207049 | 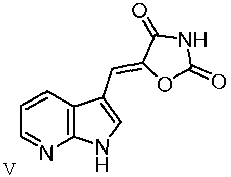 | (Z)-5-((1H-pyrrolo[2,3-b]pyridin-3-yl)methylene)oxazolidine-2,4-dione | I |
| 75 | DN207111 | 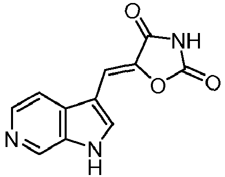 | (Z)-5-((1H-pyrrolo[2,3-c]pyridin-3-yl)methylene)oxazolidine-2,4-dione | I |
| 76 | DN207109 | 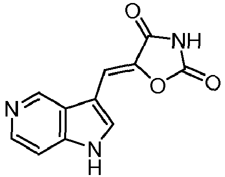 | (Z)-5-((1H-pyrrolo[3,2-c]pyridin-3-yl)methylene)oxazolidine-2,4-dione | I |
| 77 | DN207110 | 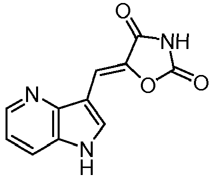 | (Z)-5-((1H-pyrrolo[3,2-b]pyridin-3-yl)methylene)oxazolidine-2,4-dione | I |
| 78 | DN207112 | 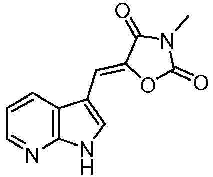 | (Z)-5-((1H-pyrrolo[2,3-b]pyridin-3-yl)methylene)-3-methyloxazolidine-2,4-dione | I |
| 79 | DN207113 | 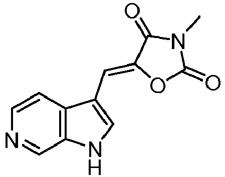 | (Z)-5-((1H-pyrrolo[2,3-c]pyridin-3-yl)methylene)-3-methyloxazolidine-2,4-dione | I |
| 80 | DN207156 | 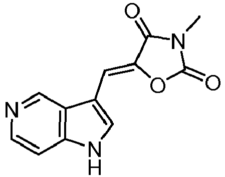 | (Z)-5-((1H-pyrrolo[3,2-c]pyridin-3-yl)methylene)-3-methyloxazolidine-2,4-dione | I |
| 81 | DN207114 | 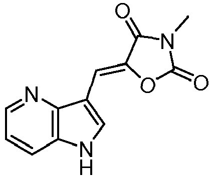 | (Z)-5-((1H-pyrrolo[3,2-b]pyridin-3-yl)methylene)-3-methyloxazolidine-2,4-dione | I |
| 82 | DN207157 | 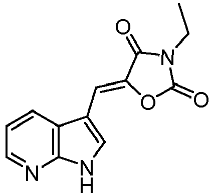 | (Z)-5-((1H-pyrrolo[2,3-b]pyridin-3-yl)methylene)-3-ethyloxazolidine-2,4-dione | I |
| 83 | DN207129 | 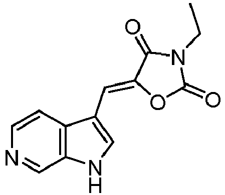 | (Z)-5-((1H-pyrrolo[2,3-c]pyridin-3-yl)methylene)-3-ethyloxazolidine-2,4-dione | I |
| 84 | DN207130 | 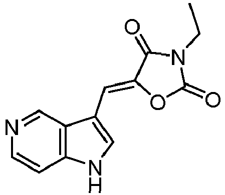 | (Z)-5-((1H-pyrrolo[3,2-c]pyridin-3-yl)methylene)-3-ethyloxazolidine-2,4-dione | I |
| 85 | DN207131 | 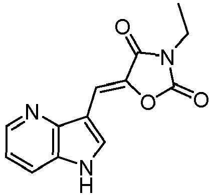 | (Z)-5-((1H-pyrrolo[3,2-b]pyridin-3-yl)methylene)-3-ethyloxazolidine-2,4-dione | I |
| 86 | DN207115 | 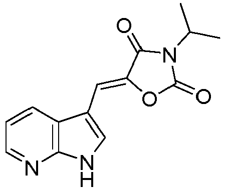 | (Z)-5-((1H-pyrrolo[2,3-b]pyridin-3-yl)methylene)-3-isopropyloxazolidine-2,4-dione | I |
| 87 | DN207116 | 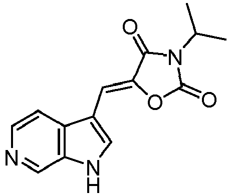 | (Z)-5-((1H-pyrrolo[2,3-c]pyridin-3-yl)methylene)-3-isopropyloxazolidine-2,4-dione | I |
| 88 | DN207117 | 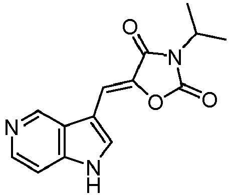 | (Z)-5-((1H-pyrrolo[3,2-c]pyridin-3-yl)methylene)-3-isopropyloxazolidine-2,4-dione | I |
| 89 | DN207118 | 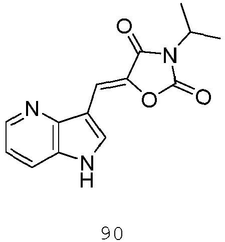 | (Z)-5-((1H-pyrrolo[3,2-b]pyridin-3-yl)methylene)-3-isopropyloxazolidine-2,4-dione | I |
| 90 | DN206760 | 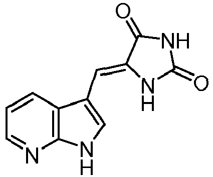 | (Z)-5-((1H-pyrrolo[2,3-b]pyridin-3-yl)methylene)imidazolidine-2,4-dione | I |
| 91 | DN206763 | 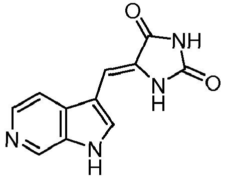 | (Z)-5-((1H-pyrrolo[2,3-c]pyridin-3-yl)methylene)imidazolidine-2,4-dione | I |
| 92 | DN206761 | 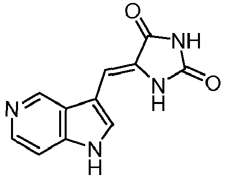 | (Z)-5-((1H-pyrrolo[3,2-c]pyridin-3-yl)methylene)imidazolidine-2,4-dione | I |
| 93 | DN206762 | 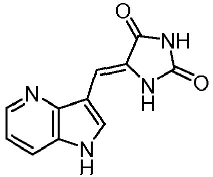 | (Z)-5-((1H-pyrrolo[3,2-b]pyridin-3-yl)methylene)imidazolidine-2,4-dione | I |
| 94 | DN206759 | 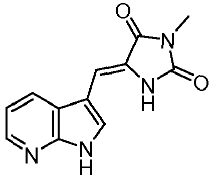 | (Z)-5-((1H-pyrrolo[2,3-b]pyridin-3-yl)methylene)-3-methylimidazolidine-2,4-dione | I |
| 95 | DN206765 | 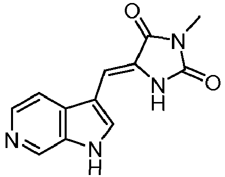 | (Z)-5-((1H-pyrrolo[2,3-c]pyridin-3-yl)methylene)-3-methylimidazolidine-2,4-dione | I |
| 96 | DN206764 | 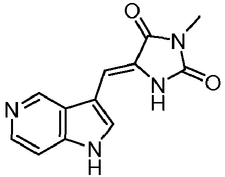 | (Z)-5-((1H-pyrrolo[3,2-c]pyridin-3-yl)methylene)-3-methylimidazolidine-2,4-dione | I |
| 97 | DN207162 | 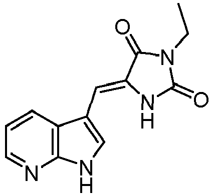 | (Z)-5-((1H-pyrrolo[2,3-b]pyridin-3-yl)methylene)-3-ethylimidazolidine-2,4-dione | I |
| 98 | DN206766 | 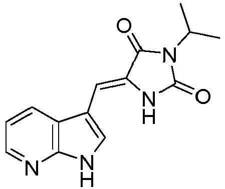 | (Z)-5-((1H-pyrrolo[2,3-b]pyridin-3-yl)methylene)-3-isopropylimidazolidine-2,4-dione | I |
| 99 | DN206769 | 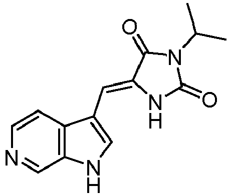 | (Z)-5-((1H-pyrrolo[2,3-c]pyridin-3-yl)methylene)-3-isopropylimidazolidine-2,4-dione | I |
| 100 | DN206767 | 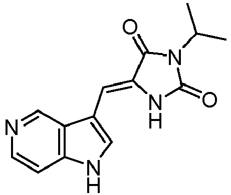 | (Z)-5-((1H-pyrrolo[3,2-c]pyridin-3-yl)methylene)-3-isopropylimidazolidine-2,4-dione | I |
| 101 | DN206768 | 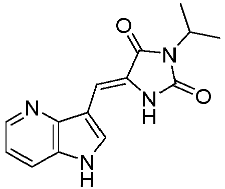 | (Z)-5-((1H-pyrrolo[3,2-b]pyridin-3-yl)methylene)-3-isopropylimidazolidine-2,4-dione | I |
| 102 | DN205355 | 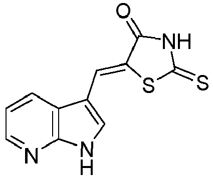 | (Z)-5-((1H-pyrrolo[2,3-b]pyridin-3-yl)methylene)-2-thioxothiazolidin-4-one | I |
| 103 | DN206962 | 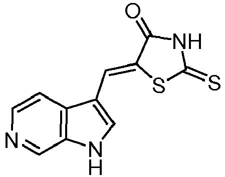 | (Z)-5-((1H-pyrrolo[2,3-c]pyridin-3-yl)methylene)-2-thioxothiazolidin-4-one | I |
| 104 | LJS 022-037 | 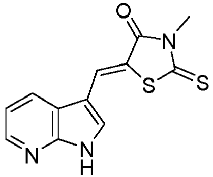 | (Z)-5-((1H-pyrrolo[2,3-b]pyridin-3-yl)methylene)-3-methyl-2-thioxothiazolidin-4-one | I |
| 105 | DN206963 | 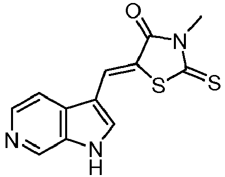 | (Z)-5-((1H-pyrrolo[2,3-c]pyridin-3-yl)methylene)-3-methyl-2-thioxothiazolidin-4-one | I |
| 106 | DN206964 | 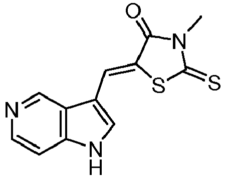 | (Z)-5-((1H-pyrrolo[3,2-c]pyridin-3-yl)methylene)-3-methyl-2-thioxothiazolidin-4-one | I |
| 107 | DN206965 | 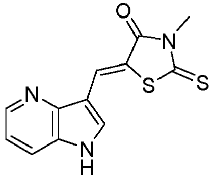 | (Z)-5-((1H-pyrrolo[3,2-b]pyridin-3-yl)methylene)-3-methyl-2-thioxothiazolidin-4-one | I |
| 108 | DN206966 | 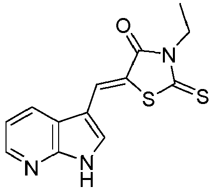 | (Z)-5-((1H-pyrrolo[2,3-b]pyridin-3-yl)methylene)-3-ethyl-2-thioxothiazolidin-4-one | I |
| 109 | DN207045 | 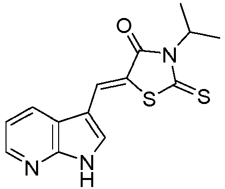 | (Z)-5-((1H-pyrrolo[2,3-b]pyridin-3-yl)methylene)-3-isopropyl-2-thioxothiazolidin-4-one | I |
| 110 | DN207047 | 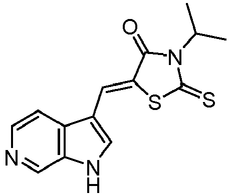 | (Z)-5-((1H-pyrrolo[2,3-c]pyridin-3-yl)methylene)-3-isopropyl-2-thioxothiazolidin-4-one | I |
| 111 | DN207048 | 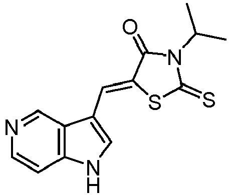 | (Z)-5-((1H-pyrrolo[3,2-c]pyridin-3-yl)methylene)-3-isopropyl-2-thioxothiazolidin-4-one | I |
| 112 | DN207046 | 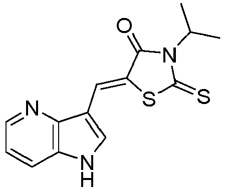 | (Z)-5-((1H-pyrrolo[3,2-b]pyridin-3-yl)methylene)-3-isopropyl-2-thioxothiazolidin-4-one | I |
| 113 | DN207121 | 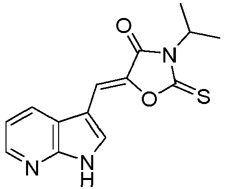 | (Z)-5-((1H-pyrrolo[2,3-b]pyridin-3-yl)methylene)-3-isopropyl-2-thioxooxazolidin-4-one | I |
| 114 | DN207122 | 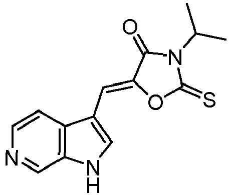 | (Z)-5-((1H-pyrrolo[2,3-c]pyridin-3-yl)methylene)-3-isopropyl-2-thioxooxazolidin-4-one | I |
| 115 | DN207153 | 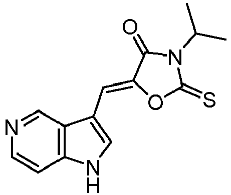 | (Z)-5-((1H-pyrrolo[3,2-c]pyridin-3-yl)methylene)-3-isopropyl-2-thioxooxazolidin-4-one | I |
| 116 | DN207123 | 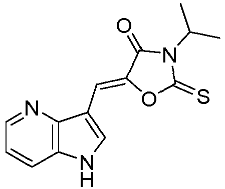 | (Z)-5-((1H-pyrrolo[3,2-b]pyridin-3-yl)methylene)-3-isopropyl-2-thioxooxazolidin-4-one | I |
| 117 | DN207124 | 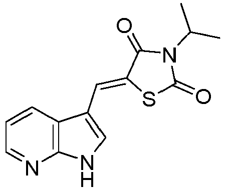 | (Z)-5-((1H-pyrrolo[2,3-b]pyridin-3-yl)methylene)-3-isopropylthiazolidine-2,4-dione | I |
| 118 | DN207125 | 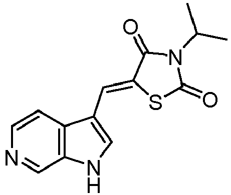 | (Z)-5-((1H-pyrrolo[2,3-c]pyridin-3-yl)methylene)-3-isopropylthiazolidine-2,4-dione | I |
| 119 | DN207126 | 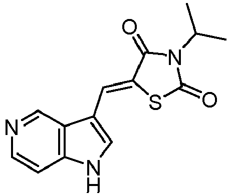 | (Z)-5-((1H-pyrrolo[3,2-c]pyridin-3-yl)methylene)-3-isopropylthiazolidine-2,4-dione | I |
| 120 | DN207127 | 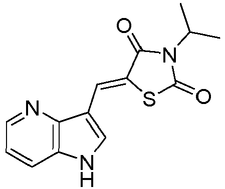 | (Z)-5-((1H-pyrrolo[3,2-b]pyridin-3-yl)methylene)-3-isopropylthiazolidine-2,4-dione | I |
| 121 | DN207155 | 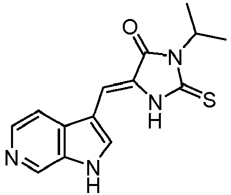 | (Z)-5-((1H-pyrrolo[2,3-c]pyridin-3-yl)methylene)-3-isopropyl-2-thioxoimidazolidin-4-one | I |
| 122 | DN207127 | 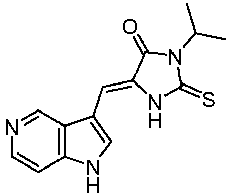 | (Z)-5-((1H-pyrrolo[3,2-c]pyridin-3-yl)methylene)-3-isopropyl-2-thioxoimidazolidin-4-one | I |
| 123 | DN207128 | 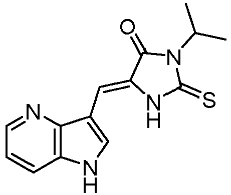 | (Z)-5-((1H-pyrrolo[3,2-b]pyridin-3-yl)methylene)-3-isopropyl-2-thioxoimidazolidin-4-one | I |

The pharmaceutically acceptable salt may be selected from acid salts, basic salts, additional salts of acids or bases, and stereochemical/optical isomers of the pyrrolidine derivative described above. The salts include all inorganic salts and organic salts that maintain the activity of the parent compound in the subject to be administered and do not cause undesirable effects, and are not particularly limited. The acid salts include inorganic acid salts and organic acid salts and, for example, may be formed using an acid selected from the group consisting of acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzenesulfonic acid, benzoic acid, stearic acid, esylic acid, lactic acid, bicarbonic acid, bisulfuric acid, bitartaric acid, oxalic acid, butyric acid, calcium edetic acid, camsylic acid, carbonic acid, chlorobenzoic acid, citric acid, edetic acid, toluenesulfonic acid, edisylic acid, esylic acid, fumaric acid, gluceptic acid, pamoic acid, gluconic acid, glycolylarsanilic acid, methyl nitric acid, polygalacturonic acid, hexylresorcinol acid, malonic acid, hydrabromic acid, hydrochloric acid, hydroiodoic acid, hydroxynaphthoic acid, isethionic acid, lactobionic acid, mandelic acid, estolic acid, mucic acid, napsylic acid, muconic acid, p-nitromethanesulfonic acid, hexamic acid, pantothenic acid, monohydrogen phosphoric acid, dihydrogen phosphoric acid, salicylic acid, sulfamic acid, sulfanilic acid, methanesulfonic acid, teoclic acid, and the like. In addition, the basic salts may be selected from the group consisting of: alkali and alkaline earth metal salts such as ammonium salts, lithium salts, sodium salts, potassium salts, magnesium salts, calcium salts and the like; salts with organic bases such as benzathine salts, N-methyl-D-glucamine salts, hydrabamine salts and the like; and salts having amino acids such as arginine and lysine. The salt may also be converted to a free form by treatment with an appropriate base or acid. The term "additional salt" includes solvates that can be formed from the pyrrolidine derivatives and salts thereof. Such solvates may include hydrates, alcoholates, and the like.

Meanwhile, human beta-amyloid is a peptide molecule containing about 36 to about 43 amino acids, is a major component of amyloid plaques expressed in the brains of Alzheimer's patients, and is known to be involved in the onset of Alzheimer's disease. Beta-amyloid peptide molecule may be obtained by cleaving an amyloid precursor protein (APP) (UniProtKB P05067) with beta secretase and gamma secretase. The beta-amyloid peptide molecules aggregate to form neurotoxic oligomers to thus cause neurological diseases.

In addition, neurofibrillary tangles (NFT), one of the major known etiological features of Alzheimer's disease, consist of paired helical filaments (PHFs) which result from hyperphosphorylation of tau proteins. The tau proteins exist in the form of alternating isomers, and contain three or four copies of the repeating sequence corresponding to the microtubule-binding domain. Neurofibrillary tangles (NFTs) composed of paired helical filaments (PHFs), which result from hyperphosphorylation of tau proteins, are known as one of the major etiologies of neurological diseases such as Alzheimer's disease. Aggregates of hyperphosphorylated tau proteins make microtubules unstable, resulting in inhibition of several signaling pathways through axons.

The present invention is based on the finding that the pyrrolidine derivatives represented by Formulas I, II, III, IV, and V and pharmaceutically acceptable salts thereof have activity of inhibiting aggregation of beta-amyloids and/or tau proteins and activity of degrading aggregates of beta-amyloids and/or tau proteins.

Accordingly, in one embodiment, the present invention provides the pharmaceutical use of the pyrrolidine derivatives represented by Formulas I, II, III, IV, and V, and pharmaceutically acceptable salts thereof for inhibition of aggregation of beta-amyloids and/or tau proteins, degradation of aggregates of beta-amyloids and/or tau proteins, inhibition of aggregation of beta-amyloids and/or tau proteins, protection of (brain cell)neurons, and prevention and/or treatment of beta-amyloid- and/or tau protein-related diseases.

In one embodiment, the present invention provides a pharmaceutical composition for inhibiting aggregation of beta-amyloids and/or tau proteins containing at least one selected from the group consisting of pyrrolidine derivatives represented by Formulas I, II, III, IV, and V, and pharmaceutically acceptable salts thereof as an active ingredient.

In another embodiment, the present invention provides a method for inhibiting aggregation of beta-amyloids and/or tau proteins including administering a pharmaceutically effective amount of at least one selected from the group consisting of pyrrolidine derivatives represented by Formulas I, II, III, IV, and V, and pharmaceutically acceptable salts thereof to a patient in need of inhibition of aggregation of beta-amyloids and/or tau proteins.

In another embodiment, the present invention provides the use of at least one selected from the group consisting of pyrrolidine derivatives represented by Formulas I, II, III, IV, and V and pharmaceutically acceptable salts thereof for inhibition of aggregation of beta-amyloids and/or tau proteins, or for preparation of a composition for inhibiting aggregation of beta-amyloids and/or tau proteins.

In another embodiment, the present invention provides a pharmaceutical composition for degrading aggregates of beta-amyloids and/or tau proteins containing at least one selected from the group consisting of pyrrolidine derivatives represented by Formulas I, II, III, IV, and V and pharmaceutically acceptable salts thereof as an active ingredient.

In another embodiment, the present invention provides a method for degrading aggregates of beta-amyloids and/or tau proteins including administering a pharmaceutically effective amount of at least one selected from the group consisting of pyrrolidine derivatives represented by Formulas I, II, III, IV, and V and pharmaceutically acceptable salts thereof to a patient in need of degradation of aggregates of beta-amyloids and/or tau proteins.

In another embodiment, the present invention provides the use of at least one selected from the group consisting of pyrrolidine derivatives represented by Formulas I, II, III, IV, and V and pharmaceutically acceptable salts thereof for degradation of aggregates of beta-amyloids and/or tau proteins, or for preparation of a composition for degrading aggregates of beta-amyloids and/or tau proteins.

In another embodiment, the present invention provides a pharmaceutical composition for protecting neurons containing at least one selected from the group consisting of pyrrolidine derivatives represented by Formulas I, II, III, IV, and V and pharmaceutically acceptable salts thereof as an active ingredient. The protection of neurons includes protection of the neurons from damage attributable to aggregation (accumulation) of beta-amyloids and/or tau proteins.

In another embodiment, the present invention provides a method for protecting neurons including administering a pharmaceutically effective amount of at least one selected from the group consisting of pyrrolidine derivatives represented by Formulas I, II, III, IV, and V and pharmaceutically acceptable salts thereof to a patient in need of protection of neurons.

In another embodiment, the present invention provides the use of at least one selected from the group consisting of pyrrolidine derivatives represented by Formulas I, II, III, IV, and V and pharmaceutically acceptable salts thereof for protection of neurons, or for preparation of a composition for protecting neurons.

In another embodiment, the present invention provides a composition for preventing and/or treating beta-amyloid- and/or tau protein-related diseases containing at least one selected from the group consisting of pyrrolidine derivatives represented by Formulas I, II, III, IV, and V and pharmaceutically acceptable salts thereof as an active ingredient.

In another embodiment, the present invention provides a health functional food for preventing and/or ameliorating beta-amyloid- and/or tau protein-related diseases containing at least one selected from the group consisting of pyrrolidine derivatives represented by Formulas I, II, III, IV, and V and pharmaceutically acceptable salts thereof as an active ingredient.

In another embodiment, the present invention provides a method for preventing and/or treating beta-amyloid- and/or tau protein-related diseases including administering a pharmaceutically effective amount of at least one selected from the group consisting of pyrrolidine derivatives represented by Formulas I, II, III, IV, and V and pharmaceutically acceptable salts thereof to a patient in need of prevention and/or treatment of beta-amyloid- and/or tau protein-related diseases.

In another embodiment, the present invention provides the use of at least one selected from the group consisting of pyrrolidine derivatives represented by Formulas I, II, III, IV, and V and pharmaceutically acceptable salts thereof for prevention and/or treatment of beta-amyloid- and/or tau protein-related diseases, or for preparation of a composition for preventing and/or treating beta-amyloid- and/or tau protein-related diseases.

As used herein, the term "beta-amyloid- and/or tau protein-related diseases" is used to encompass all neurological diseases that may be caused by the aggregation and/or accumulation of beta-amyloids and/or tau proteins as described above. Examples of the beta-amyloid- and/or tau protein-related diseases include, but are not limited to, dementia (e.g., Alzheimer's disease, vascular dementia, etc.), mild cognitive impairment, cerebral amyloid angiopathy, Down's syndrome, amyloid stroke, systemic amyloid disease, Dutch amyloidosis, tauopathy, dementia with Lewy bodies (DLB), multi-infarct dementia (MID), frontotemporal lobar degeneration (FTLD), Pick's disease, corticobasal degeneration (CBD), progressive supranuclear palsy (PSP), Parkinson's disease, Huntington's disease, and the like. The beta-amyloid- and/or tau protein-related disease may be selected from all diseases resulting from the aggregation and/or accumulation of beta-amyloids and/or tau proteins.

As used herein, the term "treatment" refers to alleviation or amelioration of pathological symptoms, reduction of the disease site, delay or amelioration of disease progression, amelioration, alleviation or stabilization of disease state or symptoms, partial or complete recovery, prolonged survival and other beneficial treatment results. The term "prevention" is used to encompass all mechanisms and/or effects that act on a subject not having a specific disease to prevent the onset of a specific disease or delay the onset of the disease. The term "protection of neurons" is used to encompass all mechanisms and/or effects of inhibiting damage to and/or death of neurons.

In one embodiment, in addition to the active ingredient (at least one selected from the group consisting of pyrrolidine derivatives represented by Formulas I, II, III, IV, and V and pharmaceutically acceptable salts thereof), the pharmaceutical composition may further contain at least one adjuvant selected from the group consisting of pharmaceutically acceptable carriers, excipients, diluents, fillers, extenders, wetting agents, disintegrants, emulsifiers (surfactants), lubricants, sweeteners, flavoring agents, suspension agents, preservatives, and the like. The adjuvant may be appropriately adjusted depending on the formulation to which the pharmaceutical composition is applied, and may include one or more selected from all adjuvants that may be commonly used in the pharmaceutical field. In one embodiment, the pharmaceutically acceptable carrier is one that is commonly used in drug formulation and includes one or more selected from the group consisting of lactose, dextrose, sucrose, trehalose, arginine, histidine, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil and the like, but is not limited thereto.

An effective amount of the active ingredient (at least one selected from the group consisting of pyrrolidine derivatives represented by Formulas I, II, III, IV, and V and pharmaceutically acceptable salts thereof), or the pharmaceutical composition may be administered orally or parenterally. Parenteral administration may be performed by intravenous injection, subcutaneous injection, intramuscular injection, intraperitoneal injection, intradermal administration, intranasal administration, intrapulmonary administration, rectal administration, or local administration at the lesion site. For oral administration, the pharmaceutical composition may be formulated in a dosage form that protects the active ingredient from degradation in the stomach, or the active ingredient may be coated in order to prevent the active ingredient from degrading in the stomach.

As used herein, the term "pharmaceutically effective amount" refers to a content or dosage of an active ingredient (i.e., at least one selected from the group consisting of pyrrolidine derivatives represented by Formulas I, II, III, IV, and V and pharmaceutically acceptable salts thereof) capable of exhibiting a desired pharmacological effect in the pharmaceutical composition and may be suitably determined depending on factors such as the formulation method, administration method, age, weight, gender, pathological condition, diet, administration time, administration interval, administration route, excretion rate and reaction sensitivity of the patient. For example, the daily or single dose of the active ingredient may be within 0.0001 to 1,000 mg/kg (body weight), 0.001 to 500 mg/kg, 0.01 to 100 mg/kg, 0.1 to 50 mg/kg, or 0.5 to 20 mg/kg, but is not limited thereto. The daily or single dose may be formulated as one formulation in a unit dose form, formulated so as to be divided into appropriate amounts, or packaged in a multi-dose container.

The content of the active ingredient (i.e., at least one selected from the group consisting of pyrrolidine derivatives represented by Formulas I, II, III, IV, and V and pharmaceutically acceptable salts thereof) in the pharmaceutical composition may be appropriately controlled depending on the use form of the pharmaceutical composition, the condition of the patient, the desired effect, and the like, and may be, for example, 0.0001 to 99.9% by weight, 0.001 to 99.9% by weight, 0.01 to 99.9% by weight, 0.1 to 99.9% by weight, 0.5 to 99.9% by weight, 1 to 99.9% by weight, 5 to 99.9% by weight, 10 to 99.9% by weight, 15 to 99.9% by weight, 20 to 99.9% by weight, 25 to 99.9% by weight, 30 to 99.9% by weight, 35 to 99.9% by weight, 40 to 99.9% by weight, 45 to 99.9% by weight, 50 to 99.9% by weight, 55 to 99.9% by weight, 0.0001 to 90% by weight, 0.001 to 90% by weight, 0.01 to 90% by weight, 0.1 to 90% by weight, 0.5 to 90% by weight, 1 to 90% by weight, 5 to 90% by weight, 10 to 90% by weight, 15 to 90% by weight, 20 to 90% by weight, 25 to 90% by weight, 30 to 90% by weight, 35 to 90% by weight, 40 to 90% by weight, 45 to 90% by weight, 50 to 90% by weight, 55 to 90% by weight, 0.0001 to 70% by weight, 0.001 to 70% by weight, 0.01 to 70% by weight, 0.1 to 70% by weight, 0.5 to 70% by weight, 1 to 70% by weight, 5 to 70% by weight, 10 to 70% by weight, 15 to 70% by weight, 20 to 70% by weight, 25 to 70% by weight, 30 to 70% by weight, 35 to 70% by weight, 40 to 70% by weight, 45 to 70% by weight, 50 to 70% by weight, 55 to 70% by weight, 0.0001 to 50% by weight, 0.001 to 50% by weight, 0.01 to 50% by weight, 0.1 to 50% by weight, 0.5 to 50% by weight, 1 to 50% by weight, 5 to 50% by weight, 10 to 50% by weight, 15 to 50% by weight, 20 to 50% by weight, 25 to 50% by weight, 30 to 50% by weight, 35 to 50% by weight, 40 to 50% by weight, 45 to 50% by weight, 0.0001 to 40% by weight, 0.001 to 40% by weight, 0.01 to 40% by weight, 0.1 to 40% by weight, 0.5 to 40% by weight, 1 to 40% by weight, 5 to 40% by weight, 10 to 40% by weight, 15 to 40% by weight, 20 to 40% by weight, 25 to 40% by weight, 30 to 40% by weight, or 35 to 40% by weight, but is not limited thereto.

The pharmaceutical composition may be formulated as a solution, suspension, syrup, or emulsion in an aqueous or oily medium, or may be formulated as a powder, granule, tablet, capsule, or the like, and may further contain a dispersant or stabilizer for formulation.

The subject to which the pharmaceutical composition is administered may be a mammal including a primate including a human, monkey or the like, and a rodent including a mouse, rat or the like, or a cell or tissue isolated from the mammal, or a culture thereof.

The health functional food is any food produced using nutrients that are commonly lacking in daily diets or raw materials or ingredients (hereinafter, referred to as "functional raw materials") that have beneficial functions to the human body, to help to maintain health or prevent and/or ameliorate certain diseases or symptoms. There is no particular restriction on the form of the final product of the health function food. For example, the health functional food may be selected from the group consisting of various types of foods, beverage compositions, food additives, and the like, but is not limited thereto.

The content of the active ingredient (i.e., at least one selected from the group consisting of pyrrolidine derivatives represented by Formulas I, II, III, IV, and V and pharmaceutically acceptable salts thereof) in the health functional food may be appropriately determined depending on the type of food, the desired application or the like. For example, the content may be 0.0001 to 99% by weight based on the total weight of the food, 0.0001 to 95% by weight, 0.0001 to 90% by weight, 0.0001 to 80% by weight, 0.0001 to 50% by weight, 0.001 to 99% by weight, 0.001 to 95% by weight, 0.001 to 90% by weight, 0.001 to 80% by weight, 0.001 to 50% by weight, 0.01 to 99% by weight, 0.01 to 95% by weight, 0.01 to 90% by weight, 0.01 to 80% by weight, 0.01 to 50% by weight, 0.1 to 99% by weight, 0.1 to 95% by weight, 0.1 to 90% by weight, 0.1 to 80% by weight, 0.1 to 50% by weight, 0.1 to 30% by weight, 0.1 to 10% by weight, 1 to 99% by weight, 1 to 95% by weight, 1 to 90% by weight, 1 to 80% by weight, 1 to 50% by weight, 1 to 30% by weight, 1 to 10% by weight, 10 to 99% by weight, 10 to 95% by weight, 10 to 90% by weight, 10 to 80% by weight, 10 to 50% by weight, 10 to 30% by weight, 25 to 99% by weight, 25 to 95% by weight, 25 to 90% by weight, 25 to 80% by weight, 25 to 50% by weight, 25 to 30% by weight, 40 to 99% by weight, 40 to 95% by weight, 40 to 90% by weight, 40 to 80% by weight, 40 to 50% by weight, 50 to 99% by weight, 50 to 95% by weight, 50 to 90% by weight, 50 to 80% by weight, 60 to 99% by weight, 60 to 95% by weight, 60 to 90% by weight, or 60 to 80% by weight, but is not limited thereto.

The health functional food may further contain at least one selected from the group consisting of various nutrients, vitamins, minerals (electrolytes), flavoring agents such as synthetic or natural flavoring agents, coloring agents, thickeners (cheese, chocolate, etc.), pectic acid or salts thereof, alginic acid or salts thereof, organic acids, protective colloidal thickeners, pH adjusters, stabilizers, preservatives, glycerin, alcohols, carbonation agents used in carbonated beverages, and the like. The content of this additive is generally determined within the range from about 0.001 to about 20 parts by weight with respect to 100 parts by weight of the total amount of the health functional food, but is not limited thereto.

In another aspect, the present invention provides a proteolysis-targeting chimera (PROTAC, hereinafter also referred to as "PROTAC compound") containing the pyrrolidine derivative or a pharmaceutically acceptable salt thereof, and the use of the pyrrolidine derivative or a pharmaceutically acceptable salt thereof for the preparation of the proteolysis-targeting chimera compound.

In a specific embodiment, the proteolysis-targeting chimera compound may further contain a target-binding ligand (i.e., a protein-targeting moiety) along with the pyrrolidine derivative or a pharmaceutically acceptable salt thereof and may preferably have a structure in which the pyrrolidine derivative or a pharmaceutically acceptable salt thereof is linked to the target-binding ligand via a linker, for example, a binding moiety or a chemical linking moiety. As will be appreciated by those skilled in the art, the PROTAC compound disclosed herein may be synthesized such that the number and positions of functional moieties can be changed as desired.

In a specific embodiment, the PROTAC compound according to the present invention may be a TAU-1 compound represented by the following Formula (VI) of (Z)-N-(2-(2,6-dioxopiperidin-3-yl)-1,3-dioxoisoindolin-4-yl)-2-(2-(2-(2-(4-((3-((1-ethyl-5-oxo-2-thioxoimidazolidin-4-ylidene)methyl)-1H-pyrrolo[3,2-b]pyridin-1-yl)methyl)-1H-1,2,3-triazol-1-yl)ethoxy)ethoxy)ethoxy)acetamide, or a TAU-2 compound represented by the following Formula (VII) of (Z)-N-(2-(2,6-dioxopiperidin-3-yl)-1,3-dioxoisoindolin-4-yl)-2-(2-(2-(4-((3-((1-ethyl-5-oxo-2-thioxoimidazolidin-4-ylidene)methyl)-1H-indol-1-yl)methyl)-1H-1,2,3-triazol-1-yl)ethoxy)ethoxy)acetamide.

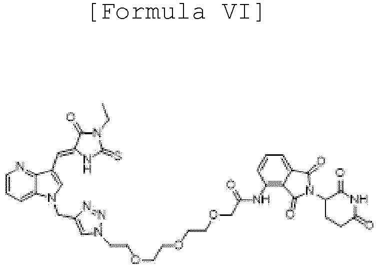

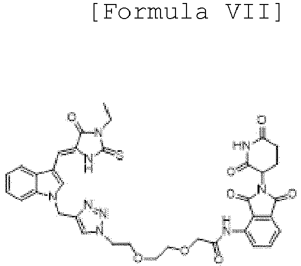

In a specific embodiment, the TAU-1 and TAU-2 compounds according to the present invention exhibit effective degradation activity in three phosphorylated tau proteins, are useful for the treatment of brain diseases, and in particular exhibit excellent selectivity, that is, have selective binding affinity to pathological P301S, while having almost no binding affinity to nonpathological WT Tau.

Accordingly, in one embodiment, the present invention provides the pharmaceutical use of the PROTAC compound for inhibition of aggregation of beta-amyloids and/or tau proteins, degradation of aggregates of beta-amyloids and/or tau proteins, inhibition of aggregation of beta-amyloids and/or tau proteins, protection of neurons, and prevention and/or treatment of beta-amyloid- and/or tau protein-related diseases.

In another embodiment, the present invention provides a method for inhibiting aggregation of beta-amyloids and/or tau proteins including administering a pharmaceutically effective amount of the PROTAC compound to a patient in need of inhibition of aggregation of beta-amyloids and/or tau proteins.

In another embodiment, the present invention provides the use of the PROTAC compound for inhibition of aggregation of beta-amyloids and/or tau proteins, or for preparation of a composition for inhibiting aggregation of beta-amyloids and/or tau proteins.

In another embodiment, the present invention provides a pharmaceutical composition for degrading aggregates of beta-amyloids and/or tau proteins containing the PROTAC compound as an active ingredient.

In another embodiment, the present invention provides a method for degrading aggregates of beta-amyloids and/or tau proteins including administering a pharmaceutically effective amount of the PROTAC compound to a patient in need of degradation of aggregates of beta-amyloids and/or tau proteins.

In another embodiment, the present invention provides the use of the PROTAC compound for degradation of aggregates of beta-amyloids and/or tau proteins, or for preparation of a composition for degrading aggregates of beta-amyloids and/or tau proteins.

In another embodiment, the present invention provides a pharmaceutical composition for protecting neurons containing the PROTAC compound as an active ingredient. The protection of neurons includes protection of the neurons from damage attributable to aggregation (accumulation) of beta-amyloids and/or tau proteins.

In another embodiment, the present invention provides a method for protecting neurons including administering a pharmaceutically effective amount of the PROTAC compound to a patient in need of protection of neurons.

In another embodiment, the present invention provides the use of the PROTAC compound for protection of neurons, or for preparation of a composition for protecting neurons.

In another embodiment, the present invention provides a composition for preventing and/or treating beta-amyloid- and/or tau protein-related diseases containing the PROTAC compound as an active ingredient.

In another embodiment, the present invention provides a health functional food for preventing and/or ameliorating beta-amyloid- and/or tau protein-related diseases containing the PROTAC compound as an active ingredient.

In another embodiment, the present invention provides a method for preventing and/or treating beta-amyloid- and/or tau protein-related diseases including administering a pharmaceutically effective amount of the PROTAC compound to a patient in need of prevention and/or treatment of beta-amyloid- and/or tau protein-related diseases.

In another embodiment, the present invention provides the use of the PROTAC compound for prevention and/or treatment of beta-amyloid- and/or tau protein-related diseases or for preparation of a composition for preventing and/or treating beta-amyloid- and/or tau protein-related diseases.

The content relating to the use of the PROTAC compound, the pharmaceutical composition, and the food composition may be the same as the use and composition of the pyrrolidine derivative or pharmaceutically acceptable salt thereof according to the present invention.

### [Mode for Invention]

Hereinafter, the present invention will be described in more detail with reference to specific examples, but these examples are provided merely for illustration and those skilled in the art will appreciate that the following examples may be modified, without departing from the scope and spirit of the present invention.

### Example 1. Synthesis of pyrrolidine derivatives

### Step 1

Aldehyde (3 mmol) and dione (1.0 eq) were added to acetic acid (3 ml), followed by stirring at room temperature. Then, ammonium acetate (3.0 eq) was added dropwise to the reaction solution and the temperature was gradually increased, followed by stirring at 95°C for 2 hours. After the completion of the reaction was confirmed through thin-layer chromatography (TLC), the resulting product was filtered to obtain crystals. The crystals thus obtained were purified by recrystallization in hexane and ethyl acetate to obtain the target compound.

### Step 2

The compound (0.5 mmol) obtained in step 1 was dissolved in a mixed solvent (1 ml) of methanol and water (at a ratio = 1:1.5 by volume). Sodium hydroxide (1.0 eq) was added dropwise at room temperature, followed by stirring for 15 minutes. Then, 30 µl of a CoCl₂-DMG complex (a solution of 42 mg of cobalt chloride and 250 mg of dimethylglyoxime in 5 ml of DMF) was added to the reaction solution, followed by stirring at room temperature for 15 minutes again. Then, sodium boron hydroxide (1.3 eq) was added thereto, followed by stirring at 50°C for 24 hours. Upon completion of the reaction, the reaction solution was allowed to cool to room temperature, the reaction was terminated with water, and ethyl acetate was further added thereto to extract the organic layer. The organic layer was washed with brine and was then dehydrated with sodium sulfite. The residue obtained by distilling the solvent under reduced pressure was separated and purified by prep HPLC to obtain the target compound.

The compound thus obtained was as follows:

**[Table 2]**

| No. | Compound name | Structural Formula | common name of compound | NMR data |
|---|---|---|---|---|
| 1 | DN204284 | 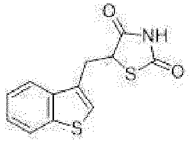 | (Z)-5-(benzo[b]thiophe n-3-ylmethylene)thia zolidine-2,4-dione | ¹H NMR (400 MHz, DMSO-d₆) δ 8.19 - 8.07 (m, 3H), 8.03 (s, 1H), 7.60 - 7.45 (m, 2H). |
| 2 | DN204286 | 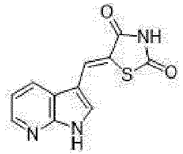 | (Z)-5-((1H-pyrrolo[2,3-b]pyridin-3-yl)methylene)thi azolidine-2,4-dione | ¹H NMR (400 MHz, DMSO-d₆) δ 12.68 (s, 1H), 12.40 (s, 1H), 8.52 - 8.26 (m, 2H), 8.06 (s, 1H), 7.83 (d, J = 2.6 Hz, 1H), 7.25 (dd, J = 7.9, 4.7 Hz, 1H). |
| 3 | DN204289 | 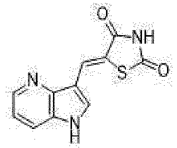 | (Z)-5-((1H-pyrrolo[3,2-b]pyridin-3-yl)methylene)thi azolidine-2,4-dione | ¹H NMR (400 MHz, DMSO-d₆) δ 12.28 (s, 1H), 12.23 (s, 1H), 8.47 (dd, J = 4.6, 1.2 Hz, 1H), 8.11 (s, 1H), 8.05 (s, 1H), 7.89 (dd, J = 8.2, 1.2 Hz, 1H), 7.27 (dd, J = 8.2, 4.6 Hz, 1H) |
| 4 | DN204290 | 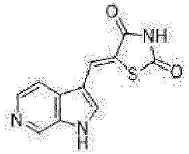 | (Z)-5-((1H-pyrrolo[2,3-c]pyridin-3-yl)methylene)thi azolidine-2,4-dione | ¹H NMR (400 MHz, DMSO-d₆) δ 12.68 (s, 1H), 12.39 (s, 1H), 11.99 (s, 1H), 8.55 - 8.24 (m, 2H), 8.06 (s, 1H), 7.83 (d, J = 2.1 Hz, 1H), 7.25 (dd, J = 7.9, 4.7 Hz, 1H) |
| 5 | DN204291 | 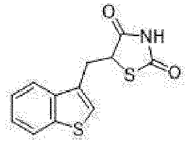 | 5-(benzo[b]thiophe n-3-ylmethyl)thiazol idine-2,4-dione | ¹H NMR (400 MHz, CDCl₃) δ 8.06 (s, 1H), 7.94 - 7.89 (m, 1H), 7.86 - 7.79 (m, 1H), 7.44 (ddd, J = 9.0, 7.5, 1.3 Hz, 2H), 7.32 (s, 1H), 4.70 (dd, J = 10.4, 3.6 Hz, 1H), 3.89 (ddd, J = 14.8, 3.6, 1.0 Hz, 1H), 3.41 (ddd, J = 14.8, 10.4, 0.5 Hz, 1H) |
| 6 | DN204300 | 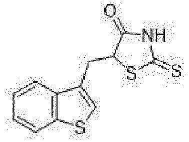 | 5-(benzo[b]thiophe n-3-ylmethyl)-2-thioxothiazolidin-4-one | ¹H NMR (400 MHz, MeOD) δ 7.90 - 7.87 (m, 1H), 7.87 - 7.84 (m, 1H), 7.44 - 7.34 (m, 3H), 4.95 (dd, J = 9.7, 4.1 Hz, 1H), 3.73 (ddd, J = 15.0, 4.1, 1.0 Hz, 1H), 3.46 (ddd, J = 15.0, 9.7, 0.5 Hz, 1H). LRMS(ESI) : m/z 280 [M+H]⁺ |
| 7 | DN204304 | 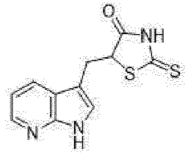 | 5-((1H-pyrrolo[2,3-b]pyridin-3-yl)methyl)-2-thioxothiazolidi n-4-one | ¹H NMR (400 MHz, MeOD) δ 8.51 (dd, J = 7.9, 1.2 Hz, 4H), 8.33 (d, J = 5.5 Hz, 4H), 7.52 (s, 4H), 7.41 (dd, J = 7.9, 5.5 Hz, 4H), 4.91 (dd, J = 6.5, 5.1 Hz, 5H), 3.56 (dd, J = 6.0, 2.1 Hz, 7H). LRMS(ESI) : m/z 264 [M+H]⁺ |
| 8 | DN204807 | 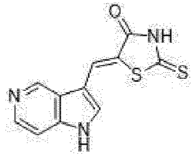 | (Z)-5-((1H-pyrrolo[3,2-c]pyridin-3-yl)methylene)-2-thioxothiazolidi n-4-one | ¹H NMR (400 MHz, DMSO) δ 11.96 (s, 1H), 9.33 (s, 1H), 8.36 (d, J = 5.8 Hz, 1H), 7.93 (s, 1H), 7.89 (s, 1H), 7.57 (d, J = 5.8 Hz, 1H). LRMS(ESI) : m/z 262 [M+H]⁺ |
| 9 | DN204808 | 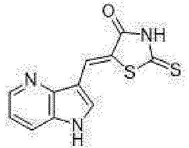 | (Z)-5-((1H-pyrrolo[3,2-b]pyridin-3-yl)methylene)-2-thioxothiazolidi n-4-one | ¹H NMR (400 MHz, DMSO) δ 13.45 (s, 1H), 12.35 (s, 1H), 8.51 (dd, J = 4.6, 1.4 Hz, 1H), 8.23 (d, J = 1.8 Hz, 1H), 7.93 (s, 1H), 7.91 (dd, J = 8.2, 1.4 Hz, 1H), 7.29 (dd, J = 8.2, 4.6 Hz, 1H). LRMS(ESI) : m/z 262 [M+H]⁺ |
| 10 | DN204810 | 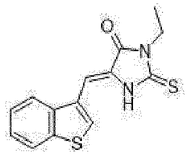 | (Z)-5-(benzo[b]thiophe n-3-ylmethylene)-3-ethyl-2-thioxoimidazolid in-4-one | ¹H NMR (400 MHz, DMSO) δ 12.42 (s, 1H), 8.56 (s, 1H), 8.08 (t, J = 7.8 Hz, 2H), 7.59 - 7.43 (m, 2H), 6.88 (s, 1H), 3.86 (q, J = 7.1 Hz, 2H), 1.19 (t, J = 7.1 Hz, 3H). LRMS(ESI) : m/z 289 [M+H]⁺ |
| 11 | DN204816 | 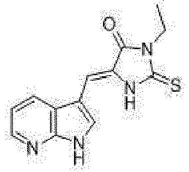 | (Z)-5-((1H-pyrrolo[2,3-b]pyridin-3-yl)methylene)-3-ethyl-2-thioxoimidazolid in-4-one | ¹H NMR (400 MHz, DMSO) δ 12.51 (s, 1H), 12.08 (s, 1H), 8.60 (s, 1H), 8.34 (d, J = 1.4 Hz, 1H), 8.32 (d, J = 3.2 Hz, 1H), 7.21 (dd, J = 7.8, 4.8 Hz, 1H), 6.95 (s, 1H), 3.85 (q, J = 7.1 Hz, 2H), 1.18 (t, J = 7.1 Hz, 3H). LRMS(ESI) : m/z 273 [M+H]⁺ |
| 12 | DN204817 | 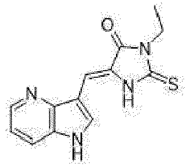 | (Z)-5-((1H-pyrrolo[3,2-b]pyridin-3-yl)methylene)-3-ethyl-2-thioxoimidazolid in-4-one | ¹H NMR (400 MHz, DMSO) δ 14.26 (s, 1H), 12.29 (s, 1H), 8.51 (dd, J = 4.7, 1.3 Hz, 1H), 8.29 (s, 1H), 8.03 (dd, J = 8.3, 1.3 Hz, 1H), 7.36 (dd, J = 8.3, 4.8 Hz, 1H), 6.94 (s, 1H), 3.83 (q, J = 7.1 Hz, 2H), 1.18 (t, J = 7.1 Hz, 3H). LRMS(ESI) : m/z 273 [M+H]⁺ |
| 13 | DN204671 | 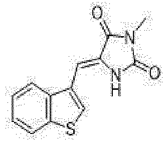 | (Z)-5-(benzo[b]thiophe n-3-ylmethylene)-3-methylimidazolid ine-2,4-dione | ¹H NMR (400 MHz, DMSO-d₆) δ 10.83 (s, 1H), 8.27 (s, 1H), 8.14 - 7.96 (m, 2H), 7.49 (td, J = 7.5, 1.2 Hz, 2H), 6.82 (s, 1H), 3.00 (s, 3H) |
| 14 | DN204673 | 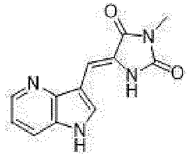 | (Z)-5-((1H-pyrrolo[3,2-b]pyridin-3-yl)methylene)-3-methylimidazolid ine-2,4-dione | ¹H NMR (400 MHz, DMSO-d₆) δ 12.43 (s, 1H), 12.05 (s, 1H), 8.65 - 8.30 (m, 1H), 8.13 (s, 1H), 7.96 (d, J = 7.2 Hz, 1H), 7.30 (dd, J = 8.2, 4.7 Hz, 1H), 6.77 (s, 1H), 2.97 (s, 3H). |
| 15 | DN205111 | 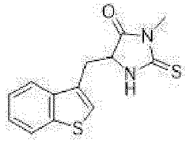 | 5-(benzo[b]thiophe n-3-ylmethyl)-3-methyl-2-thioxoimidazolid in-4-one | ¹H NMR (400 MHz, DMSO-d₆) δ 12.40 (s, 1H), 8.55 (s, 1H), 8.20 - 7.85 (m, 2H), 7.70 - 7.25 (m, 2H), 6.89 (s, 1H), 3.23 (s, 3H) |
| 16 | DN205355 | 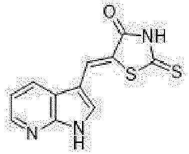 | (Z)-5-((1H-pyrrolo[2,3-b]pyridin-3-yl)methylene)-2-thioxothiazolidi n-4-one | ¹H NMR (400 MHz, DMSO) δ 13.62 (s, 1H), 12.82 (s, 1H), 11.96 (s, 1H), 8.44 (dd, J = 8.0, 1.4 Hz, 1H), 8.38 (dd, J = 4.7, 1.4 Hz, 1H), 7.95 (s, 1H), 7.92 (s, 1H), 7.27 (dd, J = 7.9, 4.7 Hz, 1H) . LRMS(ESI) : m/z 262 [M+H]⁺ |
| 17 | DN204288 | 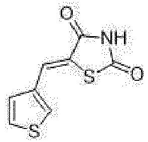 | (Z)-5-(thiophen-3-ylmethylene)thia zolidine-2,4-dione | ¹H NMR (400 MHz, DMSO-d₆) δ 12.42 (s, 1H), 8.02 (d, J = 1.9 Hz, 1H), 7.82 (s, 1H), 7.75 (dd, J = 5.0, 2.9 Hz, 1H), 7.41 - 7.35 (m, 1H) |
| 18 | DN204292 | 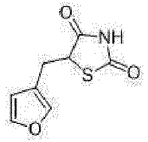 | 5-(furan-3-ylmethyl)thiazol idine-2,4-dione | ¹H NMR (400 MHz, MeOD) δ 7.46 (t, J = 1.7 Hz, 1H), 7.42 (dd, J = 1.5, 0.7 Hz, 1H), 6.39 (d, J = 1.0 Hz, 1H), 4.69 (dd, J = 8.0, 4.1 Hz, 1H), 3.22 (dd, J = 14.8, 4.0 Hz, 1H), 3.11 (dd, J = 14.8, 8.0 Hz, 1H) |
| 19 | DN204293 | 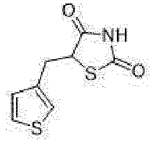 | 5-(thiophen-3-ylmethyl)thiazol idine-2,4-dione | ¹H NMR (400 MHz, MeOD) δ 7.38 (dd, J = 5.0, 3.0 Hz, 1H), 7.26 - 7.18 (m, 1H), 7.04 (dd, J = 5.0, 1.3 Hz, 1H), 4.74 (dd, J = 8.7, 4.0 Hz, 1H), 3.45 (dd, J = 14.6, 4.2 Hz, 1H), 3.28 (dd, J = 14.6, 8.6 Hz, 1H) |
| 20 | DN204294 | 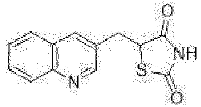 | 5-(quinolin-3-ylmethyl)thiazol idine-2,4-dione | ¹H NMR (400 MHz, MeOD) δ 9.03 (d, J = 2.1 Hz, 1H), 8.72 (s, 1H), 8.14 (d, J = 4.5 Hz, 2H), 8.07 - 7.92 (m, 1H), 7.84 (s, 1H), 4.98 (dd, J = 7.5, 5.3 Hz, 1H), 3.72 (dd, J = 14.4, 5.3 Hz, 1H), 3.63 (dd, J = 14.3, 7.5 Hz, 1H) |
| 21 | DN204307 | 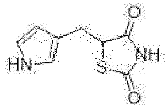 | 5-((1H-pyrrol-3-yl)methyl)thiazo lidine-2,4-dione | ¹H NMR (400 MHz, MeOD) δ 10.11 (s, 1H), 6.77 - 6.50 (m, 2H), 6.02 (d, J = 1.7 Hz, 1H), 4.62 (dd, J = 8.9, 3.8 Hz, 1H), 3.42 - 3.19 (m, 1H), 3.12 - 3.01 (m, 1H) |
| 22 | DN204297 | 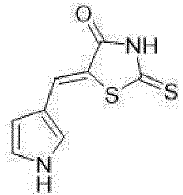 | (Z)-5-((1H-pyrrol-3-yl)methylene)-2-thioxothiazolidi n-4-one | ¹H NMR (400 MHz, DMSO) δ 14.26 (s, 1H), 12.51 (s, 1H), 8.42 (s, 1H), 8.27 (dt, J = 3.1, 1.7 Hz, 1H), 7.81 (dd, J = 4.6, 2.4 Hz, 1H), 7.12 (dd, J = 4.6, 1.9 Hz, 1H). LRMS(ESI) : m/z 211 [M+H]⁺ |
| 23 | DN204302 | 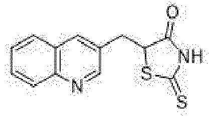 | 5-(quinolin-3-ylmethyl)-2-thioxothiazolidi n-4-one | ¹H NMR (400 MHz, MeOD) δ 9.07 (d, J = 2.0 Hz, 1H), 8.83 (d, J = 1.5 Hz, 1H), 8.20 (d, J = 8.1 Hz, 1H), 8.16 (d, J = 8.6 Hz, 1H), 8.05 (ddd, J = 8.5, 7.0, 1.3 Hz, 1H), 7.88 (ddd, J = 8.1, 7.0, 1.0 Hz, 1H), 5.04 (dd, J = 7.1, 5.8 Hz, 1H), 3.69 (qd, J = 14.5, 6.4 Hz, 2H). LRMS(ESI) : m/z 275 [M+H]⁺ |
| 24 | DN204303 | 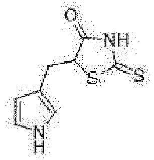 | 5-((1H-pyrrol-3-yl)methyl)-2-thioxothiazolidin-4-one | ¹H NMR (400 MHz, MeOD) δ 10.09 (s, 1H), 6.65 (dd, J = 4.6, 2.6 Hz, 1H), 6.61 (s, 1H), 6.00 (dd, J = 4.1, 2.2 Hz, 1H), 4.67 (dd, J = 8.9, 4.0 Hz, 1H), 3.25 (dd, J = 14.6, 3.9 Hz, 1H), 3.06 (dd, J = 14.6, 8.9 Hz, 1H). LRMS(ESI) : m/z 213 [M+H]⁺ |
| 25 | DN204305 | 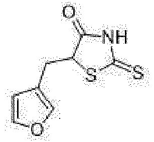 | 5-(furan-3-ylmethyl)-2-thioxothiazolidi n-4-one | ¹H NMR (400 MHz, MeOD) δ 7.44 (t, J = 1.7 Hz, 1H), 7.39 (s, 1H), 6.36 (d, J = 1.0 Hz, 1H), 4.74 (dd, J = 7.8, 4.3 Hz, 1H), 3.19 (dd, J = 14.9, 4.2 Hz, 1H), 3.11 (dd, J = 14.9, 7.8 Hz, 1H). LRMS(ESI) : m/z 214 [M+H]⁺ |
| 26 | DN204812 | 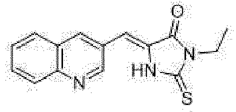 | (Z)-3-ethyl-5-(quinolin-4-ylmethylene)-2-thioxoimidazolid in-4-one | ¹H NMR (400 MHz, DMSO) δ 12.65 (s, 1H), 9.13 (d, J = 2.1 Hz, 1H), 8.86 (d, J = 1.8 Hz, 1H), 8.03 (s, 1H), 8.01 (s, 1H), 7.81 (t, J = 7.6 Hz, 1H), 7.67 (t, J = 7.6 Hz, 1H), 6.81 (s, 1H), 3.86 (q, J = 7.1 Hz, 2H), 1.19 (t, J = 7.1 Hz, 3H). LRMS(ESI) : m/z 284 [M+H]⁺ |
| 27 | DN204813 | 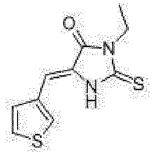 | (Z)-3-ethyl-5-(thiophen-3-ylmethylene)-2-thioxoimidazolid in-4-one | ¹H NMR (400 MHz, DMSO) δ 12.20 (s, 1H), 8.25 (d, J = 1.4 Hz, 1H), 7.67 (qd, J = 5.1, 2.0 Hz, 2H), 6.72 (s, 1H), 3.83 (q, J = 7.1 Hz, 2H), 1.16 (t, J = 7.1 Hz, 3H). LRMS(ESI) : m/z 239 [M+H]⁺ |
| 28 | DN204814 | 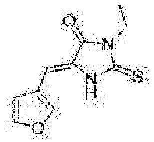 | (Z)-3-ethyl-5-(furan-3-ylmethylene)-2-thioxoimidazolid in-4-one | ¹H NMR (400 MHz, DMSO) δ 12.07 (s, 1H), 8.38 (s, 1H), 7.80 (t, J = 1.4 Hz, 1H), 7.24 (d, J = 1.7 Hz, 1H), 6.59 (s, 1H), 3.81 (q, J = 7.1 Hz, 2H), 1.15 (t, J = 7.1 Hz, 3H). LRMS(ESI) : m/z 223 [M+H]⁺ |
| 29 | DN204819 | 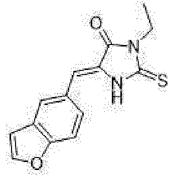 | (Z)-5-(benzofuran-5-ylmethylene)-3-ethyl-2-thioxoimidazolid in-4-one | ¹H NMR (400 MHz, DMSO) δ 12.41 (s, 1H), 8.15 (d, J = 1.3 Hz, 1H), 8.07 (d, J = 2.2 Hz, 1H), 7.71 (dd, J = 8.7, 1.7 Hz, 1H), 7.65 (d, J = 8.6 Hz, 1H), 7.01 (dd, J = 2.2, 0.7 Hz, 1H), 6.76 (s, 1H), 3.85 (q, J = 7.1 Hz, 2H), 1.18 (t, J = 7.1 Hz, 3H). LRMS(ESI) : m/z 273 [M+H]⁺ |
| 30 | DN204669 | 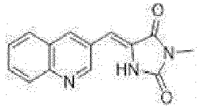 | (Z)-5-(isoquinolin-4-ylmethylene)-3-methylimidazolid ine-2,4-dione | ¹H NMR (400 MHz, DMSO-d₆) δ 9.08 (s, 1H), 8.70 (s, 1H), 8.00 (dd, J = 15.9, 7.7 Hz, 2H), 7.72 (d, J = 50.9 Hz, 2H), 6.72 (s, 1H), 3.00 (s, 3H) |
| 31 | DN204670 | 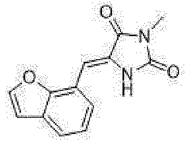 | (Z)-5-(benzofuran-7-ylmethylene)-3-methylimidazolid ine-2,4-dione | ¹H NMR (400 MHz, DMSO-d₆) δ 8.16 - 7.89 (m, 2H), 7.61 (d, J = 9.6 Hz, 2H), 6.98 (s, 1H), 6.67 (s, 1H), 2.98 (s, 3H) |
| 32 | DN205106 | 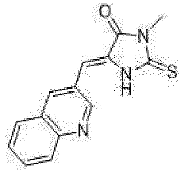 | (Z)-3-methyl-5-(quinolin-3-ylmethylene)imid azolidine-2,4-dione | ¹H NMR (400 MHz, DMSO-d₆) δ 12.63 (s, 1H), 9.12 (d, J = 2.3 Hz, 1H), 8.85 (d, J = 2.1 Hz, 1H), 8.02 (d, J = 8.4 Hz, 2H), 7.81 (ddd, J = 8.3, 6.9, 1.4 Hz, 1H), 7.72 - 7.59 (m, 1H), 6.83 (s, 1H), 3.24 (s, 3H) |
| 33 | DN205108 | 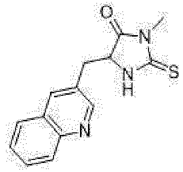 | 3-methyl-5-(quinolin-3-ylmethyl)imidazo lidine-2,4-dione | ¹H NMR (400 MHz, MeOD) δ 8.67 (d, J = 2.1 Hz, 1H), 8.31 (s, 1H), 8.02 - 7.91 (m, 2H), 7.87 - 7.74 (m, 1H), 7.65 (t, J = 7.1 Hz, 1H), 4.05 (d, J = 13.8 Hz, 1H), 3.81 (d, J = 13.8 Hz, 1H), 2.75 (s, 3H). |
| 34 | DN205109 | 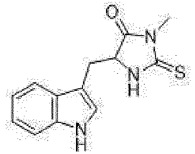 | 5-((1H-indol-3-yl)methyl)-3-methylimidazolid ine-2,4-dione | ¹H NMR (400 MHz, MeOD) δ 7.55 (d, J = 7.8 Hz, 1H), 7.30 (d, J = 8.1 Hz, 1H), 7.14 - 7.03 (m, 2H), 7.02 - 6.96 (m, 1H), 3.57 - 3.38 (m, 2H), 3.28 - 3.11 (m, 3H) |
| 35 | DN205110 | 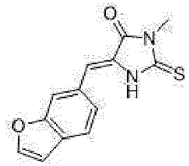 | (Z)-5-(benzofuran-6-ylmethylene)-3-methylimidazolid ine-2,4-dione | ¹H NMR (400 MHz, DMSO-d₆) δ 8.21 - 8.00 (m, 2H), 7.68 (dd, J = 19.8, 8.6 Hz, 2H), 7.01 (d, J = 2.2 Hz, 1H), 6.77 (s, 1H), 3.22 (s, 3H) |
| 36 | DN205353 | 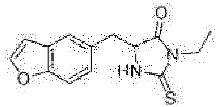 | 5-(benzofuran-5-ylmethyl)-3-ethyl-2-thioxoimidazolid in-4-one | ¹H NMR (400 MHz, DMSO) δ 10.35 (s, 1H), 7.94 (d, J = 2.2 Hz, 1H), 7.47 (d, J = 8.5 Hz, 1H), 7.41 (d, J = 1.4 Hz, 1H), 7.09 (dd, J = 8.5, 1.7 Hz, 1H), 6.91 (dd, J = 2.1, 0.8 Hz, 1H), 4.59 (dd, J = 4.8, 3.7 Hz, 1H), 3.46 (q, J = 7.1 Hz, 2H), 3.12 (d, J = 4.7 Hz, 2H), 0.66 (t, J = 7.1 Hz, 3H). LRMS(ESI) : m/z 275 [M+H]⁺ |
| 37 | DN205354 | 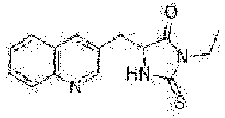 | 3-ethyl-5-(quinolin-4-ylmethyl)-2-thioxoimidazolid in-4-one | ¹H NMR (400 MHz, DMSO) δ 10.45 (s, 1H), 8.84 (d, J = 2.0 Hz, 1H), 8.31 (s, 1H), 8.05 (d, J = 8.4 Hz, 1H), 8.00 (d, J = 8.2 Hz, 1H), 7.86 - 7.79 (m, 1H), 7.68 (t, J = 7.1 Hz, 1H), 4.74 (t, J = 5.0 Hz, 1H), 3.51 (q, J = 7.1 Hz, 2H), 3.29 (ddd, J = 20.2, 14.3, 5.5 Hz, 2H), 0.70 (t, J = 7.1 Hz, 3H). LRMS(ESI) : m/z 286 [M+H]⁺ |
| 38 | DN204818 | 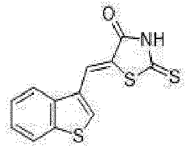 | (Z)-5-(benzo[b]thiophe n-3-ylmethylene)-2-thioxothiazolidi n-4-one | ¹H NMR (400 MHz, DMSO) δ 13.87 (s, 1H), 8.20 - 8.10 (m, 3H), 7.90 (s, 1H), 7.52 (pd, J = 7.1, 1.3 Hz, 2H). LRMS(ESI) : m/z 278 [M+H]⁺ |
| 39 | DN204285 | 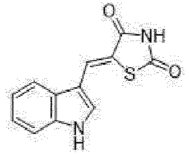 | (Z)-5-((1H-indol-3-yl)methylene)thi azolidine-2,4-dione | ¹H NMR (400 MHz, DMSO-d₆) δ 12.30 (s, 1H), 12.13 (s, 1H), 8.06 (s, 1H), 7.89 (d, J = 7.8 Hz, 1H), 7.74 (d, J = 2.9 Hz, 1H), 7.51 (d, J = 8.0 Hz, 1H), 7.23 (dt, J = 14.8, 7.1 Hz, 2H) |
| 40 | DN204295 | 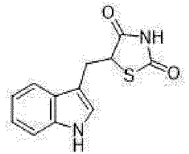 | 5-((1H-indol-3-yl)methyl)thiazo lidine-2,4-dione | ¹H NMR (400 MHz, MeOD) δ 7.60 (d, J = 7.9 Hz, 1H), 7.37 (d, J = 8.1 Hz, 1H), 7.20 - 7.10 (m, 2H), 7.07 - 7.01 (m, 1H), 4.81 (dd, J = 9.1, 4.0 Hz, 1H), 3.62 (ddd, J = 14.8, 4.0, 0.7 Hz, 1H), 3.42 - 3.29 (m, 1H) |
| 41 | DN204301 | 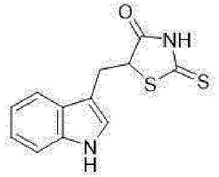 | 5-((1H-indol-3-yl)methyl)-2-thioxothiazolidi n-4-one | ¹H NMR (400 MHz, MeOD) δ 7.62 - 7.54 (m, 1H), 7.36 - 7.31 (m, 1H), 7.14 (s, 1H), 7.13 - 7.08 (m, 1H), 7.05 - 7.00 (m, 1H), 4.87 (d, J = 4.0 Hz, 1H), 3.60 (ddd, J = 14.8, 4.0, 0.8 Hz, 1H), 3.38 - 3.33 (m, 1H). LRMS(ESI) : m/z 263 [M+H]⁺ |
| 42 | DN204811 | 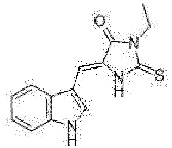 | (Z)-5-((1H-indol-3-yl)methylene)-3-ethyl-2-thioxoimidazolid in-4-one | ¹H NMR (400 MHz, DMSO) δ 12.07 (s, 1H), 12.01 (s, 1H), 8.50 (d, J = 2.9 Hz, 1H), 7.84 (d, J = 7.7 Hz, 1H), 7.47 (d, J = 7.9 Hz, 1H), 7.20 (dtd, J = 14.7, 7.5, 1.0 Hz, 2H), 6.97 (s, 1H), 3.85 (q, J = 7.1 Hz, 2H), 1.18 (t, J = 7.1 Hz, 3H). LRMS(ESI) : m/z 272 [M+H]⁺ |
| 43 | DN204815 | 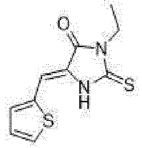 | (Z)-3-ethyl-5-(thiophen-2-ylmethylene)-2-thioxoimidazolid in-4-one | ¹H NMR (400 MHz, DMSO) δ 12.18 (s, 1H), 7.89 (d, J = 3.7 Hz, 1H), 7.84 (d, J = 5.0 Hz, 1H), 7.23 (dd, J = 4.9, 3.9 Hz, 1H), 6.77 (s, 1H), 3.82 (q, J = 7.1 Hz, 2H), 1.16 (t, J = 7.1 Hz, 3H). LRMS(ESI) : m/z 239 [M+H]⁺ |
| 44 | DN204672 | 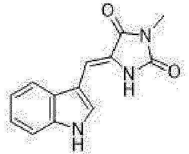 | (Z)-5-((1H-indol-3-yl)methylene)-3-methylimidazolid ine-2,4-dione | ¹H NMR (400 MHz, DMSO-d₆) δ 11.83 (s, 1H), 10.32 (s, 1H), 8.15 (s, 1H), 7.78 (d, J = 7.8 Hz, 1H), 7.43 (d, J = 8.0 Hz, 1H), 7.29 - 7.04 (m, 2H), 6.86 (s, 1H), 2.97 (s, 3H) |
| 45 | DN205779 | 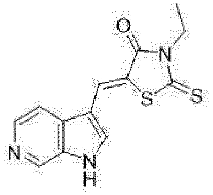 | (Z)-5-((1H-pyrrolo[2,3-c]pyridin-3-yl)methylene)-3-ethyl-2-thioxothiazolidi n-4-one | ¹H NMR (400 MHz, DMSO-d₆) δ 12.87 (s, 1H), 8.48 (d, J = 7.9 Hz, 1H), 8.38 (d, J = 4.5 Hz, 1H), 8.11 (s, 1H), 8.00 (s, 1H), 7.28 (dd, J = 7.9, 4.7 Hz, 1H), 4.08 (q, J = 7.0 Hz, 2H), 1.20 (t, J = 7.1 Hz, 3H) |
| 46 | DN205780 | 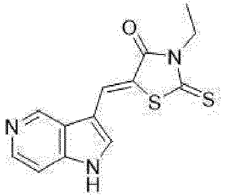 | (Z)-5-((1H-pyrrolo[3,2-c]pyridin-3-yl)methylene)-3-ethyl-2-thioxothiazolidi n-4-one | 1H NMR (400 MHz, DMSO-d₆) δ 12.39 (s, 1H), 9.28 (s, 1H), 8.34 (d, J = 5.6 Hz, 1H), 8.19 (s, 1H), 7.98 (s, 1H), 7.49 (d, J = 5.6 Hz, 1H), 4.09 (q, J = 7.0 Hz, 1H), 1.21 (t, J = 7.1 Hz, 2H) |
| 47 | DN205781 | 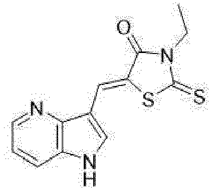 | (Z)-5-((1H-pyrrolo[3,2-b]pyridin-3-yl)methylene)-3-ethyl-2-thioxothiazolidi n-4-one | ¹H NMR (400 MHz, DMSO-d₆) δ 12.40 (s, 1H), 8.53 (d, J = 4.5 Hz, 1H), 8.32 (s, 1H), 8.08 (s, 1H), 7.92 (d, J = 8.2 Hz, 1H), 7.30 (dd, J = 8.2, 4.6 Hz, 1H), 4.07 (q, J = 7.1 Hz, 2H), 1.19 (t, J = 7.1 Hz, 3H) |
| 48 | DN205782 | 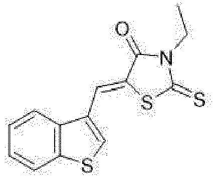 | (Z)-5-(benzo[b]thiophe n-3-ylmethylene)-3-ethyl-2-thioxothiazolidi n-4-one | 1H NMR (400 MHz, DMSO-d₆) δ 8.28 - 8.18 (m, 2H), 8.13 (d, J = 7.7 Hz, 1H), 8.07 (s, 1H), 7.60 - 7.45 (m, 2H), 4.10 (q, J = 7.0 Hz, 2H), 1.22 (t, J = 7.1 Hz, 3H) |
| 49 | DN205783 | 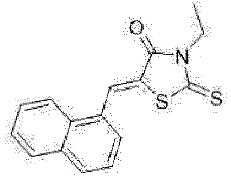 | (Z)-3-ethyl-5-(naphthalen-2-ylmethylene)-2-thioxothiazolidi n-4-one | 1H NMR (400 MHz, DMSO-d₆) δ 8.47 (s, 1H), 8.21 (d, J = 8.2 Hz, 1H), 8.12 (d, J = 7.8 Hz, 1H), 8.06 (d, J = 7.8 Hz, 1H), 7.77 - 7.57 (m, 4H), 4.11 (q, J = 7.1 Hz, 2H), 1.23 (t, J = 7.1 Hz, 3H) |
| 50 | DN205821 | 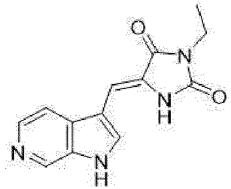 | (Z)-5-((1H-pyrrolo[2,3-c]pyridin-3-yl)methylene)-3-ethylimidazolidi ne-2,4-dione | 1H NMR (400 MHz, DMSO-d₆) δ 12.72 (s, 1H), 9.93 (s, 1H), 8.48 (s, 1H), 8.39 (dd, J = 13.1, 6.2 Hz, 2H), 8.02 (s, 1H), 7.29 (dd, J = 7.8, 4.7 Hz, 1H), 3.37 (q, J = 7.1 Hz, 2H), 1.07 (t, J = 7.2 Hz, 3H) |
| 51 | DN205822 | 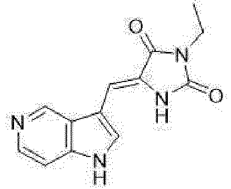 | (Z)-5-((1H-pyrrolo[3,2-c]pyridin-3-yl)methylene)-3-ethylimidazolidi ne-2,4-dione | 1H NMR (400 MHz, DMSO-d₆) δ 12.21 (s, 1H), 10.46 (s, 1H), 9.13 (s, 1H), 8.28 (s, 1H), 8.19 (s, 1H), 7.45 (d, J = 5.3 Hz, 1H), 6.92 (s, 1H), 3.53 (q, J = 7.1 Hz, 2H), 1.16 (t, J = 7.2 Hz, 3H) |
| 52 | DN205826 | 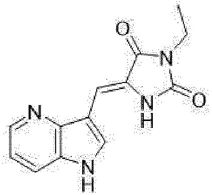 | (Z)-5-((1H-pyrrolo[3,2-b]pyridin-3-yl)methylene)-3-ethylimidazolidi ne-2,4-dione | 1H NMR (400 MHz, DMSO-d₆) δ 12.46 (s, 1H), 12.08 (s, 1H), 8.48 (d, J = 4.6 Hz, 1H), 8.15 (s, 1H), 7.96 (d, J = 8.2 Hz, 1H), 7.30 (dd, J = 8.2, 4.7 Hz, 1H), 6.77 (s, 1H), 3.52 (q, J = 7.1 Hz, 2H), 1.15 (t, J = 7.1 Hz, 3H) |
| 53 | DN205827 | 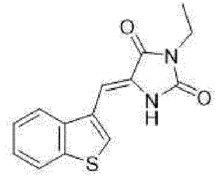 | (Z)-5-(benzo[b]thiophe n-3-ylmethylene)-3-ethylimidazolidi ne-2,4-dione | 1H NMR (400 MHz, DMSO-d₆) δ 10.85 (s, 1H), 8.27 (s, 1H), 8.05 (dd, J = 14.9, 7.8 Hz, 2H), 7.65 - 7.30 (m, 2H), 6.82 (s, 1H), 3.55 (q, J = 7.2 Hz, 2H), 1.17 (t, J = 7.2 Hz, 3H) |
| 54 | DN205828 | 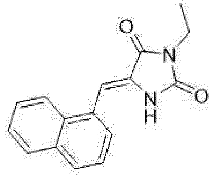 | (Z)-3-ethyl-5-(naphthalen-2-ylmethylene)imid azolidine-2,4-dione | 1H NMR (400 MHz, CDCl₃) δ 8.03 (d, J = 7.4 Hz, 1H), 7.94 - 7.84 (m, 2H), 7.63 - 7.55 (m, 2H), 7.55 - 7.48 (m, 2H), 7.34 (s, 1H), 3.72 (q, J = 7.2 Hz, 2H), 1.32 (t, J = 7.2 Hz, 3H) |
| 55 | DN204287 | 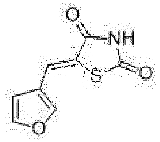 | (Z)-5-(furan-3-ylmethylene)thia zolidine-2,4-dione | ¹H NMR (400 MHz, DMSO-d₆) δ 12.49 (s, 1H), 8.25 (s, 1H), 7.88 (s, 1H), 7.69 (s, 1H), 6.75 (d, J = 1.3 Hz, 1H) |
| 56 | DN204296 | 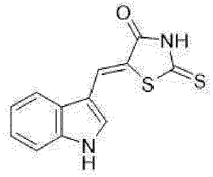 | (Z)-5-((1H-indol-3-yl)methylene)-2-thioxothiazolidi n-4-one | ¹H NMR (400 MHz, DMSO) δ 13.55 (s, 1H), 12.30 (s, 1H), 7.93 (t, J = 3.7 Hz, 2H), 7.82 (d, J = 3.0 Hz, 1H), 7.51 (d, J = 7.7 Hz, 1H), 7.24 (dtd, J = 14.8, 7.1, 1.2 Hz, 2H). LRMS(ESI) : m/z 261 [M+H]⁺ |
| 57 | DN204298 | 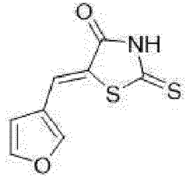 | (Z)-5-(furan-3-ylmethylene)-2-thioxothiazolidi n-4-one | ¹H NMR (400 MHz, DMSO) δ 13.73 (s, 1H), 8.31 (d, J = 0.5 Hz, 1H), 7.90 (dd, J = 2.5, 1.0 Hz, 1H), 7.61 - 7.53 (m, 1H), 6.77 (d, J = 1.7 Hz, 1H). LRMS(ESI) : m/z 212 [M+H]⁺ |
| 58 | DN204299 | 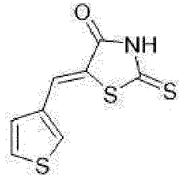 | (Z)-5-(thiophen-3-ylmethylene)-2-thioxothiazolidi n-4-one | ¹H NMR (400 MHz, DMSO) δ 13.76 (s, 1H), 8.08 (d, J = 1.6 Hz, 1H), 7.76 (dd, J = 5.1, 2.9 Hz, 1H), 7.68 (s, 1H), 7.39 (dd, J = 5.1, 1.3 Hz, 1H). LRMS(ESI) : m/z 228 [M+H]⁺ |
| 59 | DN204306 | 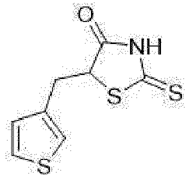 | 5-(thiophen-3-ylmethyl)-2-thioxothiazolidi n-4-one | ¹H NMR (400 MHz, MeOD) δ 7.26 (dd, J = 5.0, 3.0 Hz, 1H), 7.12 - 7.07 (m, 1H), 6.91 (dd, J = 5.0, 1.2 Hz, 1H), 4.70 (dd, J = 8.5, 4.2 Hz, 1H), 3.32 (dd, J = 14.6, 4.1 Hz, 1H), 3.19 - 3.14 (m, 1H). LRMS(ESI) : m/z 230 [M+H]⁺ |
| 60 | DN204809 | 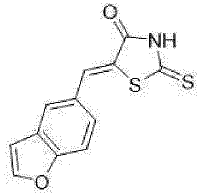 | (Z)-5-(benzofuran-5-ylmethylene)-2-thioxothiazolidi n-4-one | ¹H NMR (400 MHz, DMSO) δ 13.83 (s, 1H), 8.12 (d, J = 2.2 Hz, 1H), 7.95 (d, J = 1.8 Hz, 1H), 7.79 (s, 1H), 7.77 (s, 1H), 7.59 (dd, J = 8.7, 1.9 Hz, 1H), 7.11 (dd, J = 2.2, 0.9 Hz, 1H). LRMS(ESI) : m/z 262 [M+H]⁺ |
| 61 | DN205107 | 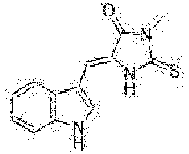 | (Z)-5-((1H-indol-3-yl)methylene)-3-methylimidazolid ine-2,4-dione | ¹H NMR (400 MHz, DMSO-d₆) δ 12.07 (s, 1H), 12.01 (s, 1H), 8.51 (d, J = 2.9 Hz, 1H), 7.84 (d, J = 7.8 Hz, 1H), 7.55 - 7.34 (m, 1H), 7.30 - 7.08 (m, 2H), 6.98 (s, 1H), 3.22 (d, J = 4.2 Hz, 3H) |
| 62 | | 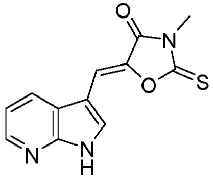 | (Z)-5-((1H-pyrrolo[2,3-b]pyridin-3-yl)methylene)-3-methyl-2-thioxooxazolidin -4-one | 1H NMR (400 MHz, DMSO) δ 12.69 (s, 1H), 8.61 (d, J = 8.0 Hz, 1H), 8.36 (d, J = 4.6 Hz, 1H), 8.19 (s, 1H), 7.34 (s, 1H), 7.28 (dd, J = 8.0, 4.7 Hz, 1H), 3.25 (s, 3H). 13C NMR (400 MHz, DMSO) δ 183.70, 161.74, 149.51, 144.94, 136.29, 133.78, 129.14, 118.57, 117.87, 108.66, 107.37, 29.33. LRMS(ESI) : m/z 260 [M+H]+ |
| 63 | | 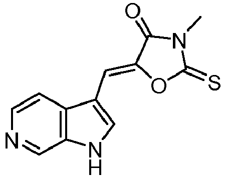 | (Z)-5-((1H-pyrrolo[2,3-c]pyridin-3-yl)methylene)-3-methyl-2- | 1H NMR (400 MHz, DMSO) δ 12.55 (s, 1H), 8.87 (d, J = 0.7 Hz, 1H), 8.29 (d, J = 5.5 Hz, 1H), 8.27 (s, 1H), 8.14 (dd, J = 5.5, 0.8 Hz, 1H), 7.37 (s, 1H), 3.25 (s, 3H). 13C NMR (400 MHz, DMSO) δ 183.77, 161.67, 141.73, 140.40, 136.54, 135.94, 134.11, 131.18, 114.69, 107.99, 107.79, 29.35. LRMS(ESI) : m/z 260 [M+H]+ |
| 64 | | 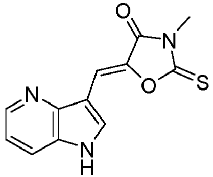 | (Z)-5-((1H-pyrrolo[3,2-b]pyridin-3-yl)methylene)-3-methyl-2-thioxooxazolidin -4-one | 1H NMR (400 MHz, DMSO) δ 12.30 (s, 1H), 8.50 (dd, J = 4.6, 1.3 Hz, 1H), 8.29 (s, 1H), 7.94 (dd, J = 8.2, 1.3 Hz, 1H), 7.28 (dd, J = 8.2, 4.6 Hz, 1H), 7.24 (s, 1H), 3.25 (s, 3H). 13C NMR (400 MHz, DMSO) δ 183.90, 161.64, 144.78, 144.57, 137.12, 133.64, 129.18, 120.54, 118.68, 107.71, 105.44, 29.37. LRMS(ESI) : m/z 260 [M+H]+ |
| 65 | | 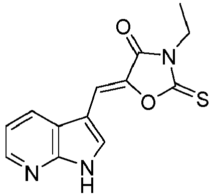 | (Z)-5-((1H-pyrrolo[2,3-b]pyridin-3-yl)methylene)-3-ethyl-2-thioxooxazolidin -4-one | 1H NMR (400 MHz, DMSO) δ 12.71 (s, 1H), 8.61 (dd, J = 8.0, 1.4 Hz, 1H), 8.36 (dd, J = 4.6, 1.4 Hz, 1H), 8.19 (s, 1H), 7.33 (s, 1H), 7.28 (dd, J = 8.0, 4.7 Hz, 1H), 3.84 (q, J = 7.2 Hz, 2H), 1.23 (t, J = 7.2 Hz, 3H). 13C NMR (400 MHz, DMSO) δ 182.98, 161.44, 149.53, 144.94, 136.07, 133.88, 129.15, 118.57, 117.88, 108.78, 107.39, 38.05, 12.32. LRMS(ESI) : m/z 274 [M+H]+ |
| 66 | DN207298 | 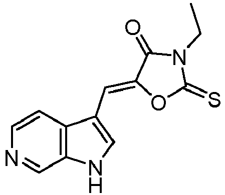 | (Z)-5-((1H-pyrrolo[2,3-c]pyridin-3-yl)methylene)-3-ethyl-2-thioxooxazolidin -4-one | ¹H NMR (400 MHz, DMSO) δ 12.33 (s, 1H), 8.87 (d, J = 0.7 Hz, 1H), 8.29 (d, J = 5.5 Hz, 1H), 8.27 (s, 1H), 8.13 (dd, J = 5.5, 0.8 Hz, 1H), 7.37 (s, 1H), 3.84 (q, J = 7.2 Hz, 2H), 1.23 (t, J = 7.2 Hz, 3H). ¹³C NMR (400 MHz, DMSO) δ 183.05, 161.38, 140.38, 136.33, 136.06, 135.94, 134.15, 131.20, 114.69, 108.09, 107.81, 38.06, 12.31. LRMS(ESI) : m/z 274 [M+H]⁺ |
| 67 | DN207299 | 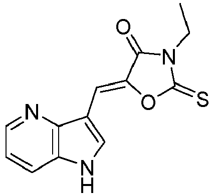 | (Z)-5-((1H-pyrrolo[3,2-b]pyridin-3-yl)methylene)-3-ethyl-2-thioxooxazolidin -4-one | ¹H NMR (400 MHz, DMSO) δ 12.29 (s, 1H), 8.50 (dd, J = 4.6, 1.3 Hz, 1H), 8.29 (s, 1H), 7.94 (dd, J = 8.2, 1.3 Hz, 1H), 7.28 (dd, J = 8.2, 4.6 Hz, 1H), 7.25 (s, 1H), 3.84 (q, J = 7.2 Hz, 2H), 1.23 (t, J = 7.2 Hz, 3H). ¹³C NMR (400 MHz, DMSO) δ 183.19, 161.35, 144.78, 144.57, 136.91, 133.70, 129.17, 120.54, 118.69, 107.73, 105.54, 38.10, 12.31. LRMS(ESI) : m/z 274 [M+H]⁺ |
| 68 | DN207300 | 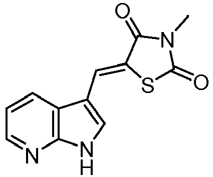 | (Z)-5-((1H-pyrrolo[2,3-b]pyridin-3-yl)methylene)-3-methylthiazolidi ne-2,4-dione | ¹H NMR (400 MHz, DMSO) δ 12.73 (s, 1H), 8.44 (dd, J = 7.9, 1.4 Hz, 1H), 8.37 (dd, J = 4.7, 1.4 Hz, 1H), 8.19 (s, 1H), 7.89 (s, 1H), 7.26 (dd, J = 7.9, 4.7 Hz, 1H), 3.12 (s, 3H). ¹³C NMR (400 MHz, DMSO) δ 167.59, 166.20, 149.19, 145.07, 140.55, 129.80, 128.03, 125.37, 119.48, 117.71, 109.69, 28.24. LRMS(ESI) : m/z 260 [M+H]⁺ |
| 69 | | 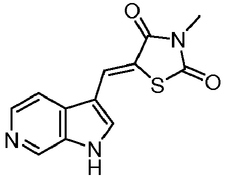 | (Z)-5-((1H-pyrrolo[2,3-c]pyridin-3-yl)methylene)-3-methylthiazolidi ne-2,4-dione | ¹H NMR (400 MHz, DMSO) δ 12.60 (s, 1H), 8.85 (d, J = 0.5 Hz, 1H), 8.28 (d, J = 5.5 Hz, 1H), 8.18 (s, 1H), 8.01 (s, 1H), 7.97 (dd, J = 5.5, 0.7 Hz, 1H), 3.11 (s, 3H). ¹³C NMR (400 MHz, DMSO) δ 167.52, 166.14, 140.24, 135.82, 133.85, 132.39, 131.93, 125.04, 116.16, 113.83, 110.32, 28.25. LRMS(ESI) : m/z 260 [M+H]⁺ |
| 70 | | 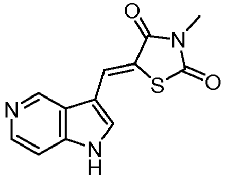 | (Z)-5-((1H-pyrrolo[3,2-c]pyridin-3-yl)methylene)-3-methylthiazolidine-2,4-dione | ¹H NMR (400 MHz, DMSO) δ 12.44 (s, 1H), 9.25 (s, 1H), 8.33 (d, J = 5.7 Hz, 1H), 8.26 (s, 1H), 7.88 (s, 1H), 7.49 (d, J = 5.7 Hz, 1H), 3.13 (s, 3H). LRMS(ESI) : m/z 260 [M+H]⁺ |
| 71 | | 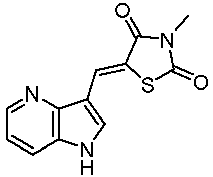 | (Z)-5-((1H-pyrrolo[3,2-b]pyridin-3-yl)methylene)-3-methylthiazolidi ne-2,4-dione | ¹H NMR (400 MHz, DMSO) δ 12.16 (s, 1H), 8.49 (dd, J = 4.6, 1.3 Hz, 1H), 8.22 (s, 1H), 8.13 (s, 1H), 7.90 (dd, J = 8.2, 1.3 Hz, 1H), 7.28 (dd, J = 8.2, 4.6 Hz, 1H), 3.10 (s, 3H). ¹³C NMR (400 MHz, DMSO) δ 168.78, 166.73, 144.28, 144.07, 133.76, 129.68, 125.66, 120.43, 118.76, 115.74, 111.06, 28.06. LRMS(ESI) : m/z 260 [M+H]⁺ |
| 72 | | 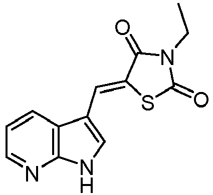 | (Z)-5-((1H-pyrrolo[2,3-b]pyridin-3-yl)methylene)-3-ethylthiazolidin e-2,4-dione | ¹H NMR (400 MHz, DMSO) δ 12.76 (s, 1H), 8.43 (dd, J = 7.9, 1.4 Hz, 1H), 8.37 (dd, J = 4.6, 1.4 Hz, 1H), 8.19 (s, 1H), 7.89 (s, 1H), 7.26 (dd, J = 7.9, 4.7 Hz, 1H), 3.69 (q, J = 7.1 Hz, 2H), 1.17 (t, J = 7.2 Hz, 3H). ¹³C NMR (400 MHz, DMSO) δ 167.33, 165.83, 149.14, 145.08, 129.80, 128.02, 125.56, 119.46, 117.73, 115.86, 109.69, 36.96, 13.35. LRMS(ESI) : m/z 274 [M+H]⁺ |
| 73 | DN207305 | 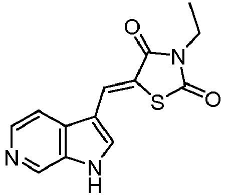 | (Z)-5-((1H-pyrrolo[2,3-c]pyridin-3-yl)methylene)-3-ethylthiazolidin e-2,4-dione | ¹H NMR (400 MHz, DMSO) δ 12.45 (s, 1H), 8.86 (s, 1H), 8.28 (d, J = 5.5 Hz, 1H), 8.19 (s, 1H), 8.02 (s, 1H), 7.97 (d, J = 5.0 Hz, 1H), 3.69 (q, J = 7.2 Hz, 2H), 1.17 (t, J = 7.2 Hz, 3H). ¹³C NMR (400 MHz, DMSO) δ 167.29, 165.80, 140.20, 135.83, 133.91, 132.56, 131.97, 125.27, 116.00, 113.83, 110.34, 36.99, 13.34. LRMS(ESI) : m/z 274 [M+H]⁺ |
| 74 | DN207049 | 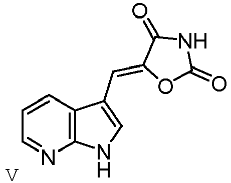 | (Z)-5-((1H-pyrrolo[2,3-b]pyridin-3-yl)methylene)oxazolidine-2,4-dione | ¹H NMR (400 MHz, DMSO-d₆) δ 9.96 (s, 1H), 8.51 (s, 1H), 8.49 8.37 (m, 2H), 7.31 (dd, J = 7.8, 4.7 Hz, 1H). |
| 75 | DN207111 | 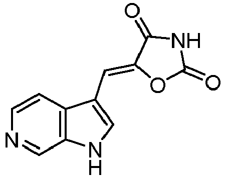 | (Z)-5-((1H-pyrrolo[2,3-c]pyridin-3-yl)methylene)oxa zolidine-2,4-dione | ¹H NMR (400 MHz, DMSO-d₆) δ 12.25 (s, 1H), 11.11 (s, 1H), 10.21 (s, 1H), 8.78 (s, 1H), 8.30 (s, 1H), 8.20 (d, J = 5.5 Hz, 1H), 7.79 (d, J = 5.4 Hz, 1H). |
| 76 | DN207109 | 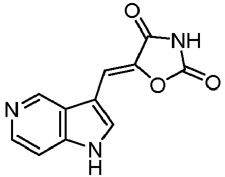 | (Z)-5-((1H-pyrrolo[3,2-c]pyridin-3-yl)methylene)oxa zolidine-2,4-dione | ¹H NMR (400 MHz, DMSO-d₆) δ 12.22 (s, 1H), 11.16 (s, 1H), 10.26 (s, 1H), 9.12 (s, 1H), 8.27 (d, J = 5.6 Hz, 1H), 8.19 (s, 1H), 7.46 (d, J = 5.3 Hz, 1H). |
| 77 | DN207110 | 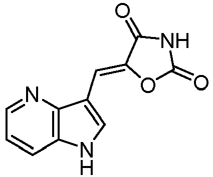 | (Z)-5-((1H-pyrrolo[3,2-b]pyridin-3-yl)methylene)oxa zolidine-2,4-dione | ¹H NMR (400 MHz, DMSO-d₆) δ 12.04 (s, 1H), 10.97 (s, 1H), 8.46 (d, J = 4.6 Hz, 1H), 8.12 (s, 1H), 7.95 (d, J = 8.2 Hz, 1H), 7.29 (dd, J = 8.2, 4.7 Hz, 1H), 6.65 (s, 1H). |
| 78 | DN207112 | 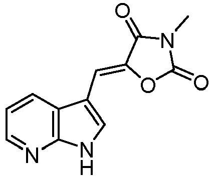 | (Z)-5-((1H-pyrrolo[2,3-b]pyridin-3-yl)methylene)-3-methyloxazolidin e-2,4-dione | ¹H NMR (400 MHz, DMSO-d₆) δ 12.51 (s, 1H), 8.55 8.47 (m, 1H), 8.33 (dd, J = 4.6, 1.3 Hz, 1H), 8.06 (d, J = 2.8 Hz, 1H), 7.29 7.19 (m, 2H), 3.03 (s, 3H). |
| 79 | DN207113 | 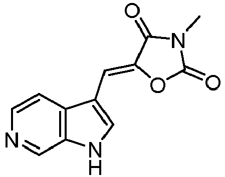 | (Z)-5-((1H-pyrrolo[2,3-c]pyridin-3-yl)methylene)-3-methyloxazolidin e-2,4-dione | ¹H NMR (400 MHz, MeOD) δ 8.90 (s, 1H), 8.41 (s, 1H), 8.20 (d, J = 6.2 Hz, 1H), 8.09 (d, J = 6.3 Hz, 1H), 6.89 (s, 1H), 3.00 (s, 3H). |
| 80 | DN207156 | 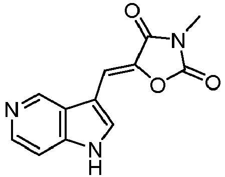 | (Z)-5-((1H-pyrrolo[3,2-c]pyridin-3-yl)methylene)-3-methyloxazolidin e-2,4-dione | ¹H NMR (400 MHz, CDCl₃) δ 8.97 (s, 1H), 8.32 (d, J = 5.9 Hz, 1H), 7.35 (d, J = 5.9 Hz, 1H), 7.26 (s, 1H), 6.69 (d, J = 2.9 Hz, 1H), 3.38 (s, 3H). |
| 81 | DN207114 | 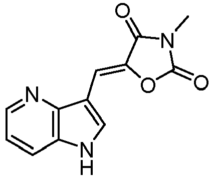 | (Z)-5-((1H-pyrrolo[3,2-b]pyridin-3-yl)methylene)-3-methyloxazolidin e-2,4-dione | ¹H NMR (400 MHz, DMSO-d₆) δ 8.51 (d, J = 4.3 Hz, 1H), 8.19 (s, 1H), 8.00 (s, 1H), 7.37 7.21 (m, 1H), 6.80 (s, 1H), 2.97 (s, 3H). |
| 82 | DN207157 | 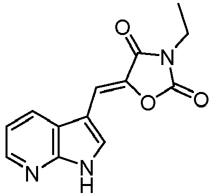 | (Z)-5-((1H-pyrrolo[2,3-b]pyridin-3-yl)methylene)-3-ethyloxazolidine -2,4-dione | ¹H NMR (400 MHz, DMSO-d₆) δ 12.49 (s, 1H), 8.50 (dd, J = 8.0, 1.5 Hz, 1H), 8.33 (dd, J = 4.6, 1.5 Hz, 1H), 8.06 (s, 1H), 7.35 7.01 (m, 2H), 3.57 (q, J = 7.2 Hz, 2H), 1.21 (t, J = 7.2 Hz, 3H). |
| 83 | DN207129 | 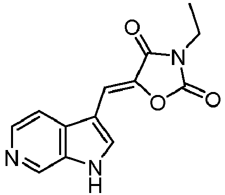 | (Z)-5-((1H-pyrrolo[2,3-c]pyridin-3-yl)methylene)-3-ethyloxazolidine -2,4-dione | ¹H NMR (400 MHz, DMSO-d₆) δ 8.94 (s, 1H), 8.53 8.23 (m, 2H), 8.19 (d, J = 5.7 Hz, 1H), 7.29 (s, 1H), 3.57 (q, J = 7.2 Hz, 2H), 1.21 (t, J = 7.2 Hz, 3H). |
| 84 | DN207130 | 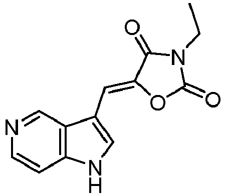 | (Z)-5-((1H-pyrrolo[3,2-c]pyridin-3-yl)methylene)-3-ethyloxazolidine -2,4-dione | ¹H NMR (400 MHz, DMSO-d₆) δ 12.26 (s, 1H), 9.33 (s, 1H), 8.29 (d, J = 5.6 Hz, 1H), 8.04 (s, 1H), 7.47 (d, J = 5.6 Hz, 1H), 7.29 (s, 1H), 3.57 (q, J = 7.2 Hz, 2H), 1.21 (t, J = 7.2 Hz, 3H). |
| 85 | DN207131 | 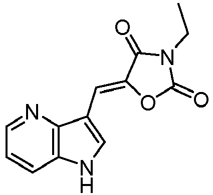 | (Z)-5-((1H-pyrrolo[3,2-b]pyridin-3-yl)methylene)-3-ethyloxazolidine -2,4-dione | ¹H NMR (400 MHz, MeOD) δ 8.64 8.35 (m, 1H), 8.26 (s, 1H), 8.07 7.84 (m, 1H), 7.45 (s, 1H), 7.32 (dd, J = 8.2, 4.7 Hz, 1H), 3.71 (q, J = 7.2 Hz, 2H), 1.32 (t, J = 7.2 Hz 3H). |
| 86 | DN207115 | 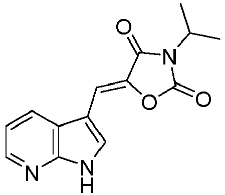 | (Z)-5-((1H-pyrrolo[2,3-b]pyridin-3-yl)methylene)-3-isopropyloxazoli dine-2,4-dione | ¹H NMR (400 MHz, MeOD) δ 8.45 (dd, J = 8.0, 1.4 Hz, 1H), 8.32 (dd, J = 4.8, 1.4 Hz, 1H), 8.06 (s, 1H), 7.28 (dd, J = 8.0, 4.8 Hz, 1H), 7.14 (s, 1H), 4.43 (dt, J = 13.8, 6.9 Hz, 1H), 1.50 (d, J = 6.9 Hz, 6H). |
| 87 | DN207116 | 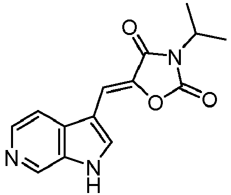 | (Z)-5-((1H-pyrrolo[2,3-c]pyridin-3-yl)methylene)-3-isopropyloxazoli dine-2,4-dione | ¹H NMR (400 MHz, MeOD) δ 8.80 (s, 1H), 8.29 8.20 (m, 2H), 8.01 (d, J = 5.7 Hz, 1H), 7.17 (s, 1H), 4.43 (dt, J = 13.8, 6.9 Hz, 1H), 1.50 (d, J = 6.9 Hz, 6H). |
| 88 | DN207117 | 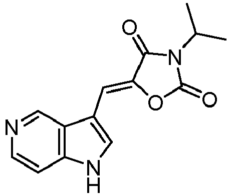 | (Z)-5-((1H-pyrrolo[3,2-c]pyridin-3-yl)methylene)-3-isopropyloxazoli dine-2,4-dione | ¹H NMR (400 MHz, MeOD) δ 9.23 (s, 1H), 8.29 (d, J = 5.8 Hz, 1H), 8.05 (s, 1H), 7.53 (d, J = 5.8 Hz, 1H), 7.21 (s, 1H), 4.43 (dt, J = 13.8, 6.9 Hz, 1H), 1.50 (d, J = 6.9 Hz, 6H). |
| 89 | DN207118 | 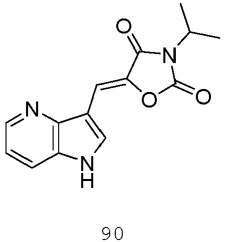 | (Z)-5-((1H-pyrrolo[3,2-b]pyridin-3-yl)methylene)-3-isopropyloxazoli dine-2,4-dione | ¹H NMR (400 MHz, MeOD) δ 8.45 (dd, J = 4.7, 1.1 Hz, 1H), 8.25 (s, 1H), 7.94 (dd, J = 8.2, 1.2 Hz, 1H), 7.41 (s, 1H), 7.31 (dd, J = 8.2, 4.7 Hz, 1H), 4.42 (dt, J = 13.8, 6.9 Hz, 1H), 1.50 (d, J = 6.9 Hz, 6H). |
| 90 | DN206760 | 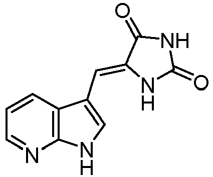 | (Z)-5-((1H-pyrrolo[2,3-b]pyridin-3-yl)methylene)imi dazolidine-2,4-dione | ¹H NMR (400 MHz, DMSO-d₆) δ 8.33 8.20 (m, 3H), 7.17 (dd, J = 7.9, 4.7 Hz, 1H), 6.73 (s, 1H). |
| 91 | DN206763 | 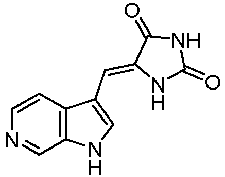 | (Z)-5-((1H-pyrrolo[2,3-c]pyridin-3-yl)methylene)imidazolidine-2,4-dione | ¹H NMR (400 MHz, DMSO-d₆) δ 8.78 (s, 1H), 8.31 (s, 1H), 8.20 (d, J = 5.5 Hz, 1H), 7.80 (d, J = 5.5 Hz, 1H), 6.73 (s, 1H). |
| 92 | DN206761 | 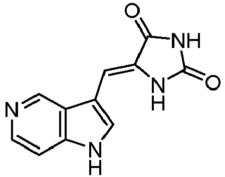 | (Z)-5-((1H-pyrrolo[3,2-c]pyridin-3-yl)methylene)imi dazolidine-2,4-dione | ¹H NMR (400 MHz, DMSO-d₆) δ 12.15 (s, 1H), 11.13 (s, 1H), 10.22 (s, 1H), 9.08 (s, 1H), 8.26 (d, J = 5.7 Hz, 1H), 8.16 (s, 1H), 7.43 (d, J = 5.8 Hz, 1H), 6.80 (s, 1H). |
| 93 | DN206762 | 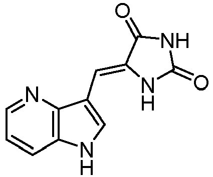 | (Z)-5-((1H-pyrrolo[3,2-b]pyridin-3-yl)methylene)imi dazolidine-2,4-dione | ¹H NMR (400 MHz, DMSO-d₆) δ 12.19 (s, 1H), 8.46 (dd, J = 4.7, 1.3 Hz, 1H), 8.10 (s, 1H), 7.95 (dd, J = 8.2, 1.3 Hz, 1H), 7.30 (dd, J = 8.2, 4.7 Hz, 1H), 6.64 (s, 1H). |
| 94 | DN206759 | 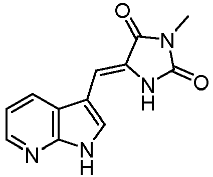 | (Z)-5-((1H-pyrrolo[2,3-b]pyridin-3-yl)methylene)-3-methylimidazolid ine-2,4-dione | ¹H NMR (400 MHz, DMSO-d₆) δ 8.33 8.21 (m, 3H), 7.18 (dd, J = 7.9, 4.7 Hz, 1H), 6.85 (s, 1H), 2.97 (s, 3H). |
| 95 | DN206765 | 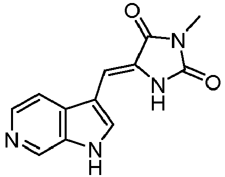 | (Z)-5-((1H-pyrrolo[2,3-c]pyridin-3-yl)methylene)-3-methylimidazolid ine-2,4-dione | ¹H NMR (400 MHz, DMSO-d₆) δ 8.79 (s, 1H), 8.32 (s, 1H), 8.21 (d, J = 5.5 Hz, 1H), 7.82 (d, J = 5.5 Hz, 1H), 6.84 (s, 1H), 2.98 (s, 3H). |
| 96 | DN206764 | 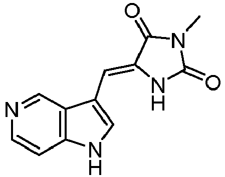 | (Z)-5-((1H-pyrrolo[3,2-c]pyridin-3-yl)methylene)-3-methylimidazolid ine-2,4-dione | ¹H NMR (400 MHz, DMSO-d₆) δ 9.09 (s, 1H), 8.26 (d, J = 5.7 Hz, 1H), 8.16 (s, 1H), 7.42 (d, J = 5.7 Hz, 1H), 6.90 (s, 1H), 2.98 (s, 3H). |
| | | | | |
| 97 | DN207162 | 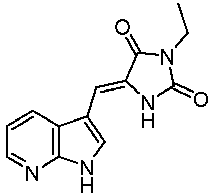 | (Z)-5-((1H-pyrrolo[2,3-b]pyridin-3-yl)methylene)-3-ethylimidazolidi ne-2,4-dione | ¹H NMR (400 MHz, DMSO-d₆) δ 12.72 (s, 1H), 9.93 (s, 1H), 8.48 (s, 1H), 8.39 (dd, J = 13.1, 6.2 Hz, 2H), 8.02 (s, 1H), 7.29 (dd, J = 7.8, 4.7 Hz, 1H), 3.37 (q, J = 7.1 Hz, 2H), 1.07 (t, J = 7.2 Hz, 3H). |
| 98 | DN206766 | 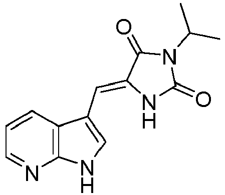 | (Z)-5-((1H-pyrrolo[2,3-b]pyridin-3-yl)methylene)-3-isopropylimidazo lidine-2,4-dione | ¹H NMR (400 MHz, DMSO- d₆) δ 12.30 (s, 1H), 10.40 (s, 1H), 8.34 8.20 (m, 3H), 7.17 (dd, J = 7.9, 4.7 Hz, 1H), 6.80 (s, 1H), 4.37 4.22 (m, 1H), 1.38 (d, J = 6.9 Hz, 6H). |
| 99 | DN206769 | 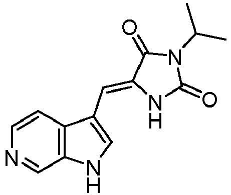 | (Z)-5-((1H-pyrrolo[2,3-c]pyridin-3-yl)methylene)-3-isopropylimidazo lidine-2,4-dione | ¹H NMR (400 MHz, DMSO- d₆) δ 12.28 (s, 1H), 10.40 (s, 1H), 8.88 8.71 (m, 1H), 8.31 (s, 1H), 8.21 (d, J = 5.4 Hz, 1H), 7.80 (d, J = 5.3 Hz, 1H), 6.80 (s, 1H), 4.30 (dt, J = 13.7, 6.8 Hz, 1H), 1.39 (d, J = 6.8 Hz, 6H). |
| 100 | DN206767 | 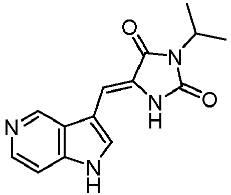 | (Z)-5-((1H-pyrrolo[3,2-c]pyridin-3-yl)methylene)-3-isopropylimidazo lidine-2,4-dione | ¹H NMR (400 MHz, DMSO-d₆) δ 12.16 (s, 1H), 10.43 (s, 1H), 9.08 (s, 1H), 8.26 (d, J = 5.6 Hz, 1H), 8.16 (s, 1H), 7.42 (d, J = 5.7 Hz, 1H), 6.87 (s, 1H), 4.55 4.06 (m, 1H), 1.39 (d, J = 6.9 Hz, 6H) . |
| 101 | DN206768 | 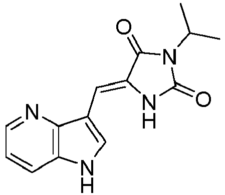 | (Z)-5-((1H-pyrrolo[3,2-b]pyridin-3-yl)methylene)-3-isopropylimidazo lidine-2,4-dione | ¹H NMR (400 MHz, MeOD) δ 8.43 (d, J = 4.7 Hz, 1H), 7.86 (d, J = 9.8 Hz, 2H), 7.26 (dd, J = 8.0, 4.9 Hz, 1H), 6.74 (s, 1H), 4.71 4.34 (m, 1H), 1.40 (d, J = 6.9 Hz, 6H). |
| 102 | DN205355 | 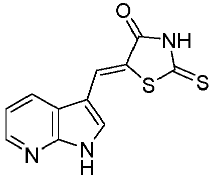 | (Z)-5-((1H-pyrrolo[2,3-b]pyridin-3-yl)methylene)-2-thioxothiazolidi n-4-one | ¹H NMR (400 MHz, DMSO-d₆) δ 13.62 (s, 1H), 11.96 (s, 1H), 8.43 (dd, J = 8.0, 1.4 Hz, 1H), 8.37 (dd, J = 4.7, 1.5 Hz, 1H), 7.93 (s, 1H), 7.90 (s, 1H), 7.27 (dd, J = 7.9, 4.7 Hz, 1H). |
| 103 | DN206962 | 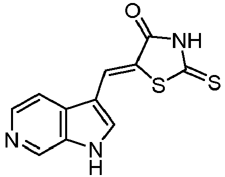 | (Z)-5-((1H-pyrrolo[2,3-c]pyridin-3-yl)methylene)-2-thioxothiazolidi n-4-one | ¹H NMR (400 MHz, DMSO-d₆) δ 11.97 (s, 1H), 8.86 (d, J = 0.8 Hz, 1H), 8.30 (d, J = 5.6 Hz, 1H), 8.08 7.95 (m, 2H), 7.91 (s, 1H). |
| 104 | LJS 022-037 | 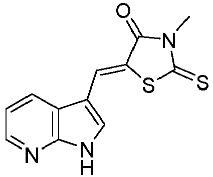 | (Z)-5-((1H-pyrrolo[2,3-b]pyridin-3-yl)methylene)-3-methyl-2-thioxothiazolidi n-4-one | ¹H NMR (400 MHz, DMSO-d₆) δ 12.68 (s, 2H), 8.46 8.33 (m, 2H), 7.97 (s, 1H), 7.88 (d, J = 2.4 Hz, 1H), 7.26 (dd, J = 7.9, 4.7 Hz, 1H), 2.74 (s, 3H). |
| 105 | DN206963 | 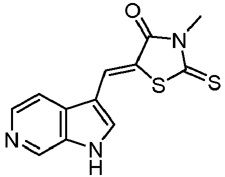 | (Z)-5-((1H-pyrrolo[2,3-c]pyridin-3-yl)methylene)-3-methyl-2-thioxothiazolidi n-4-one | ¹H NMR (400 MHz, DMSO-d₆) δ 12.02 (s, 1H), 8.85 (d, J = 0.9 Hz, 1H), 8.30 (d, J = 5.5 Hz, 1H), 8.11 (d, J = 5.6 Hz, 2H), 8.02 (dd, J = 5.5, 0.9 Hz, 1H), 3.42 (s, 3H). |
| 106 | DN206964 | 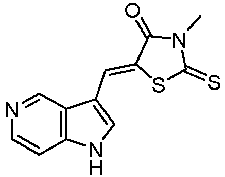 | (Z)-5-((1H-pyrrolo[3,2-c]pyridin-3-yl)methylene)-3-methyl-2-thioxothiazolidi n-4-one | ¹H NMR (400 MHz, DMSO-d₆) δ 12.85 (s, 1H), 11.98 (s, 1H), 9.56 (s, 1H), 8.42 (d, J = 5.9 Hz, 1H), 8.04 (s, 1H), 7.92 7.67 (m, 2H), 2.68 (s, 3H). |
| 107 | DN206965 | 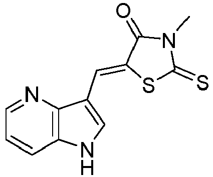 | (Z)-5-((1H-pyrrolo[3,2-b]pyridin-3-yl)methylene)-3-methyl-2-thioxothiazolidi n-4-one | ¹H NMR (400 MHz, DMSO-d₆) δ 12.25 (s, 1H), 8.80 8.28 (m, 1H), 8.18 8.03 (m, 2H), 7.93 (d, J = 8.1 Hz, 1H), 7.29 (dd, J = 8.2, 4.6 Hz, 1H), 2.74 (s, 3H). |
| 108 | DN206966 | 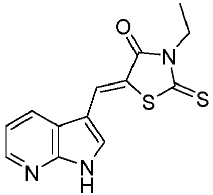 | (Z)-5-((1H-pyrrolo[2,3-b]pyridin-3-yl)methylene)-3-ethyl-2-thioxothiazolidi n-4-one | ¹H NMR (400 MHz, DMSO-d₆) δ 8.46 (dd, J = 7.9, 1.4 Hz, 1H), 8.38 (d, J = 4.7 Hz, 1H), 8.10 (s, 1H), 7.99 (s, 1H), 7.28 (dd, J = 8.0, 4.7 Hz, 1H), 4.08 (q, J = 7.1 Hz, 7.1 Hz, 2H), 1.20 (t, J = 7.1 Hz, 3H). |
| 109 | DN207045 | 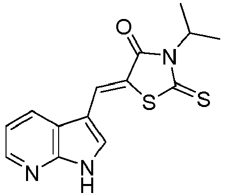 | (Z)-5-((1H-pyrrolo[2,3-b]pyridin-3-yl)methylene)-3-isopropyl-2-thioxothiazolidi n-4-one | ¹H NMR (400 MHz, DMSO-d₆) δ 12.88 (s, 1H), 8.45 (dd, J = 7.9, 1.4 Hz, 1H), 8.38 (dd, J = 4.6, 1.3 Hz, 1H), 8.02 (s, 1H), 7.97 (s, 1H), 7.28 (dd, J = 7.9, 4.7 Hz, 1H), 5.29 (dt, J = 13.8, 6.9 Hz, 1H), 1.50 (d, J = 6.9 Hz, 6H). |
| 110 | DN207047 | 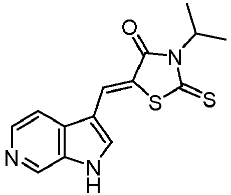 | (Z)-5-((1H-pyrrolo[2,3-c]pyridin-3-yl)methylene)-3-isopropyl-2-thioxothiazolidi n-4-one | ¹H NMR (400 MHz, DMSO-d₆) δ 12.49 (s, 1H), 8.85 (s, 1H), 8.30 (d, J = 5.5 Hz, 1H), 8.08 (s, 1H), 8.03 (s, 1H), 7.99 (d, J = 5.5 Hz, 1H), 5.29 (dt, J = 13.8, 6.9 Hz, 1H), 1.50 (d, J = 6.9 Hz, 6H). |
| 111 | DN207048 | 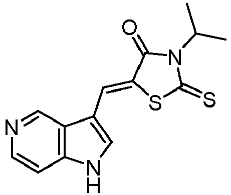 | (Z)-5-((1H-pyrrolo[3,2-c]pyridin-3-yl)methylene)-3-isopropyl-2-thioxothiazolidi n-4-one | ¹H NMR (400 MHz, DMSO-d₆) δ 12.39 (s, 1H), 9.26 (s, 1H), 8.34 (d, J = 5.7 Hz, 1H), 8.10 (s, 1H), 7.94 (s, 1H), 7.49 (d, J = 5.7 Hz, 1H), 5.47 4.94 (m, 1H), 1.50 (d, J = 6.9 Hz, 6H) |
| 112 | DN207046 | 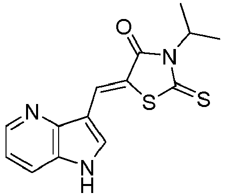 | (Z)-5-((1H-pyrrolo[3,2-b]pyridin-3-yl)methylene)-3-isopropyl-2-thioxothiazolidi n-4-one | ¹H NMR (400 MHz, DMSO-d₆) δ 12.39 (s, 1H), 8.62 8.36 (m, 1H), 8.28 (s, 1H), 7.99 (s, 1H), 7.91 (d, J = 8.2 Hz, 1H), 7.29 (dd, J = 8.2, 4.6 Hz, 1H), 5.30 (dt, J = 13.8, 7.0 Hz, 1H), 1.49 (d, J = 6.9 Hz, 6H). |
| 113 | DN207121 | 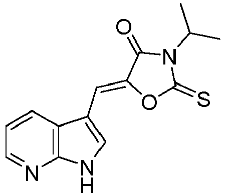 | (Z)-5-((1H-pyrrolo[2,3-b]pyridin-3-yl)methylene)-3-isopropyl-2-thioxooxazolidin -4-one | ¹H NMR (400 MHz, DMSO-d₆) δ 12.70 (s, 1H), 8.60 (dd, J = 8.0, 1.4 Hz, 1H), 8.36 (dd, J = 4.7, 1.4 Hz, 1H), 8.18 (s, 1H), 7.40 7.15 (m, 2H), 4.73 (dt, J = 13.9, 6.9 Hz, 1H), 1.48 (d, J = 6.9 Hz, 6H). |
| 114 | DN207122 | 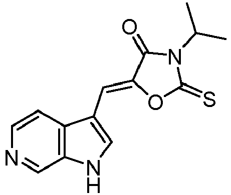 | (Z)-5-((1H-pyrrolo[2,3-c]pyridin-3-yl)methylene)-3-isopropyl-2-thioxooxazolidin -4-one | ¹H NMR (400 MHz, DMSO-d₆) δ 12.55 (s, 1H), 8.86 (s, 1H), 8.46 8.15 (m, 2H), 8.12 (s, 1H), 7.29 (s, 1H), 4.91 4.53 (m, 1H), 1.48 (d, J = 6.9 Hz, 6H). |
| 115 | DN207153 | 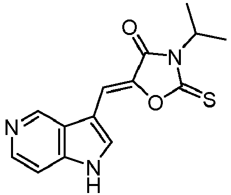 | (Z)-5-((1H-pyrrolo[3,2-c]pyridin-3-yl)methylene)-3-isopropyl-2-thioxooxazolidin -4-one | ¹H NMR (400 MHz, MeOD) δ 8.45 (d, J = 3.5 Hz, 1H), 8.25 (s, 1H), 7.95 (d, J = 8.2 Hz, 1H), 7.42 (s, 1H), 7.32 (dd, J = 8.2, 4.7 Hz, 1H), 4.43 (dt, J = 13.8, 6.9 Hz, 1H), 1.50 (d, J = 6.9 Hz, 6H) |
| 116 | DN207123 | 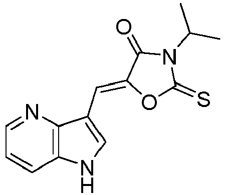 | (Z)-5-((1H-pyrrolo[3,2-b]pyridin-3-yl)methylene)-3-isopropyl-2-thioxooxazolidin -4-one | ¹H NMR (400 MHz, DMSO-d₆) δ 12.29 (s, 1H), 8.49 (dd, J = 4.6, 1.2 Hz, 1H), 8.27 (s, 1H), 7.93 (dd, J = 8.2, 1.2 Hz, 1H), 7.28 (dd, J = 8.2, 4.6 Hz, 1H), 7.20 (s, 1H), 5.02 4.63 (m, 1H), 1.48 (d, J = 6.9 Hz, 6H). |
| 117 | DN207124 | 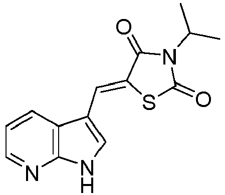 | (Z)-5-((1H-pyrrolo[2,3-b]pyridin-3-yl)methylene)-3-isopropylthiazol idine-2,4-dione | ¹H NMR (400 MHz, DMSO-d₆) δ 8.38 (dd, J = 18.3, 5.1 Hz, 2H), 8.15 (s, 1H), 7.87 (s, 1H), 7.24 (dd, J = 7.8, 4.6 Hz, 1H), 4.55 (dt, J = 13.8, 7.0 Hz, 1H), 1.40 (d, J = 6.9 Hz, 6H). |
| 118 | DN207125 | 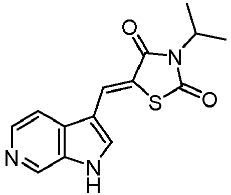 | (Z)-5-((1H-pyrrolo[2,3-c]pyridin-3-yl)methylene)-3-isopropylthiazol idine-2,4-dione | ¹H NMR (400 MHz, DMSO-d₆) δ 12.25 (s, 1H), 8.85 (s, 1H), 8.27 (d, J = 5.5 Hz, 1H), 8.16 (s, 1H), 8.01 7.91 (m, 2H), 4.62 4.28 (m, 1H), 1.41 (d, J = 6.9 Hz, 6H) |
| 119 | DN207126 | 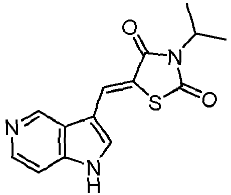 | (Z)-5-((1H-pyrrolo[3,2-c]pyridin-3-yl)methylene)-3-isopropylthiazol idine-2,4-dione | ¹H NMR (400 MHz, DMSO-d₆) δ 12.40 (s, 1H), 9.22 (s, 1H), 8.32 (d, J = 5.6 Hz, 1H), 8.22 (s, 1H), 7.86 (s, 1H), 7.48 (d, J = 5.7 Hz, 1H), 4.55 (dt, J = 13.8, 6.9 Hz, 1H), 1.40 (d, J = 6.9 Hz, 6H). |
| 120 | DN207127 | 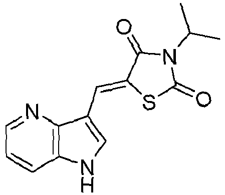 | (Z)-5-((1H-pyrrolo[3,2-b]pyridin-3-yl)methylene)-3-isopropylthiazol idine-2,4-dione | ¹H NMR (400 MHz, DMSO-d₆) δ 12.23 (s, 1H), 8.49 (dd, J = 4.6, 1.3 Hz, 1H), 8.21 (s, 1H), 8.11 (s, 1H), 7.90 (dd, J = 8.2, 1.3 Hz, 1H), 7.27 (dd, J = 8.2, 4.6 Hz, 1H), 4.82 4.23 (m, 1H), 1.41 (d, J = 6.9 Hz, 6H). |
| 121 | DN207155 | 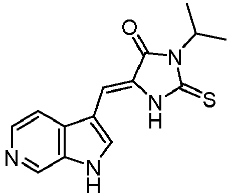 | (Z)-5-((1H-pyrrolo[2,3-c]pyridin-3-yl)methylene)-3-isopropyl-2-thioxoimidazolid in-4-one | ¹H NMR (400 MHz, DMSO-d₆) δ 12.33 (s, 1H), 9.08 (s, 1H), 8.84 (s, 1H), 8.27 (d, J = 5.5 Hz, 1H), 7.64 (d, J = 5.5 Hz, 1H), 7.00 (s, 1H), 5.02 4.56 (m, 1H), 1.45 (d, J = 6.9 Hz, 6H). |
| 122 | DN207127 | 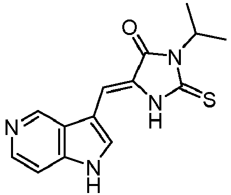 | (Z)-5-((1H-pyrrolo[3,2-c]pyridin-3-yl)methylene)-3-isopropyl-2-thioxoimidazolid in-4-one | ¹H NMR (400 MHz, MeOD) δ 9.10 (s, 1H), 8.30 (d, J = 5.9 Hz, 1H), 8.25 (s, 1H), 7.58 (d, J = 5.9 Hz, 1H), 6.98 (s, 1H), 5.05 (dq, J = 14.0, 7.0 Hz, 1H), 1.52 (d, J = 7.0 Hz, 6H). |
| 123 | DN207128 | 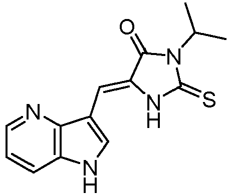 | (Z)-5-((1H-pyrrolo[3,2-b]pyridin-3-yl)methylene)-3-isopropyl-2-thioxoimidazolid in-4-one | ¹H NMR (400 MHz, MeOD) δ 8.49 (dd, J = 4.7, 1.3 Hz, 1H), 7.95 (s, 1H), 7.89 (dd, J = 8.3, 1.3 Hz, 1H), 7.29 (dd, J = 8.3, 4.8 Hz, 1H), 6.75 (s, 1H), 5.04 (dq, J = 13.9, 7.0 Hz, 1H), 1.52 (d, J = 7.0 Hz, 6H). |

### Example 2. Beta-amyloid aggregation inhibition test

The inhibitory effect of the test compound against the aggregation of beta-amyloids was measured by thioflavin assay using thioflavin T (ThT). ThT is fluorescent when bound to a beta-sheet-rich structure such as a beta-amyloid aggregate, so the intensity of fluorescence, measured using a microplate fluorescence reader, indicates the degree to which each compound inhibits aggregation of beta-amyloids. For use in thioflavin assay, thioflavin-T (Sigma-Aldrich) was dissolved at a concentration of 5 mM in 50 mM of glycine buffer (pH 8.5). Then, the resulting solution was diluted to 5 uM using 50 mM glycine buffer (pH 8.5) and stored in a dark laboratory in the absence of light.

Aβ (beta-amyloid; human Aβ 1-42 monomer (UniProtKB-P05067, a.a. 672-713)) was dissolved at a concentration of 10 mM in dimethyl sulfoxide (DMSO), and each compound synthesized in Example 1 (50, 500 µM; hereinafter, referred to as "test compound") was mixed with beta-amyloid such that the final concentration thereof reached 25 µM, and then aggregation of beta-amyloids was induced at 37°C for 3 days.

25 µl of the reaction solution was charged in each well of a 96-well plate for fluorescence analysis and 75 µl of the prepared thioflavin-T solution was charged in each well. The reaction was allowed to proceed at room temperature in the absence of light for 5 minutes, and then fluorescence values were measured at an excitation wavelength of 450 nm and an emission wavelength of 485 nm using a multi-mode microplate reader.

The ratio of the fluorescence value measured in the group obtained by treating with each test compound to 100% of the fluorescence value of the group (control group) obtained by treating only with beta-amyloids and inducing aggregation for 3 days is set forth in Table 3 below:

**[Table 3]**

| Test compounds | 50 uM | 500 uM |
|---|---|---|
| DN204296 | 71.07 | 12.21 |
| DN204297 | N.T. | 50.79 |
| DN204298 | 75.21 | 39.61 |
| DN204299 | N.T. | 88.83 |
| DN204301 | N.T. | 93.19 |
| DN204302 | 94.18 | 92.05 |
| DN204303 | N.T. | 78.10 |
| DN204304 | 92.88 | 73.49 |
| DN204289 | N.T. | 69.93 |
| DN204290 | N.T. | 94.90 |
| DN204286 | N.T. | 75.33 |
| DN204294 | N.T. | 87.87 |
| DN204307 | N.T. | 92.60 |
| DN204669 | 84.52 | 81.72 |
| DN204670 | 97.66 | 99.21 |
| DN204671 | 93.64 | N.T. |
| DN204672 | N.T. | 94.99 |
| DN204673 | 58.75 | 24.00 |
| DN204807 | N.T. | 62.37 |
| DN204808 | 89.04 | 72.37 |
| DN204809 | 87.52 | 56.51 |
| DN204810 | 73.36 | 81.82 |
| DN204811 | 72.38 | 66.92 |
| DN204812 | 77.54 | 75.08 |
| DN204814 | 66.60 | -30.47 |
| DN204815 | 91.36 | 86.65 |
| DN204816 | 75.68 | 59.37 |
| DN204817 | 78.51 | 71.39 |
| DN204818 | 65.20 | 30.26 |
| DN205106 | 88.62 | 69.10 |
| DN205107 | 72.97 | 71.30 |
| DN205108 | 82.19 | 73.38 |
| DN205109 | 88.68 | 56.97 |
| DN205110 | 84.63 | 90.96 |
| DN205111 | 96.92 | 94.99 |
| DN205353 | 90.73 | 90.30 |
| DN205354 | 95.41 | 53.64 |
| DN205355 | 86.14 | 31.32 |
| DN205779 | N.T. | 73.87 |
| DN205780 | 80.24 | 32.80 |
| DN205781 | 88.25 | 49.47 |
| DN205782 | 93.20 | 69.68 |
| DN205783 | 92.17 | 83.37 |

### Beta-amyloid aggregation inhibitory activity (% of control)

### (N.T.: not tested)

As can be seen from Table 3, the compounds disclosed herein exhibit beta-amyloid aggregation inhibitory activity ranging from about 5% or more to about 75% or more of that of the control group.

### Example 3. Beta-amyloid aggregate degradation test

Aβ (beta-amyloid; human Aβ 1-42 monomer (UniProtKB-P05067, a.a. 672-713)) was dissolved at a concentration of 10 mM in dimethyl sulfoxide (DMSO), and the resulting solution was diluted to 250 uM using distilled water, and then incubated at 37°C for 3 days to induce aggregation. The aggregated beta-amyloid was added with each test compound in an amount of 50 or 500 uM such that the final concentration reached 25 uM, and was further cultured at 37°C for 3 days to induce reaction.

The aggregate degradation effect of each test compound was measured by thioflavin T (ThT) assay (see Example 2). Specifically, 25 uL of the reaction solution was charged in each well of a 96-well plate for fluorescence analysis and 75 uL of the prepared thioflavin-T solution was charged in each well. The reaction was allowed to proceed at room temperature in a dark laboratory for 5 minutes and then fluorescence values were measured at an excitation wavelength of 450 nm and an emission wavelength of 485 nm using a multi-mode microplate reader.

The ratio of the fluorescence value measured in the group obtained by treating with each test compound to 100% of the fluorescence value of the group (control group) obtained by treating only with beta-amyloids and inducing aggregation for 3 days is set forth in Table 4 below:

**[Table 4]**

| Test compounds | 50 uM | 500 uM |
|---|---|---|
| DN204296 | 68.90 | 17.28 |
| DN204297 | 86.80 | 28.19 |
| DN204298 | 59.37 | 7.79 |
| DN204299 | N.T. | 80.07 |
| DN204303 | 90.66 | 33.65 |
| DN204304 | 97.47 | 73.11 |
| DN204289 | 83.66 | 54.07 |
| DN204286 | N.T. | 94.23 |
| DN204294 | 88.64 | 74.80 |
| DN204673 | 86.89 | 30.52 |
| DN204807 | N.T. | 53.56 |
| DN204808 | N.T. | 73.30 |
| DN204809 | 95.14 | 25.32 |
| DN204811 | 74.64 | 28.97 |
| DN204812 | N.T. | 89.68 |
| DN204814 | 58.02 | -40.60 |
| DN204816 | N.T. | 92.41 |
| DN204817 | N.T. | 78.39 |
| DN204818 | 95.08 | 35.83 |
| DN205107 | N.T. | 91.94 |
| DN205109 | N.T. | 83.20 |
| DN205353 | N.T. | 90.19 |
| DN205354 | N.T. | 64.83 |
| DN205355 | 93.80 | 44.61 |
| DN205780 | 51.31 | 35.73 |
| DN205781 | 85.76 | 55.35 |

| | | |
|---|---|---|
| Beta-amyloid aggregate degradation activity (% of control) (N.T.: not tested) | | |

As can be seen from Table 4 above, the compounds disclosed herein exhibit beta-amyloid aggregate degradation activity ranging from about 5% or more to about 90% or more of that of the control group.

### Example 4. Tau protein aggregation inhibition test

Tau protein (wild-type K18) was dissolved in phosphate-buffered saline (PBS, pH 7.4) at a concentration of 1 mg/mL to prepare a tau protein solution. In order to induce aggregation of tau proteins, 0.1 mg/mL of heparin and 100 uM dithiothreitol (DTT) were added to 1 mg/mL of the tau protein solution, followed by incubation at 37°C for 5 days. At this time, the result was mixed with each test compound (50, 500 uM) such that the final concentration reached 0.5 mg/mL, followed by further incubation.

The inhibitory effect of each compound against tau aggregation was measured based on the intensity of fluorescence by thioflavin T (ThT) assay (see Example 2). More specifically, 25 uL of the reaction solution was charged in each well of a 96-well plate for fluorescence analysis, and 75 uL of the prepared thioflavin-T solution was then charged in each well. The reaction was allowed to proceed at room temperature in a dark laboratory for 5 minutes, and then fluorescence values were measured at an excitation wavelength of 450 nm and an emission wavelength of 485 nm using a multi-mode microplate reader.

The ratio of the fluorescence value measured in the group obtained by treating with each test compound to 100% of the fluorescence value of the group (control group) obtained by treating only with tau proteins and inducing aggregation for 5 days is set forth in Table 5 below:

**[Table 5]**

| Test compounds | 50 uM | 500 uM |
|---|---|---|
| DN204296 | 13.243 | 4.984 |
| DN204297 | 48.721 | 16.233 |
| DN204298 | 71.163 | 33.817 |
| DN204299 | 60.521 | 33.932 |
| DN204300 | 92.633 | N.T. |
| DN204301 | 90.337 | 39.019 |
| DN204302 | 67.16 | 47.38 |
| DN204303 | 82.4 | 29.14 |
| DN204304 | 70.66 | 39.54 |
| DN204305 | 83.45 | 56.13 |
| DN204306 | 66.31 | 52.16 |
| DN204287 | 69.16 | 59.86 |
| DN204288 | 74.09 | 80.79 |
| DN204289 | 60.71 | 46.16 |
| DN204290 | 63.13 | 42.61 |
| DN204285 | 53.14 | 42.56 |
| DN204284 | 80.94 | N.T. |
| DN204295 | 74.98 | 59.6 |
| DN204292 | 63.18 | 60.16 |
| DN204293 | 55.97 | 58.22 |
| DN204291 | 65.99 | N.T. |
| DN204286 | 56.04 | 82.71 |
| DN204294 | 85.91 | N.T. |
| DN204307 | N.T. | 57.26 |
| DN204669 | 94.79 | 48.9 |
| DN204670 | 87.73 | N.T. |
| DN204671 | 88.61 | 77.02 |
| DN204673 | N.T. | 55.87 |
| DN204807 | 93.56 | 74.24 |
| DN204808 | 27.48 | 16.09 |
| DN204809 | 36.72 | 32.09 |
| DN204810 | 87.61 | 65.66 |
| DN204811 | 43.22 | 40.21 |
| DN204812 | 72.49 | 49.52 |
| DN204814 | N.T. | 28.47 |
| DN204815 | N.T. | 70.7 |
| DN204816 | 81.42 | 56.3 |
| DN204817 | 87.3 | 66.18 |
| DN204818 | 20.35 | 1.71 |
| DN205106 | 72.17 | 56.3 |
| DN205107 | 60.34 | 40.23 |
| DN205108 | 82.78 | 71.29 |
| DN205109 | 86.52 | 47.52 |
| DN205110 | 96 | 76.31 |
| DN205111 | 91.5 | 83.98 |
| DN205353 | 61.551 | 66.948 |
| DN205354 | 72.646 | 46.964 |
| DN205355 | 40.038 | 25.883 |
| DN205779 | 83.56 | 62.51 |
| DN205780 | 69.83 | 33.02 |
| DN205781 | 74.46 | 52.99 |
| DN205782 | 97.55 | 75.35 |
| DN205783 | 81.81 | 69.79 |
| DN205821 | 70.81 | 79.94 |
| DN205822 | 83.97 | N.T. |

| | | |
|---|---|---|
| Tau protein aggregation inhibitory activity (% of control) (N.T.: not tested) | | |

As can be seen from Table 5 above, the compounds disclosed herein exhibit tau protein aggregation inhibitory activity ranging from about 5% or more to about 90% or more of that of the control group.

### Example 5. Tau protein aggregate degradation test

Tau protein (wild-type K18) was dissolved in phosphate-buffered saline (PBS, pH 7.4) at a concentration of 1 mg/mL to prepare a tau protein solution. 0.1 mg/mL of heparin and 100 uM dithiothreitol (DTT) were added to 1 mg/mL of the tau protein solution, followed by incubation at 37°C for 5 days to induce tau protein aggregation. The aggregated tau protein was mixed with each test compound in an amount of 50 or 500 uM such that the final concentration reached 0.5 mg/mL, followed by further incubation at 37°C for 5 days to induce reaction.

The degradation effect of each compound on the tau aggregates was measured by thioflavin T (ThT) assay (see Example 2). More specifically, 25 uL of the reaction solution was charged in each well of a 96-well plate for fluorescence analysis and 75 uL of the prepared thioflavin-T solution was charged in each well. The reaction was allowed to proceed at room temperature in a dark laboratory for 5 minutes and then fluorescence values were measured at an excitation wavelength of 450 nm and an emission wavelength of 485 nm using a multi-mode microplate reader.

The ratio of the fluorescence value measured in the group obtained by treating with each test compound to 100% of the fluorescence value of the group (control group) obtained by treating only with tau proteins and inducing aggregation for 5 days is set forth in Table 6 below:

**[Table 6]**

| Test compounds | 50 uM | 500 uM |
|---|---|---|
| DN204296 | 30.26 | 16.48 |
| DN204297 | 52.16 | 17.97 |
| DN204298 | 61.36 | 29.73 |
| DN204299 | 72.21 | 45.09 |
| DN204301 | 80.31 | 45.47 |
| DN204303 | 81.77 | 39.19 |
| DN204304 | 67.53 | 50.23 |
| DN204305 | 61.22 | 56.43 |
| DN204306 | 66.03 | 73.34 |
| DN204287 | 61.17 | 71.42 |
| DN204289 | 57.57 | 52.83 |
| DN204290 | 54.41 | 40.38 |
| DN204285 | 64.04 | 78.38 |
| DN204295 | 89.98 | N.T. |
| DN204292 | 82.23 | 77.46 |
| DN204293 | N.T. | 95.86 |
| DN204808 | 62.2 | 41.11 |
| DN204809 | 51.93 | 30.87 |
| DN204811 | 50.44 | 31.04 |
| DN204812 | 45.75 | 44 |
| DN204814 | 30.07 | -22.72 |
| DN204815 | 39.88 | 36.97 |
| DN204816 | 40.39 | 33.92 |
| DN204817 | 49.84 | 43.06 |
| DN204818 | 14.94 | 5.16 |
| DN205106 | 59.16 | 39.38 |
| DN205107 | 42.65 | 42.34 |
| DN205108 | 85.88 | 95.34 |
| DN205109 | 82.78 | 44.77 |
| DN205110 | 78.08 | 77.98 |
| DN205353 | 66.25 | 80.59 |
| DN205354 | 77.68 | 43.5 |
| DN205355 | 49.41 | 30.65 |
| DN205780 | 27.50 | 10.84 |
| DN205781 | 28.74 | 23.79 |

| | | |
|---|---|---|
| Tau protein aggregate degradation activity (% of control) | | |

### (N.T.: not tested)

As can be seen from Table 6 above, the compounds disclosed herein exhibit tau protein aggregate degradation activity ranging from about 10% or more to about 90% or more of that of the control group.

### Example 6. Preparation of tau PROTAC compound containing compound of present invention

### 6-1. Preparation of (Z)-N-(2-(2,6-dioxopiperidin-3-yl)-1,3-dioxoisoindolin-4-yl)-2-(2-(2-(2-(4-((3-((1-ethyl-5-oxo-2-thioxoimidazolidin-4-ylidene)methyl)-1H-pyrrolo[3,2-b]pyridin-1-yl)methyl)-1H-1,2,3-triazol-1-yl)ethoxy)ethoxy)ethoxy)acetamide (TAU-1)

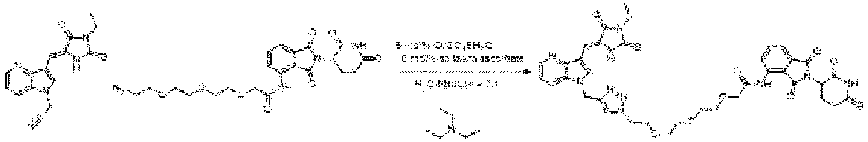

The following method was performed to prepare the TAU-1 compound, which is a tau PROTAC compound containing the compound of the present invention.

(Z)-3-ethyl-5-((1-(prop-2-yn-1-yl)-1H-pyrrolo[3,2-b]pyridin-3-yl)methylene)-2-thioxoimidazolidin-4-one (1 eq) and 2-(2-(2-(2-azidoethoxy)ethoxy)ethoxy)-N-(2-(2,6-dioxopiperidin-3-yl)-1,3-dioxoisoindolin-4-yl)acetamide (1 eq) were added to a solution of water and t-butanol at a ratio of 1:1 and then a 1M aqueous copper sulfate solution (5 mol %) and sodium ascorbate (0.3 eq) were added thereto. Then, the resulting mixture was stirred at room temperature for 4 hours. When the reaction was completed, an aqueous ammonia solution was added to the reaction solution to terminate the reaction, and ethyl acetate was further added thereto to extract the organic layer. The organic layer was washed with brine and dehydrated with sodium disulfite. The residue obtained by distilling the solvent under reduced pressure was separated and purified by prep HPLC to obtain the target compound.

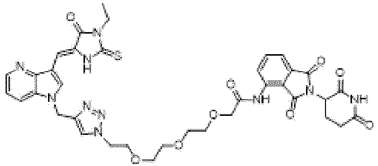

¹H NMR (400MHz, CDCl3) δ 10.362 (s, 1H), 8.765 (d, J=8.4 Hz, 1H), 8.605 (s, 1H), 8.544 (d, J=4.2 Hz, 1H), 7.942 (d, J=8.2 Hz, 1H), 7.795 (s, 2H), 7.700 (t, J=7.6 Hz, 1H), 7.554 (d, J=7.2 Hz, 1H), 7.254-7.287 (m, 2H), 6.704 (s, 1H), 5.488 (s, 2H), 4.946-4.990 (m, 1H), 4.513 (t, J=4.8 Hz, 2H), 4.164 (s, 2H), 3.938 (d, J=8.0 Hz, 2H), 3.837 (t, J=4.8 Hz, 2H), 3.763-3.783 (m, 2H), 3.684-3.704 (m, 2H), 3.565 (s, 4H), 2.763-2.923 (m, 3H), 2.182-2.192 (m, 1H), 1.254-1.297 (m, 3H)

### 6-2. Preparation of (Z)-N-(2-(2,6-dioxopiperidin-3-yl)-1,3-dioxoisoindolin-4-yl)-2-(2-(2-(4-((3-((1-ethyl-5-oxo-2-thioxoimidazolidin-4-ylidene)methyl)-1H-indol-1-yl)methyl)-1H-1,2,3-triazol-1-yl)ethoxy)ethoxy)acetamide (TAU-2)

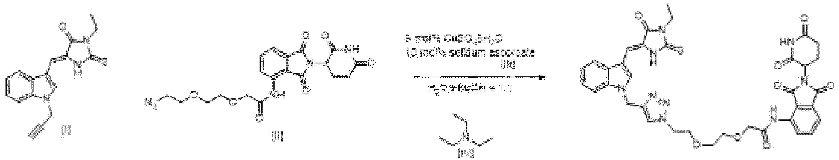

The following method was performed to prepare the TAU-2 compound, which is a tau PROTAC compound containing the compound of the present invention.

Specifically, a TAU-2 compound was synthesized in the same manner as in 6-1, except that 2-(2-(2-azidoethoxy)ethoxy)-N-(2-(2,6-dioxopiperidin-3-yl)-1,3-dioxoisoindolin-4-yl)acetamide (1 eq) was used. 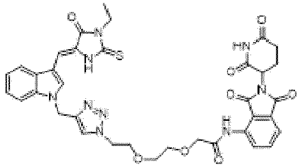 ¹H NMR (400MHz, CDCl3) δ 9.991(s, 1H), 9.942 (s, 1H), 8.928 (s, 1H), 8.828 (d, J=8.0 Hz, 1H), 7.895 (s, 1H), 7.699 (t, J=8.0 Hz, 1H), 7.604(t, J=8.0 Hz, 2H), 7.499 (d, J=7.2 Hz, 1H), 7.397 (d, J=8.0 Hz, 1H), 7.204-7.297 (m, 2H), 6.704 (s, 1H), 5.358(d, J=4.88, 2H), 5.045 (dd, J=5.6 Hz, 12.8 Hz, 1H), 4.481-4.576 (m, 2H), 3.835-4.002 (m, 6H), 3.688-3.739 (m, 1H), 3.641 (t, J=8.2 Hz, 3H), 2.788-2.929 (m, 2H), 2.6525 (s, 1H), 2.240 (t, J=8.0Hz, 1H), 1.334 (t, J=7.2 Hz, 3H)

### Example 7. Identification of phosphorylated tau degradation activity of tau PROTAC in HEK293T P301L mutant cell line

The following experiment was performed to evaluate the phosphorylated tau degradation activity of the compound according to the present invention.

Specifically, the pRK5-EGFP-Tau P301L vector from Addgene (Addgene, MA, USA) was purchased and used in order to stably express P301L, which is a known representative mutation of pathological tau, in HEK293T cells. The P301L vector was inserted into HEK293T cells using a FuGENE HD transfection reagent (Promega, WI, USA) and then stabilized. The cells were removed therefrom and then only the cells into which the vector was inserted were isolated using a fluorescence-activated cell sorter (BD FACS Aria III, BD Bioscience, NJ, USA). After the isolated cells were incubated for 2 to 3 days, they were removed again and separated and cultured using a FACS twice to finally obtain a HEK293T P301 mutant cell line.

The obtained cells were seeded in each well of a 6-well plate and stabilized for 18 hours, and then each well was treated with the compound of Example 1 such that the final concentration reached 1 or 10 uM. The control group was treated with the same concentration (0.01%) of DMSO used as the solvent of the compound of Example. 72 hours later, the protein was extracted with a lysis buffer and quantified, and then Western blot was performed. A protein sample was electrophoresed on a 4-20% mini-PROTEIN TGX precast protein gel (Bio-Rad, CA, USA) and transferred to a PVDF membrane (Immobilon-P, Merck, Darmstadt, Germany). The primary antibodies used herein were P-Tau AT8 (#MN1020), P-Tau S396 (#44-752G), and P-Tau S356 (#44-751G) from ThermoFisher scientific (MA, USA), and GAPDH (SC-47724) from SANTA CRUZ Biotechnology (TX, USA). The secondary antibody used herein was rabbit/mouse IgG from GeneTex (CA, USA). The image obtained through ImageQuant LAS 4000 (GE healthcare, IL, USA) and the result of quantification using an Image J program are shown in FIG. 1.

As can be seen from FIG. 1, in P-Tau AT8 (S202, T205), the group treated with 10 µM TAU-1 PROTAC prepared in Example 6-1 exhibited degradation activity of 26% compared to the control group and the group treated with the TAU-2 PROTAC prepared in Example 6-2 exhibited degradation activity of 59 and 53% at concentrations of 1 and 10 µM, respectively. In P-Tau (S396), TAU-1 PROTAC exhibited degradation activity of 67% at concentrations of 1 and 10 µM, and TAU-2 PROTAC exhibited degradation activity of 30% at a concentration of 1 µM. In addition, in P-Tau (S356), the group treated with 10 µM TAU-1 PROTAC exhibited degradation activity of 81%, and the group treated with TAU-2 PROTAC exhibited phosphorylated tau degradation activity of 62% at 1 µM and 80% at 10 µM. The result showed that treatment with TAU-1 and TAU-2 PROTAC exhibited effective degradation activity in three phosphorylated tau proteins, TAU-1 PROTAC exhibited more effective degradation activity in P-Tau S396, and TAU-2 PROTAC exhibited more effective degradation activity in P-Tau S202, T205, and S356. Accordingly, the compounds according to examples of the present invention have phosphorylated tau degradation activity and are particularly useful for the treatment of brain diseases attributable to phosphorylated tau proteins.

### Example 8. Determination of binding affinity (K_{d}) of tau PROTAC to WT tau or pathological tau

The following experiment was performed to verify that the compound according to the present invention selectively degrades only phosphorylated tau.

In order to biophysically analyze selective binding of the tau PROTAC to P301S, which is known as a representative mutant of pathological tau, non-pathological (WT) tau and pathological (P301S) tau were cloned into pRSET-B vectors to obtain plasmids. Star pLysS cells were used to overexpress proteins and 1 mM IPTG was added to the medium at an OD₆₀₀ of 0.4 to 0.6. The intracellular proteins were extracted from the cells lysed in a lysis buffer through heat shock (95°C), and WT tau and P301S tau were obtained at a high purity (>95%) using cation exchange chromatography and size exclusion chromatography.

Whether or not the obtained proteins bound to the tau PROTAC was determined through an SPR experiment. A CM5 sensor chip including dextran attached thereto was used, tau PROTAC was allowed to flow and the binding affinity between the protein and the compound was determined based on a response unit (RU). The binding affinity (K_{d}) to the protein was measured using concentration-dependent kinetic assay. The results of measurement of binding affinity between the tau PROTAC compound and non-pathological WT Tau or pathological P301S tau are shown in FIG. 2. As can be seen from FIG. 2, the tau PROTAC selectively binds to the pathological P301S, but hardly binds to nonpathological WT tau. Tau-1 PROTAC 1 and Tau-2 PROTAC compounds were found to have binding affinity to tau of 190.05 µM and 198.7 µM, respectively, which means that the compounds have very low or no binding affinity. On the other hand, Tau-1 PROTAC 1 and Tau-2 PROTAC compounds had binding affinity to the P301S tau of 42.05 µM and 18.74 µM, respectively. Therefore, the compounds according to the examples of the present invention are degrading only pathological tau without degrading nonpathological WT tau through selective binding to P301S tau, thus being useful for the treatment of brain diseases.

## Claims

1. A pyrrolidine derivative represented by the following Formula I, II, III, IV, or V, or a pharmaceutically acceptable salt thereof: 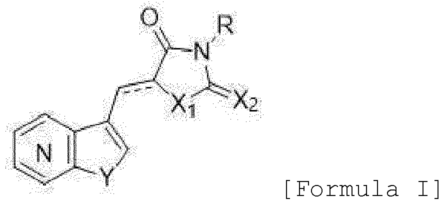 in Formula I, X₁ and X₂ are each independently NH, O, or S, Y is NH or O, and R is H or an alkyl group having 1 to 6 carbon atoms ("N" in the 6-membered ring means that any one randomly selected from the 6 carbons constituting the ring is substituted); 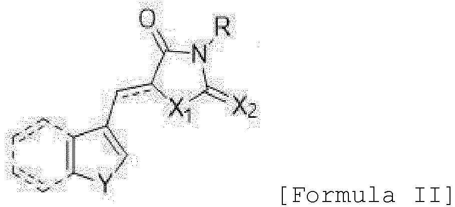 in Formula II, X₁ and X₂ are each independently NH, O, or S, Y is NH, O, or S, and R is H or an alkyl group having 1 to 6 carbon atoms; 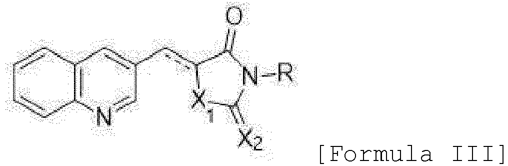 in Formula III, X₁ and X₂ are each independently NH or S, and R is H or an alkyl group having 1 to 6 carbon atoms; 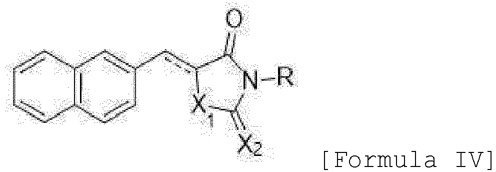 in Formula III, X₁ and X₂ are each independently NH, O or S, and R is H or an alkyl group having 1 to 6 carbon atoms; and 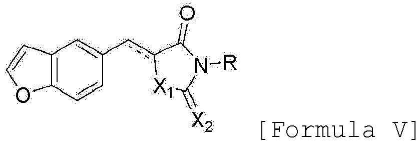 in Formula V, X₁ and X₂ are each independently NH, O, or S, and R is H or an alkyl group having 1 to 6 carbon atoms.

2. The pyrrolidine derivative or pharmaceutically acceptable salt thereof according to claim 1,
wherein, in Formulas I to V, R is H or alkyl having 1 to 4 carbon atoms.

3. The pyrrolidine derivative or pharmaceutically acceptable salt thereof according to claim 1, wherein, 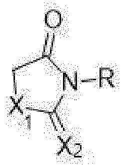 in Formulas I to V may be

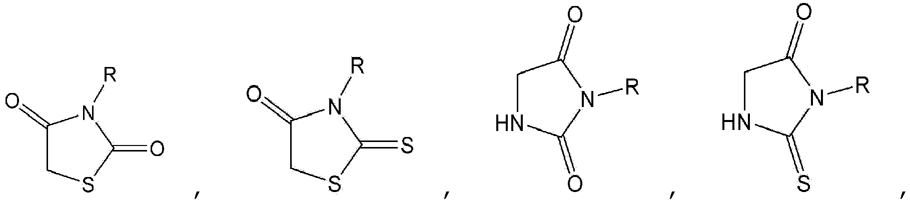

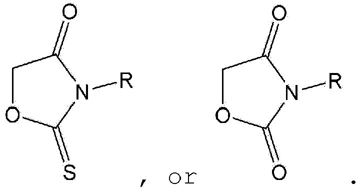

4. The pyrrolidine derivative or pharmaceutically acceptable salt thereof according to claim 1, wherein, 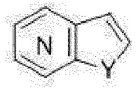 of Formula I may be

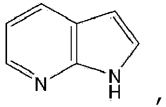

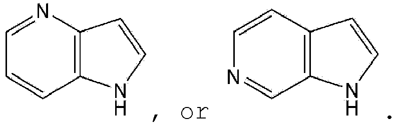

5. The pyrrolidine derivative or pharmaceutically acceptable salt thereof according to claim 1, wherein, 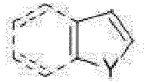 in Formula II may be

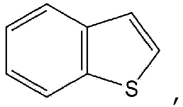

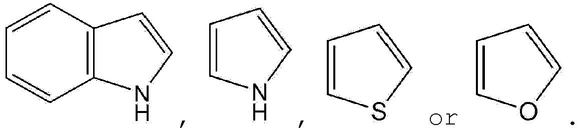

6. The pyrrolidine derivative or pharmaceutically acceptable salt thereof according to claim 1,
wherein the pyrrolidine derivative or pharmaceutically acceptable salt thereof is selected from the group consisting of the following compounds:
(Z)-5-(benzo[b]thiophen-3-ylmethylene)thiazolidine-2,4-dione;
(Z)-5-((1H-pyrrolo[2,3-b]pyridin-3-yl)methylene)thiazolidine-2,4-dione;
(Z)-5-((1H-pyrrolo[3,2-b]pyridin-3-yl)methylene)thiazolidine-2,4-dione;
(Z)-5-((1H-pyrrolo[2,3-c]pyridin-3-yl)methylene)thiazolidine-2,4-dione;
5-(benzo[b]thiophen-3-ylmethyl)thiazolidine-2,4-dione;
5-(benzo[b]thiophen-3-ylmethyl)-2-thioxothiazolidin-4-one;
5-((1H-pyrrolo[2,3-b]pyridin-3-yl)methyl)-2-thioxothiazolidin-4-one;
(Z)-5-((1H-pyrrolo[3,2-c]pyridin-3-yl)methylene)-2-thioxothiazolidin-4-one;
(Z)-5-((1H-pyrrolo[3,2-b]pyridin-3-yl)methylene)-2-thioxothiazolidin-4-one;
(Z)-5-(benzo[b]thiophen-3-ylmethylene)-3-ethyl-2-thioxoimidazolidin-4-one;
(Z)-5-((1H-pyrrolo[2,3-b]pyridin-3-yl)methylene)-3-ethyl-2-thioxoimidazolidin-4-one;
(Z)-5-((1H-pyrrolo[3,2-b]pyridin-3-yl)methylene)-3-ethyl-2-thioxoimidazolidin-4-one;
(Z)-5-(benzo[b]thiophen-3-ylmethylene)-3-methylimidazolidine-2,4-dione;
(Z)-5-((1H-pyrrolo[3,2-b]pyridin-3-yl)methylene)-3-methylimidazolidine-2,4-dione;
5-(benzo[b]thiophen-3-ylmethyl)-3-methyl-2-thioxoimidazolidin-4-one;
(Z)-5-((1H-pyrrolo[2,3-b]pyridin-3-yl)methylene)-2-thioxothiazolidin-4-one;
(Z)-5-(thiophen-3-ylmethylene)thiazolidine-2,4-dione;
5-(furan-3-ylmethyl)thiazolidine-2,4-dione;
5-(thiophen-3-ylmethyl)thiazolidine-2,4-dione;
5-(quinolin-3-ylmethyl)thiazolidine-2,4-dione;
5-((1H-pyrrol-3-yl)methyl)thiazolidine-2,4-dione;
(Z)-5-((1H-pyrrol-3-yl)methylene)-2-thioxothiazolidin-4-one;
5-(quinolin-3-ylmethyl)-2-thioxothiazolidin-4-one;
5-((1H-pyrrol-3-yl)methyl)-2-thioxothiazolidin-4-one;
5-(furan-3-ylmethyl)-2-thioxothiazolidin-4-one;
(Z)-3-ethyl-5-(quinolin-4-ylmethylene)-2-thioxoimidazolidin-4-one;
(Z)-3-ethyl-5-(thiophen-3-ylmethylene)-2-thioxoimidazolidin-4-one;
(Z)-3-ethyl-5-(furan-3-ylmethylene)-2-thioxoimidazolidin-4-one;
(Z)-5-(benzofuran-5-ylmethylene)-3-ethyl-2-thioxoimidazolidin-4-one;
(Z)-5-(isoquinolin-4-ylmethylene)-3-methylimidazolidine-2,4-dione;
(Z)-5-(benzofuran-7-ylmethylene)-3-methylimidazolidine-2,4-dione;
(Z)-3-methyl-5-(quinolin-3-ylmethylene)imidazolidine-2,4-dione;
3-methyl-5-(quinolin-3-ylmethyl)imidazolidine-2,4-dione;
5-((1H-indol-3-yl)methyl)-3-methylimidazolidine-2,4-dione;
(Z)-5-(benzofuran-6-ylmethylene)-3-methylimidazolidine-2,4-dione;
5-(benzofuran-5-ylmethyl)-3-ethyl-2-thioxoimidazolidin-4-one;
3-ethyl-5-(quinolin-4-ylmethyl)-2-thioxoimidazolidin-4-one;
(Z)-5-(benzo[b]thiophen-3-ylmethylene)-2-thioxothiazolidin-4-one;
(Z)-5-((1H-indol-3-yl)methylene)thiazolidine-2,4-dione;
5-((1H-indol-3-yl)methyl)thiazolidine-2,4-dione;
5-((1H-indol-3-yl)methyl)-2-thioxothiazolidin-4-one;
(Z)-5-((1H-indol-3-yl)methylene)-3-ethyl-2-thioxoimidazolidin-4-one;
(Z)-3-ethyl-5-(thiophen-2-ylmethylene)-2-thioxoimidazolidin-4-one;
(Z)-5-((1H-indol-3-yl)methylene)-3-methylimidazolidine-2,4-dione;
(Z)-5-((1H-pyrrolo[2,3-c]pyridin-3-yl)methylene)-3-ethyl-2-thioxothiazolidin-4-one;
(Z)-5-((1H-pyrrolo[3,2-c]pyridin-3-yl)methylene)-3-ethyl-2-thioxothiazolidin-4-one;
(Z)-5-((1H-pyrrolo[3,2-b]pyridin-3-yl)methylene)-3-ethyl-2-thioxothiazolidin-4-one;
(Z)-5-(benzo[b]thiophen-3-ylmethylene)-3-ethyl-2-thioxothiazolidin-4-one;
(Z)-3-ethyl-5-(naphthalen-2-ylmethylene)-2-thioxothiazolidin-4-one;
(Z)-5-((1H-pyrrolo[2,3-c]pyridin-3-yl)methylene)-3-ethylimidazolidine-2,4-dione;
(Z)-5-((1H-pyrrolo[3,2-c]pyridin-3-yl)methylene)-3-ethylimidazolidine-2,4-dione;
(Z)-5-((1H-pyrrolo[3,2-b]pyridin-3-yl)methylene)-3-ethylimidazolidine-2,4-dione;
(Z)-5-(benzo[b]thiophen-3-ylmethylene)-3-ethylimidazolidine-2,4-dione;
(Z)-3-ethyl-5-(naphthalen-2-ylmethylene)imidazolidine-2,4-dione;
(Z)-5-(furan-3-ylmethylene)thiazolidine-2,4-dione;
(Z)-5-((1H-indol-3-yl)methylene)-2-thioxothiazolidin-4-one;
(Z)-5-(furan-3-ylmethylene)-2-thioxothiazolidin-4-one;
(Z)-5-(thiophen-3-ylmethylene)-2-thioxothiazolidin-4-one;
5-(thiophen-3-ylmethyl)-2-thioxothiazolidin-4-one;
(Z)-5-(benzofuran-5-ylmethylene)-2-thioxothiazolidin-4-one;
(Z)-5-((1H-indol-3-yl)methylene)-3-methylimidazolidine-2,4-dione;
(Z)-5-((1H-pyrrolo[2,3-b]pyridin-3-yl)methylene)-3-methyl-2-thioxooxazolidin-4-one;
(Z)-5-((1H-pyrrolo[2,3-c]pyridin-3-yl)methylene)-3-methyl-2-thioxooxazolidin-4-one;
(Z)-5-((1H-pyrrolo[3,2-b]pyridin-3-yl)methylene)-3-methyl-2-thioxooxazolidin-4-one;
(Z)-5-((1H-pyrrolo[2,3-b]pyridin-3-yl)methylene)-3-ethyl-2-thioxooxazolidin-4-one;
(Z)-5-((1H-pyrrolo[2,3-c]pyridin-3-yl)methylene)-3-ethyl-2-thioxooxazolidin-4-one;
(Z)-5-((1H-pyrrolo[3,2-b]pyridin-3-yl)methylene)-3-ethyl-2-thioxooxazolidin-4-one;
(Z)-5-((1H-pyrrolo[2,3-b]pyridin-3-yl)methylene)-3-methylthiazolidine-2,4-dione;
(Z)-5-((1H-pyrrolo[2,3-c]pyridin-3-yl)methylene)-3-methylthiazolidine-2,4-dione;
(Z)-5-((1H-pyrrolo[3,2-c]pyridin-3-yl)methylene)-3-methylthiazolidine-2,4-dione;
(Z)-5-((1H-pyrrolo[3,2-b]pyridin-3-yl)methylene)-3-methylthiazolidine-2,4-dione;
(Z)-5-((1H-pyrrolo[2,3-b]pyridin-3-yl)methylene)-3-ethylthiazolidine-2,4-dione;
(Z)-5-((1H-pyrrolo[2,3-c]pyridin-3-yl)methylene)-3-ethylthiazolidine-2,4-dione;
(Z)-5-((1H-pyrrolo[2,3-b]pyridin-3-yl)methylene)oxazolidine-2,4-dione;
(Z)-5-((1H-pyrrolo[2,3-c]pyridin-3-yl)methylene)oxazolidine-2,4-dione;
(Z)-5-((1H-pyrrolo[3,2-c]pyridin-3-yl)methylene)oxazolidine-2,4-dione;
(Z)-5-((1H-pyrrolo[3,2-b]pyridin-3-yl)methylene)oxazolidine-2,4-dione;
(Z)-5-((1H-pyrrolo[2,3-b]pyridin-3-yl)methylene)-3-methyloxazolidine-2,4-dione;
(Z)-5-((1H-pyrrolo[2,3-c]pyridin-3-yl)methylene)-3-methyloxazolidine-2,4-dione;
(Z)-5-((1H-pyrrolo[3,2-c]pyridin-3-yl)methylene)-3-methyloxazolidine-2,4-dione;
(Z)-5-((1H-pyrrolo[3,2-b]pyridin-3-yl)methylene)-3-methyloxazolidine-2,4-dione;
(Z)-5-((1H-pyrrolo[2,3-b]pyridin-3-yl)methylene)-3-ethyloxazolidine-2,4-dione;
(Z)-5-((1H-pyrrolo[2,3-c]pyridin-3-yl)methylene)-3-ethyloxazolidine-2,4-dione;
(Z)-5-((1H-pyrrolo[3,2-c]pyridin-3-yl)methylene)-3-ethyloxazolidine-2,4-dione;
(Z)-5-((1H-pyrrolo[3,2-b]pyridin-3-yl)methylene)-3-ethyloxazolidine-2,4-dione;
(Z)-5-((1H-pyrrolo[2,3-b]pyridin-3-yl)methylene)-3-isopropyloxazolidine-2,4-dione;
(Z)-5-((1H-pyrrolo[2,3-c]pyridin-3-yl)methylene)-3-isopropyloxazolidine-2,4-dione;
(Z)-5-((1H-pyrrolo[3,2-c]pyridin-3-yl)methylene)-3-isopropyloxazolidine-2,4-dione;
(Z)-5-((1H-pyrrolo[3,2-b]pyridin-3-yl)methylene)-3-isopropyloxazolidine-2,4-dione;
(Z)-5-((1H-pyrrolo[2,3-b]pyridin-3-yl)methylene)imidazolidine-2,4-dione;
(Z)-5-((1H-pyrrolo[2,3-c]pyridin-3-yl)methylene)imidazolidine-2,4-dione;
(Z)-5-((1H-pyrrolo[3,2-c]pyridin-3-yl)methylene)imidazolidine-2,4-dione;
(Z)-5-((1H-pyrrolo[3,2-b]pyridin-3-yl)methylene)imidazolidine-2,4-dione;
(Z)-5-((1H-pyrrolo[2,3-b]pyridin-3-yl)methylene)-3-methylimidazolidine-2,4-dione;
(Z)-5-((1H-pyrrolo[2,3-c]pyridin-3-yl)methylene)-3-methylimidazolidine-2,4-dione;
(Z)-5-((1H-pyrrolo[3,2-c]pyridin-3-yl)methylene)-3-methylimidazolidine-2,4-dione;
(Z)-5-((1H-pyrrolo[2,3-b]pyridin-3-yl)methylene)-3-ethylimidazolidine-2,4-dione;
(Z)-5-((1H-pyrrolo[2,3-b]pyridin-3-yl)methylene)-3-isopropylimidazolidine-2,4-dione;
(Z)-5-((1H-pyrrolo[2,3-c]pyridin-3-yl)methylene)-3-isopropylimidazolidine-2,4-dione;
(Z)-5-((1H-pyrrolo[3,2-c]pyridin-3-yl)methylene)-3-isopropylimidazolidine-2,4-dione;
(Z)-5-((1H-pyrrolo[3,2-b]pyridin-3-yl)methylene)-3-isopropylimidazolidine-2,4-dione;
(Z)-5-((1H-pyrrolo[2,3-b]pyridin-3-yl)methylene)-2-thioxothiazolidin-4-one;
(Z)-5-((1H-pyrrolo[2,3-c]pyridin-3-yl)methylene)-2-thioxothiazolidin-4-one;
(Z)-5-((1H-pyrrolo[2,3-b]pyridin-3-yl)methylene)-3-methyl-2-thioxothiazolidin-4-one;
(Z)-5-((1H-pyrrolo[2,3-c]pyridin-3-yl)methylene)-3-methyl-2-thioxothiazolidin-4-one;
(Z)-5-((1H-pyrrolo[3,2-c]pyridin-3-yl)methylene)-3-methyl-2-thioxothiazolidin-4-one;
(Z)-5-((1H-pyrrolo[3,2-b]pyridin-3-yl)methylene)-3-methyl-2-thioxothiazolidin-4-one;
(Z)-5-((1H-pyrrolo[2,3-b]pyridin-3-yl)methylene)-3-ethyl-2-thioxothiazolidin-4-one;
(Z)-5-((1H-pyrrolo[2,3-b]pyridin-3-yl)methylene)-3-isopropyl-2-thioxothiazolidin-4-one;
(Z)-5-((1H-pyrrolo[2,3-c]pyridin-3-yl)methylene)-3-isopropyl-2-thioxothiazolidin-4-one;
(Z)-5-((1H-pyrrolo[3,2-c]pyridin-3-yl)methylene)-3-isopropyl-2-thioxothiazolidin-4-one;
(Z)-5-((1H-pyrrolo[3,2-b]pyridin-3-yl)methylene)-3-isopropyl-2-thioxothiazolidin-4-one;
(Z)-5-((1H-pyrrolo[2,3-b]pyridin-3-yl)methylene)-3-isopropyl-2-thioxooxazolidin-4-one;
(Z)-5-((1H-pyrrolo[2,3-c]pyridin-3-yl)methylene)-3-isopropyl-2-thioxooxazolidin-4-one;
(Z)-5-((1H-pyrrolo[3,2-c]pyridin-3-yl)methylene)-3-isopropyl-2-thioxooxazolidin-4-one;
(Z)-5-((1H-pyrrolo[3,2-b]pyridin-3-yl)methylene)-3-isopropyl-2-thioxooxazolidin-4-one;
(Z)-5-((1H-pyrrolo[2,3-b]pyridin-3-yl)methylene)-3-isopropylthiazolidine-2,4-dione;
(Z)-5-((1H-pyrrolo[2,3-c]pyridin-3-yl)methylene)-3-isopropylthiazolidine-2,4-dione;
(Z)-5-((1H-pyrrolo[3,2-c]pyridin-3-yl)methylene)-3-isopropylthiazolidine-2,4-dione;
(Z)-5-((1H-pyrrolo[3,2-b]pyridin-3-yl)methylene)-3-isopropylthiazolidine-2,4-dione;
(Z)-5-((1H-pyrrolo[2,3-c]pyridin-3-yl)methylene)-3-isopropyl-2-thioxoimidazolidin-4-one;
(Z)-5-((1H-pyrrolo[3,2-c]pyridin-3-yl)methylene)-3-isopropyl-2-thioxoimidazolidin-4-one; and
(Z)-5-((1H-pyrrolo[3,2-b]pyridin-3-yl)methylene)-3-isopropyl-2-thioxoimidazolidin-4-one.

7. The pyrrolidine derivative or pharmaceutically acceptable salt thereof according to claim 1, wherein the pyrrolidine derivative or pharmaceutically acceptable salt thereof is used to prepare a proteolysis-targeting chimera (PROTAC) compound.

8. A proteolysis-targeting chimera (PROTAC) compound comprising the pyrrolidine derivative or pharmaceutically acceptable salt thereof according to claim 1.

9. The proteolysis-targeting chimera (PROTAC) compound according to claim 8, further comprising a target-binding ligand or linker, in addition to the pyrrolidine derivative or pharmaceutically acceptable salt thereof.

10. The proteolysis-targeting chimera (PROTAC) compound according to claim 8, wherein the PROTAC compound is a TAU-1 compound represented by the following Formula VI or a TAU-2 compound represented by the following Formula VII:

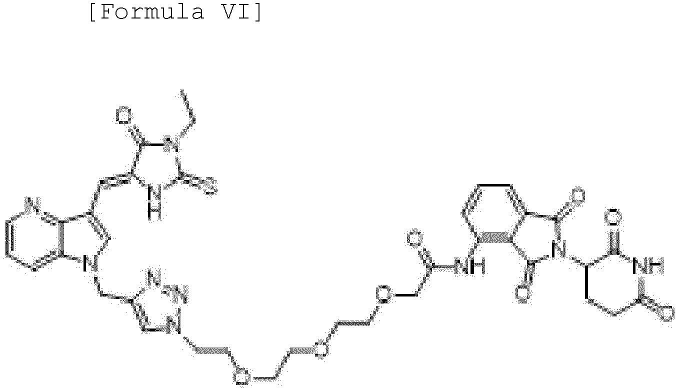

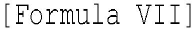

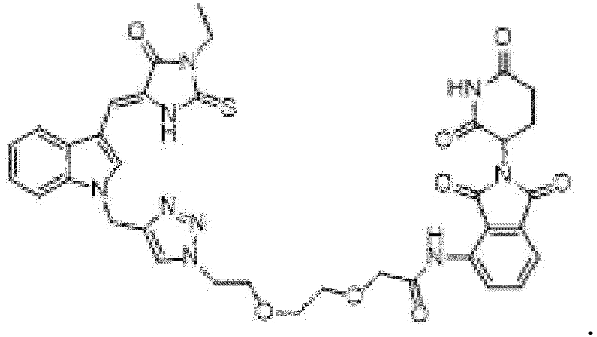

11. A composition for inhibiting aggregation of beta-amyloids comprising at least one selected from the group consisting of the pyrrolidine derivative and pharmaceutically acceptable salt thereof according to any one of claims 1 to 7 and the proteolysis-targeting chimera (PROTAC) compound according to claim 8 as an active ingredient.

12. A composition for inhibiting aggregation of tau proteins comprising at least one selected from the group consisting of the pyrrolidine derivative and pharmaceutically acceptable salt thereof according to any one of claims 1 to 7 and the proteolysis-targeting chimera (PROTAC) compound according to claim 8 as an active ingredient.

13. A composition for degrading aggregates of beta-amyloids comprising at least one selected from the group consisting of the pyrrolidine derivative and pharmaceutically acceptable salt thereof according to any one of claims 1 to 7 and the proteolysis-targeting chimera (PROTAC) compound according to claim 8 as an active ingredient.

14. A composition for degrading aggregates of tau proteins comprising at least one selected from the group consisting of the pyrrolidine derivative and pharmaceutically acceptable salt thereof according to any one of claims 1 to 7 and the proteolysis-targeting chimera (PROTAC) compound according to claim 8 as an active ingredient.

15. A composition for inhibiting aggregation of beta-amyloids comprising at least one selected from the group consisting of the pyrrolidine derivative and pharmaceutically acceptable salt thereof according to any one of claims 1 to 7 and the proteolysis-targeting chimera (PROTAC) compound according to claim 8 as an active ingredient.

16. A composition for protecting neurons comprising at least one selected from the group consisting of the pyrrolidine derivative and pharmaceutically acceptable salt thereof according to any one of claims 1 to 7 and the proteolysis-targeting chimera (PROTAC) compound according to claim 8 as an active ingredient.

17. The composition for protecting neurons according to claim 16,
wherein the protecting neurons comprises protecting the neurons from damage due to aggregation or accumulation of beta-amyloids or tau proteins.

18. A composition for preventing or treating a beta-amyloid- and/or tau protein-related disease comprising at least one selected from the group consisting of the pyrrolidine derivative and pharmaceutically acceptable salt thereof according to any one of claims 1 to 7 and the proteolysis-targeting chimera (PROTAC) compound according to claim 8 as an active ingredient.

19. The composition according to claim 18, wherein the beta-amyloid- and/or tau protein-related disease is Alzheimer's disease, vascular dementia, mild cognitive impairment, cerebral amyloid angiopathy, Down's syndrome, amyloid stroke, systemic amyloid disease, Dutch amyloidosis, tauopathy, dementia with Lewy bodies (DLB), multi-infarct dementia (MID), frontotemporal lobar degeneration (FTLD), Pick's disease, corticobasal degeneration (CBD), progressive supranuclear palsy (PSP), Parkinson's disease, or Huntington's disease.
